# EUROPEAN PATENT APPLICATION

(11) **EP 3 882 247 A1**
(43) Date of publication of application: **22.09.2021**
(21) Application number: 19883726.2
(22) Date of filing: 13.11.2019
(51) Int. Cl.: C07D 471/04, A61K 31/4375, A61P 35/00

(54) **SIX-MEMBERED AND SIX-MEMBERED HETEROCYCLIC COMPOUND AND USES THEREOF SERVING AS PROTEIN RECEPTOR KINASE INHIBITOR**

(30) Priority: 13.11.2018 CN 201811348040
(71) Applicant: Shanghai Ennovabio Pharmaceuticals Co., Ltd., Shanghai 201203 (CN)
(72) Inventor: JIANG, Lei, Shanghai 201203 (CN); FENG, Zhiyong, Shanghai 201203 (CN); JIN, Xian, Shanghai 201203 (CN); QIAO, Zhi, Shanghai 201203 (CN); SHOU, Jianyong, Shanghai 201203 (CN); SHANG, Ke, Shanghai 201203 (CN); WU, Danyi, Shanghai 201203 (CN); XU, Lingling, Shanghai 201203 (CN); XU, Yuan, Shanghai 201203 (CN); ZHANG, Shuyun, Shanghai 201203 (CN); ZHANG, Yi, Shanghai 201203 (CN); ZHANG, Yuxing, Shanghai 201203 (CN)
(74) Representative: dompatent von Kreisler Selting Werner - Partnerschaft von Patent- und Rechtsanwälten mbB
(86) International application number: PCT/CN2019/118217
(87) International publication number: WO 2020/098723

(57) **Abstract**

Provided are a preparation and applications of a six-membered fused with six-membered heterocyclic compound, specifically, provided in the present invention is a compound as represented by formula I as follows, where the definitions of the groups are as described in the description. The compound has TRK kinase inhibiting activity and can serve as a pharmaceutical composition for treating TRK dysfunction-related diseases.

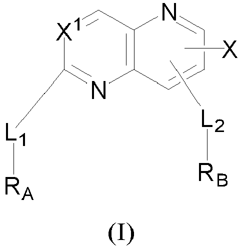

## Description

### FIELD OF THE INVENTION

The present invention relates to the field of small molecule medicine, and in particular, a class of TRK kinase inhibitors and the preparation and use thereof.

### BACKGROUND OF THE INVENTION

Tropomyosin-receptor kinase (TRK) is a type of nerve growth factor receptor which belongs to the receptor tyrosine kinase family, and mainly includes three highly homologous members TRKA, TRKB and TRKC, which are respectively encoded by NTRK1, NTRK2, and NTRK3. These receptor tyrosine kinases are mainly expressed in nerve tissues and play an important role in the development and physiological functions of nerve system through the activation of NTs (neurotrophins). As a tyrosine kinase receptor, each TRK possess a respective ligand binding to it so as to activate the downstream signaling pathway. NGF (nerve growth factor) specifically binds to and activates TRKA; TRKB ligand includes BDGF (brain-derived growth factor) and NT-4/5 (neurotrophin-4/5); and NT-3 specifically binds to and activates TRKC. All the three TRK receptors contain an extracellular ligand binding domains, transmembrane domains and intracellular domains kinase domain.

Ligand binding to the corresponding receptors triggers receptor dimerization and activation of the intrinsic cytoplasmic kinase domain and receptor autophosphorylation. The activated receptors initiate diverse signaling pathways such as Ras/MAPK, PLCγ/PKC and PI3K/AKT pathways, and further regulating a series of physiological processes such as proliferation, differentiation, and survival of neuronal cells (Bergman, et al. 1999). The TRK signal pathway is usually precisely regulated, and its abnormal activation thereof closely relates to tumorgenesis (Amatu, et al. 2016). The results show that there are many mechanisms which causes of abnormal activation of TRK pathways, including gene fusion, excessive expression of proteins, and mononucleotide mutations. Such abnormal activation closely relates to the pathogenesis of tumors, especially NTRK gene fusion, which has been proven to play an important role in the development of various type of cancers of any histology multiple kinds of tumorgenesis regardless of tissue sources and types of tumors. Due to the rapid development of NGS techniques and precision medical care, more and more NTRK fusion genes are found, such as ETV6-NTRK3, MPRIP-NTRK1, CD74-NTRK1, and the like have been shown to be sensitive to TRK inhibition and have significant response rate to TRK inhibitors in clinical trials. (Drilon, et al. 2018). Therefore, more and more TRK target inhibitors are reported in, such as WO2010048314, WO201146336, WO2017004342. At the same time, drug resistance occured in some treated patients during the clinical trial, and it is proven that such drug resistance is caused by mutations in some bases of the enzymatic domain, such as NTRK1 G595R or G667C mutation, NTRK3 G623R or G696A mutation. The development of new generation of TRK kinase inhibitors is expected to solve these problems.

In summary, there is an urgent to develop new generation of TRK kinase inhibitors.

### SUMMARY OF THE INVENTION

The object of the present invention is to provide a type of novel TRK kinase inhibitors.

In the first aspect of the invention, a compound of formula I is provided: 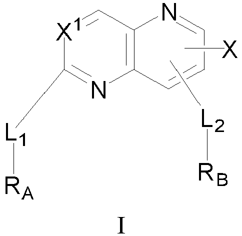 wherein,
X is H, halogen, D, CN or -CONH₂;
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or a substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is selected from the group consisting of a substituted or unsubstituted -(X⁴)_{z}-, wherein each of the X⁴ is independently selected from the group consisting of a substituted or unsubstituted C₁-C₈ alkylene, -O-, -C(=O)-, -S-, -S(=O)-, -S(=O)₂-, -NH-, -CONH-, -NHCO-, -NHCS-, -NHCONH-, -NHS(=O)-, -NHS(=O)₂-;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 hetero atoms selected from N, S or O, substituted or unsubstituted 5-10 membered heterocyclic group comprising 1-3 hetero atoms selected from N, S or O (including a monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amido(-C(=O)-N(RC)₂ and -NH-C(=O)(Rc), Rc is H or C1-C5 alkyl), C1-C6 alkyl-(C1-C6 amido), 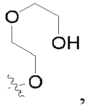 or a substituted or unsubstituted group selected from the group consisting of C1-C6 alkyl, C3-C8 cycloalkyl, C1-C6 amido, C6-C10 aryl, 5-10 membered heteroaryl comprising 1-3 heteroatoms selected from N, S or O, 3-12 membered heterocyclic group comprising 1-3 heteroatoms selected from N, S or O (including a monocyclic, bicyclic, spiro or bridged ring), - (CH₂) -C6-C10 aryl, -(CH₂)-(5-10 membered heteroaryl comprising 1-3 heteroatoms selected from N, S or O), wherein the substituent is selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxyl, -NH₂, carboxyl, C1-C6 amido (-C(=O)-N(Rc)₂ or -NH-C= O)(Rc), Rc is H or C1-C5 alkyl), C1-C6 alkyl-(C1-C6 amido), 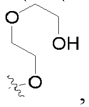 C1-C6 alkyl , C3-C8 cycloalkyl, C1-C6 amine group, C6-C10 aryl, 5-10 membered heteroaryl comprising 1-3 heteroatoms selected from N, S or O, 3-12 membered heterocyclic group comprising 1-3 heteroatoms selected from N, S or O (including a monocyclic, bicyclic, spiro or bridged ring), -(CH₂)-C6-C10 aryl, -(CH₂)-(5-10 membered heteroaryl comprising 1-3 heteroatoms selected from N, S, or O);
is the connection site of the group;
with the proviso that compounds of formula I are chemical stable structures.

In another preferred example, the L₁ is selected from the group consisting of: 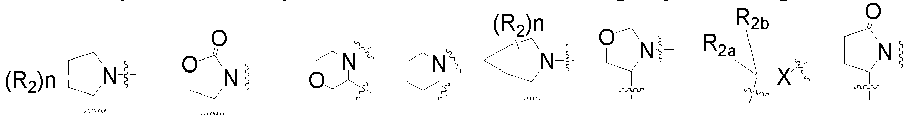
n is selected from the group consisting of 0, 1, 2 and 3;
R₂, R₂ₐ and R_{2b} are each independently selected from the group consisting of H, OH, halogen, substituted or unsubstituted C₁-C₈ alkyl;
X is selected from the group consisting of NH, O, -CONH-, -NHCO-, S, -S(=O)₂-, -NHS(=O)-, -NHS(=O)₂-;
R_{A} is 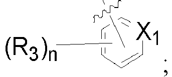 wherein the 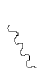 is the connection site of R_{A} and L₁;
L₂ is 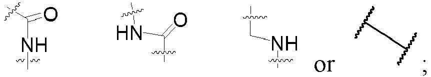
R_{B} is 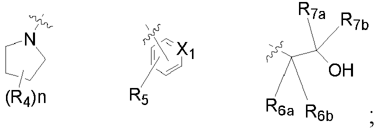 wherein the 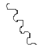 is the connection site of R_{B} and L₂;
R₃ is selected from the group consisting of H, halogen, C1-C6 alkoxy, halogenated C1-C6 alkyl, halogenated C1-C6 alkoxy;
R₄ and R₅ are each independently selected from the group consisting of H, OH, halogen, C1-C6 alkyl-OH, C₁-C₆ alkoxy, C₁-C₆ alkyl amine group, C₁-C₆ alkyl amido, -(C₁-C₆ alkyl)-NH-(C₁-C₆ alkyl), -(C₁-C₆ alkyl amido)-(C₁-C₆ alkyl);
R₆ₐ, R_{6b}, R₇ₐ, R_{7b} are each independently selected from the group consisting of H, OH, halogen; or R₆ₐ, R_{6b}, R₇ₐ, R_{7b} together with carbon atoms to which they are connected form a 5-12 membered heterocyclic group comprising 1-3 heteroatoms selected from N, S or O.

In another preferred embodiment, the compound has the structure shown in the following formula II: 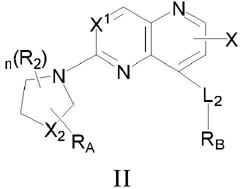 wherein the X₂ is selected from the group consisting of C=O, -CH₂-, O and NH.

In another preferred embodiment, the compound has the structure shown in the following formula IIIa:

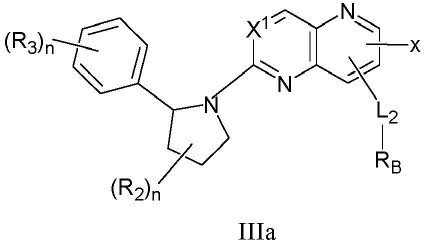

In another aspect of the invention, a compound of formula IV is provided: 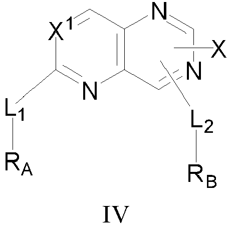 wherein,
X is H, D or halogen;
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is a 5-10 membered heterocycloalkylene group having 1-3 heteroatoms selected from N, S or O, 5-10 membered heteroaryl having 1-3 heteroatoms selected from N, S or O;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 heteroatoms selected from N, S or O, substituted or unsubstituted 5-10 membered heterocyclic group comprising 1-3 heteroatoms selected from N, S or O (including monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amido (-C(=O)-N(Rc)₂ or -NH-C=ORc Rc is H or C1-C5 alkyl), C1-C6 alkyl-(C1-C6 amido), 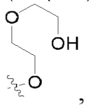 or a substituted or unsubstituted group selected from the group consisting of a C1-C6 alkyl unsubstituted or substituted by one or more hydroxyls, C3-C8 cycloalkyl, C1-C6 amido, C6-C10 aryl, 5-10 membered heteroaryl comprising 1-3 heteroatoms selected from N, S, or O, 5-12 membered heterocyclic ring comprising1-3 heteroatoms selected from N, S or O (including monocyclic, bicyclic, spiro or bridged ring), -(CH₂)-C6-C10 aryl, -(CH₂)-(5-10 membered heteroaryl comprising 1-3 heteroatoms selected from N, S, or O), wherein the substituent is selected from the group consisting of a halogen, C1-C6 alkyl unsubstituted or substituted by one or more hydroxyls, C1-C6 alkoxy, oxo, -CN, -NH₂, -OH, C6-C10 aryl, C1-C6 amino, C1-C6 amido, 5-10 membered heteroaryl comprising 1-3 heteroatoms selected from N, S, or O; 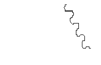
is the connection site of the group;
with the proviso that compounds of formula I are chemical stable structures.

In another preferred embodiment, the compound has the structure shown in the following formula V: 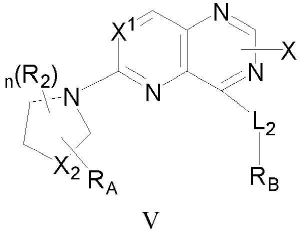 wherein the X₂ is selected from the group consisting of C=O, -CH₂-, O and NH.

In another preferred embodiment, the compound has the structure shown in the following formula IIIa:

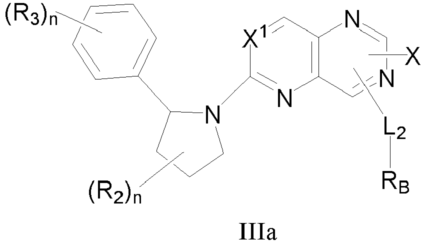

In the second aspect of the invention, a pharmaceutical composition is provided, comprising (1) a compound according to the first aspect of the invention, or a stereoisomer or tautomer thereof, or a pharmaceutically acceptable salt, hydrate or solvate thereof; (2) pharmaceutically acceptable carriers.

In another preferred embodiment, the pharmaceutical composition is an injection, capsule, tablet, pill, powder or granule.

In another preferred embodiment, the disease is selected from the group consisting of cancer, proliferative disease, pain, skin disease or condition, metabolic disease, muscle disease, neurological disease, autoimmune disease, itching caused by dermatitis, inflammation related diseases, bone related diseases.

In another preferred embodiment, the cancer is selected from the group consisting of TRK function abnormalities (abnormal activation functions induced by TRK gene amplification, overexpression, mutation or gene fusion) related cancer (including, but not limited to): neuroblastoma, prostate cancer, thyroid cancer, lung cancer, ovarian cancer, pancreatic cancer, colorectal cancer, non-small cell lung cancer, fibrosarcoma, etc.

In the third aspect of the invention, a use of the compound of the present invention or a stereoisomer or a tautomer thereof, or a pharmaceutically acceptable salt, hydrate or solvate thereof, or pharmaceutical compositions according to the second aspect of the invention is provided, in the preparation of pharmaceutical compositions for preventing and/or treating diseases related to TRK function abnormalities (abnormal activation functions induced by TRK gene amplification, overexpression, mutation or gene fusion).

In another preferred embodiment, the disease is selected from the group consisting of cancer, proliferative disease, pain, skin disease or condition, metabolic disease, muscle disease, neurological disease, autoimmune disease, itching caused by dermatitis.

In the fourth aspect of the invention, an TRK inhibitor is provided, which comprises the compound, or a stereoisomer thereof, a tautomer thereof, or a pharmaceutically acceptable salt, hydrate or solvent thereof of the first aspect of the present invention.

It should be understood that, in the present invention, each of the technical features specifically described above and below (such as those in the Examples) can be combined with each other, thereby constituting new or preferred technical solutions which need not be specified again herein .

### DESCRIPTION OF THE DRAWINGS

Figure 1 is the inhibition rate (%) of compounds tested in cellular assays;
Figure 2 shows the curve of mice tumor volume vs time after administration of the compound in the mouse model test.

### EMBODIMENTS FOR CARRYING OUT THE INVENTION

### Terms

Unless otherwise defined, all technical and scientific terms used herein have the same meaning as commonly understood by one of ordinary skill in the art to which this invention belongs.

As used herein, when used in reference to a particular recited value, the term "about" means that the value can vary by no more than 1% from the recited value. For example, as used herein, the expression "about 100" includes all the values between 99 and 101 (e.g., 99.1, 99.2, 99.3, 99.4, etc.).

As used herein, the terms "containing" or "including (comprising)" may be an open-ended form, semi-close-ended form, or close-ended form. In other words, the terms also include "essentially consisting of..." or "consisting of...".

### DEFINITIONS

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyls having 2-6 carbon atoms, such as ethynyl, propynyl, butynyl, and the like.

As used herein, the term "C₃-C₈ cycloalkyl" refers to cycloalkyl groups having 3 to 8 carbon atoms. It may be a monocyclic ring, such as cyclopropyl, cyclobutyl, cyclopentyl, cyclohexyl, and the like. It may also be of a bicyclic form, such as a bridged or spiro ring form.

As used herein, the term "C₁-C₈ alkoxy" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-12 membered heterocycloalkyl comprising 1-3 heteroatoms selected from the group consisting of N, S and O" refers to a saturated or partially saturated cyclic group comprising 3-12 atoms, among which 1-3 atoms are heteroatoms selected from the group consisting of N, S and O. It may be a monocyclic ring or bicyclic form, such as a bridged or spiro ring form. Specific examples may be oxetane, azetidine, tetrahydro-2H-pyranyl, piperidinyl, tetrahydrofuranyl, morpholinyl and pyrrolidinyl, and the like.

As used herein, the term "C₆-C₁₀ aryl" refers to aryl groups comprising 6 to 10 carbon atoms, such as phenyl, naphthyl, and the like.

As used herein, the term "5-10 membered heteroaryl comprising 1-3 heteroatoms selected from the group consisitng of N, S and O" refers to cyclic aromatic groups comprising 5-10 atoms, among which 1-3 atoms are heteroatoms selected from the group consisting of N, S and O. It may be a monocyclic ring or fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, etc.

Unless otherwise specified, all the groups of the present invention may be substituted with a substituent selected from the group consisting of a halogen, nitrile, nitro, hydroxy, amino, C₁-C₆ alkyl-amine group, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

As used herein, "halogen" or "halogen atom" refers to F, Cl, Br, or I. More preferably, said halogen or halogen atom is selected from F, Cl or Br. "Halogenated" means that a group is substituted by atoms selected from the group consisting of F, Cl, Br and I.

Unless otherwise specified, the structural formula described herein are intended to include all isomeric forms (such as enantiomeric, diastereomeric, and geometric isomers (or conformational isomers)): for example, R, S configuration of asymmetrical centers, (Z), (E) isomers of double bonds, etc. Therefore, a mixture of single stereochemical isomers or enantiomers, diastereomers or geometric isomers (or conformers) of the compounds of the invention falls within the scope of the invention.

As used herein, the term "tautomer" means that structural isomers having different energies can exceed the low energy barrier and thereby transform between each other. For example, proton tautomers (proton shift) includes interconversion by proton transfer, such as 1H-carbazole and 2H-carbazole. Valence tautomers include interconversion through the recombination of some bonding electrons.

As used herein, the term "solvate" refers to a complex formed by a compound of the invention coordinating to a solvent molecule at a specific ratio.

### Compound of Formula I

The present invention provides a compounds as shown in Formula I: 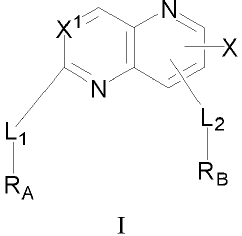 wherein,
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is selected from the group consisting of a substituted or unsubstituted -(X⁴)_{z}-, wherein each of the X⁴ is independently selected from the group consisting of a substituted or unsubstituted C₁-C₈ alkylene, -O-, -C(=O)-, -S-, -S(=O)-, -S(=O)₂-, -NH-, -CONH-, -NHCO-, -NHCONH-, -NHS(=O)-, -NHS(=O)₂-;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 heteroatoms selected from N, S or O, substituted or unsubstituted 5-10 membered heterocyclic group comprising 1-3 heteroatoms selected from N, S or O (including a monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amide (-C(=O)-N(Rc)₂ or -NH-C(=O)(Rc), Rc is H or C1-C5 alkyl), C1-C6 alkyl-(C1-C6 amide), 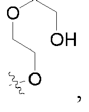 or a substituted or unsubstituted group selected from the group consisting of a C1-C6 alkyl, C3-C8 cycloalkyl, C1-C6 amido, C6-C10 aryl, 5-10 membered heteroaryl comprising 1-3 heteroatoms selected from N, S, or O, 5-12 membered heterocyclic ring group comprising 1-3 heteroatoms selected from N, S or O (including a monocyclic, bicyclic, spiro or bridged ring), -(CH₂)-C6-C10 aryl, -(CH₂)-(5-10 membered heteroaryl having 1-3 heteroatoms selected from N, S, or O), wherein the substituent is selected from the group consisting of a halogen, C1-C6 alkyl, C1-C6 alkoxy, oxo, -CN, -NH₂, -OH, C6-C10 aryl, C1-C6 amino, C1-C6 amido, 5-10 membered heteroaryl comprising 1-3 heteroatoms selected from N, S, or O;
or R_{A} together with -L₂-R_{B} form -Art¹-L₄-L₃-; wherein L₃ is selected from the group consisting of a substituted or unsubstituted -(X⁴)_{z}-, wherein each of the X⁴ is independently selected from the group consisting of a substituted or unsubstituted C₁-C₈ alkylene, -O-, -C(=O)-, -S-, -S(=O)-, -S(=O)₂-, -NH-, -CONH-, -NHCO-, -NHCONH-, -NHS(=O)-, -NHS(=O)₂-;
L₄ is selected from the group consisting of a substituted or unsubstituted -(X⁵)_{w}-, wherein each X⁵ is independently selected from the group consisting of a substituted or unsubstituted C₁-C₈ alkylene, -O -, -C(=O)-, -S-, -S(=O)-, -S(=O)₂-, -NH-, -CONH-, -NHCO-, -NHCONH-, -NHS(=O)-, -NHS(=O)₂-, substituted or unsubstituted C3-C8 cycloalkylene, substituted or unsubstituted 5-10 membered heteroarylene comprising 1-3 heteroatoms selected from N, S, or O, sbstituted or unsubstituted 5-12 membered heterocycloalkylene group comprising 1-3 heteroatoms selected from N, S, or O;
z and w are each independently selected from the group consisiting of 1, 2, 3, 4, 5, 6 and 7;
and the sum of z and w is ≤ 10;
Art¹ is selected from the group consisting of a substituted or unsubstituted phenyl ring, substituted or unsubstituted 5-10 membered heteroaryl comprising 1-3 heteroatoms selected from N, S or O; 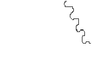 is the connection site of the group;
with the proviso that compounds of formula I are chemical stable structures.

In another preferred embodiment, X¹, L₁, L₂, R_{A} and R_{B} are each independently the corresponding group of the compound in the examples.

In another preferred embodiment, the compound of the present invention has a structure as shown in the following formula: 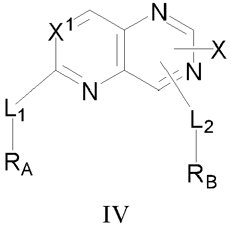 wherein,
X is H, halogen, CN or -CONH₂;
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is a substituted or unsubstituted 5-10 membered heterocycloalkylene group comprising 1-3 heteroatoms selected from N, S or O;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 heteroatoms selected from N, S or O, substituted or unsubstituted 5-10 membered heterocyclic group comprising 1-3 heteroatoms selected from N, S or O (including a monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amido (-C(=O)-N(Rc)₂ or -NH-C(=O)(Rc), Rc is H or C1-C5 alkyl), C1-C6 alkyl-(C1-C6 amido), 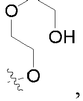
or a substituted or unsubstituted group selected from the group consisting of a C1-C6 alkyl, C3-C8 cycloalkyl, C1-C6 amido, C6-C10 aryl, 5-10 membered heteroaryl comprising 1-3 heteroatoms selected from N, S, or O, 5-12 membered heterocyclic ring group comprising 1-3 heteroatoms selected from N, S or O (including a monocyclic, bicyclic, spiro or bridged ring), -(CH₂)-C6-C10 aryl, -(CH₂)-(5-10 membered heteroaryl comprising 1-3 heteroatoms selected from N, S, or O), wherein the substituent is selected from the group consisting of a halogen, C1-C6 alkyl unsubstituted or unsubstituted by one or more hydroxyls, C1-C6 alkoxy, oxo, -CN, -NH₂, -OH, C6-C10 aryl, C1-C6 amino, C1-C6 amido, 5-10 membered heteroaryl comprising 1-3 heteroatoms selected from N. S. or O; 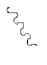
is the connection site of the group;
with the proviso that compounds of formula I are chemical stable structures.

In another preferred embodiment, the compound of formula I is a compound shown in the table below.

| Compound | Structure | Compound | Structure |
|---|---|---|---|
| Example 1 | 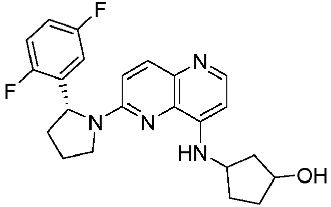 | Example 53 | 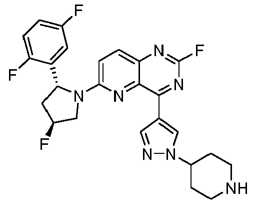 |
| Example 2 | 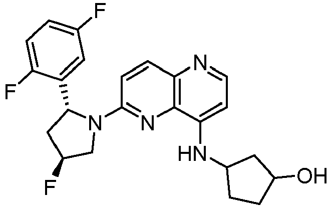 | Example 54 | 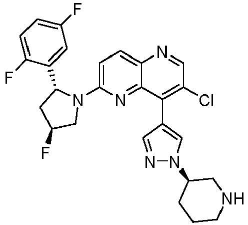 |
| Example 3 | 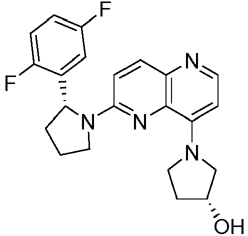 | Example 55 | 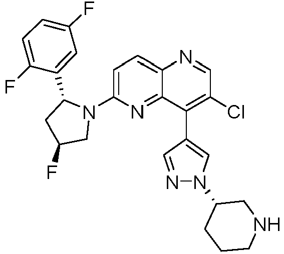 |
| Example 4 | 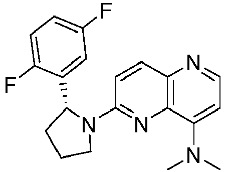 | Example 56 | 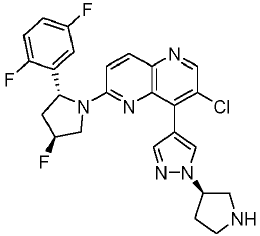 |
| Example 5 | 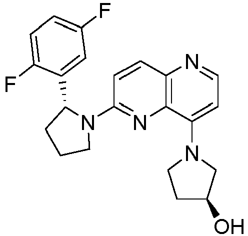 | Example 57 | 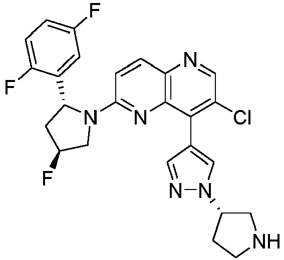 |
| Example 6 | 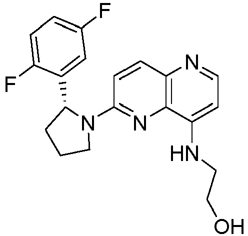 | Example 58 | 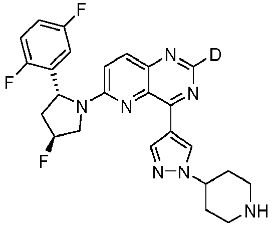 |
| Example 7 | 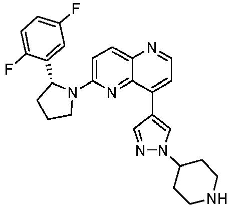 | Example 59 | 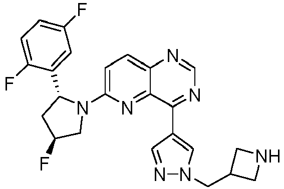 |
| Example 8 | 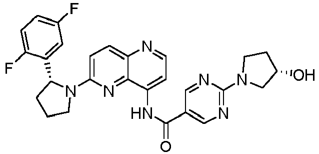 | Example 60 | 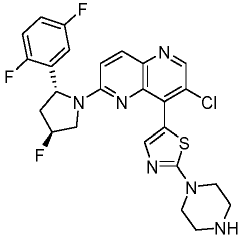 |
| Example 9 | 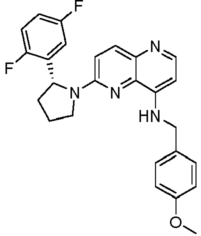 | Example 61 | 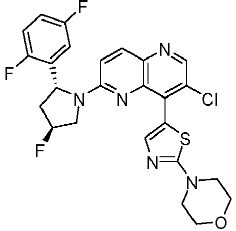 |
| Example 10 | 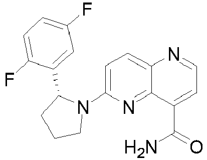 | Example 62 | 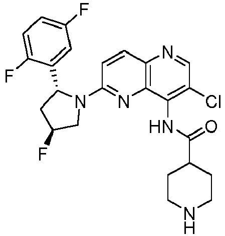 |
| Example 11 | 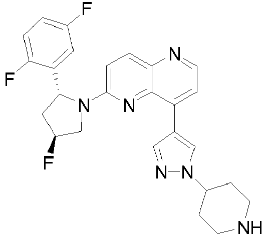 | Example 63 | 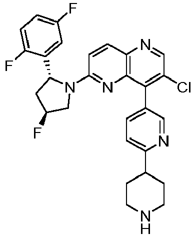 |
| Example 12 | 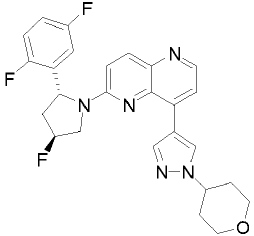 | Example 64 | 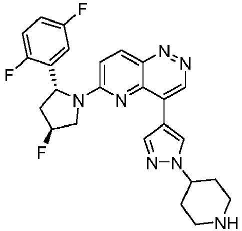 |
| Example 13 | 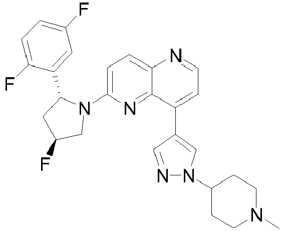 | Example 65 | 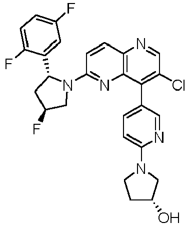 |
| Example 14 | 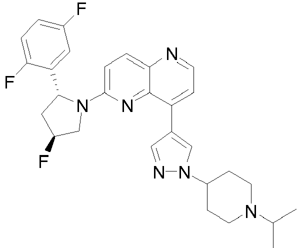 | Example 66 | 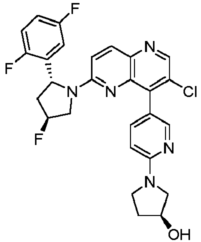 |
| Example 15 | 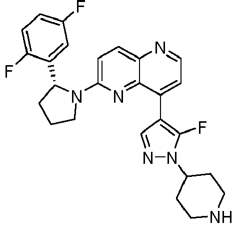 | Example 67 | 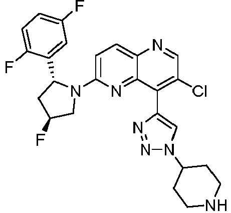 |
| Example 16 | 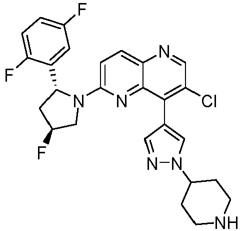 | Example 68 | 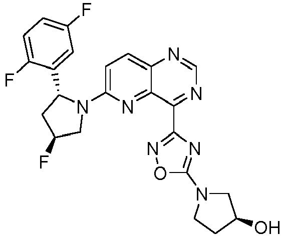 |
| Example 17 | 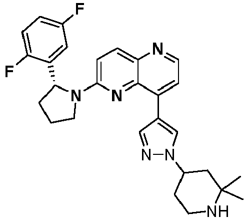 | Example 69 | 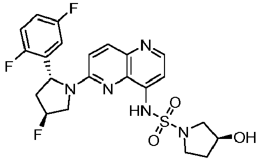 |
| Example 18 | 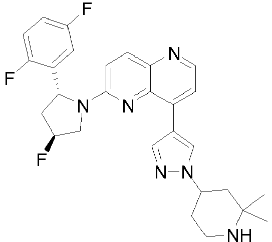 | Example 70 | 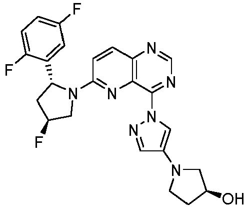 |
| Example 19 | 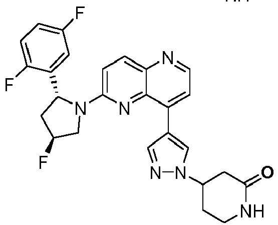 | Example 71 | 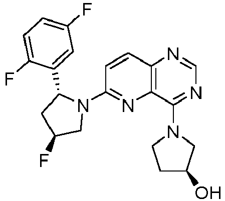 |
| Example 20 | 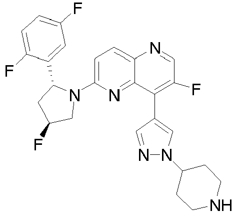 | Example 72 | 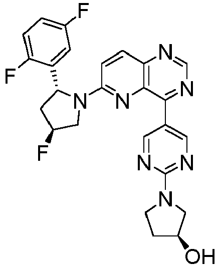 |
| Example 21 | 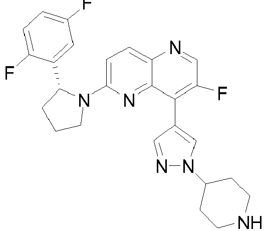 | Example 73 | 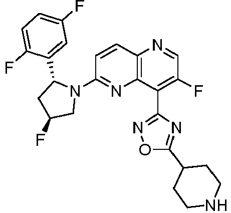 |
| Example 22 | 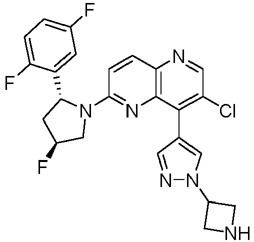 | Example 74 | 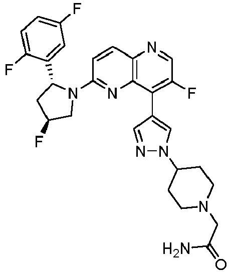 |
| Example 23 | 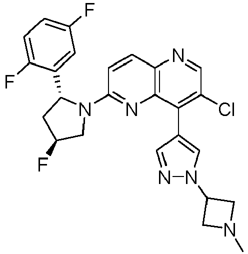 | Example 75 | 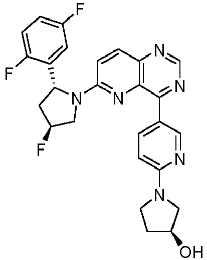 |
| Example 24 | 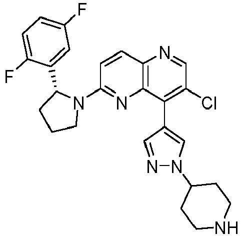 | Example 76 | 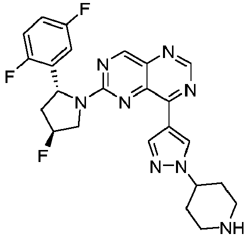 |
| Example 25 | 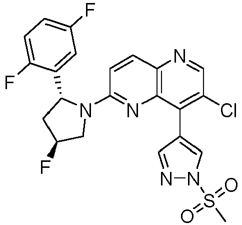 | Example 77 | 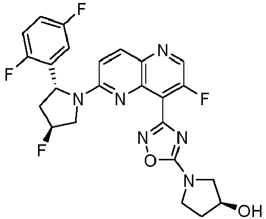 |
| Example 26 | 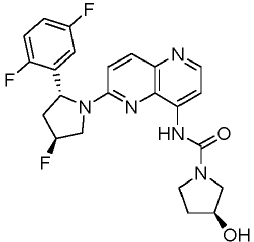 | Example 78 | 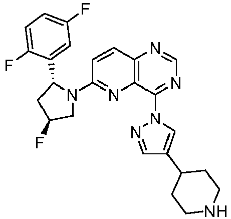 |
| Example 27 | 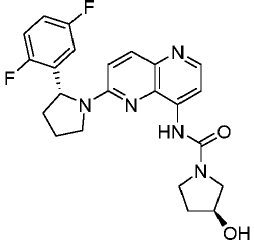 | Example 79 | 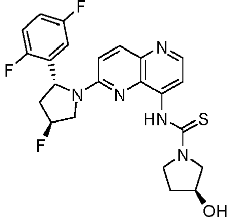 |
| Example 28 | 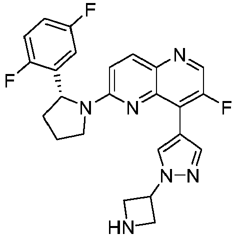 | Example 80 | 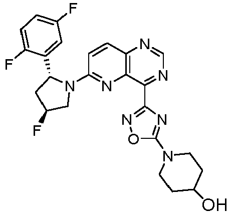 |
| Example 29 | 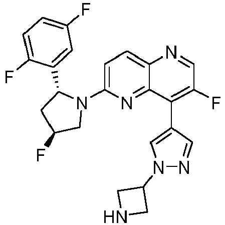 | Example 81 | 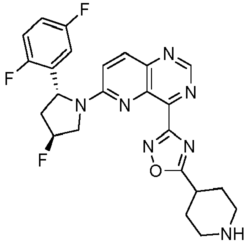 |
| Example 30 | 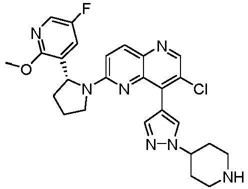 | Example 82 | 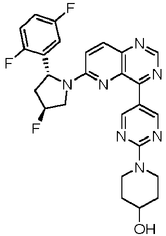 |
| Example 31 | 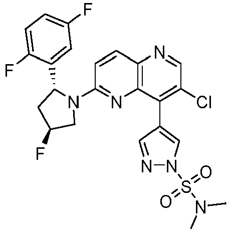 | Example 83 | 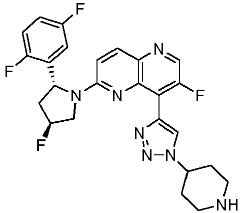 |
| Example 32 | 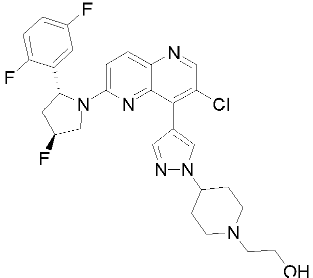 | Example 84 | 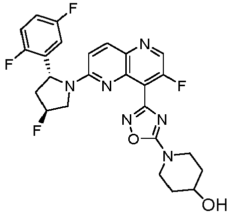 |
| Example 33 | 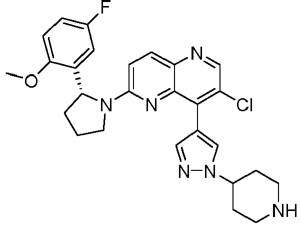 | Example 85 | 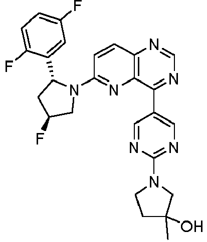 |
| Example 35 | 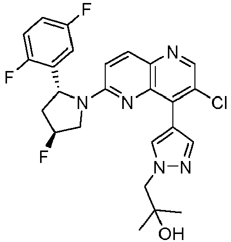 | Example 86 | 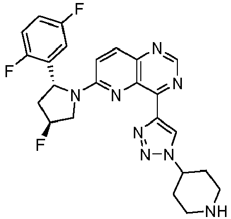 |
| Example 36 | 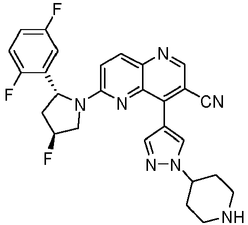 | Example 87 | 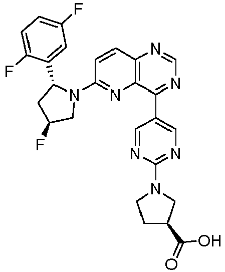 |
| Example 37 | 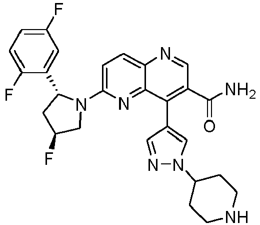 | Example 88 | 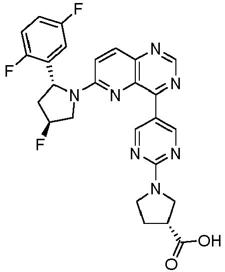 |
| Example 38 | 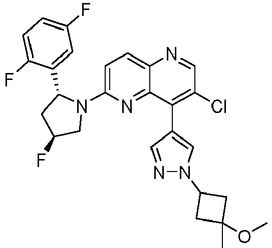 | Example 89 | 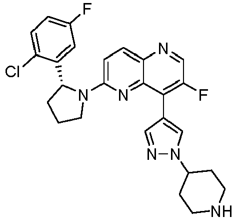 |
| Example 39 | 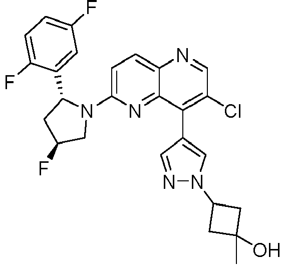 | Example 90 | 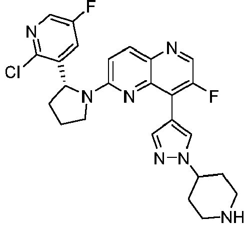 |
| Example 40 | 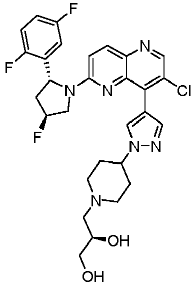 | Example 91 | 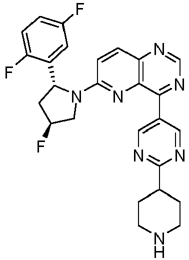 |
| Example 41 | 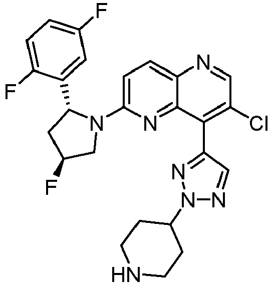 | Example 92 | 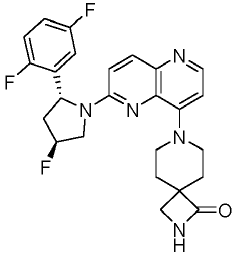 |
| Example 43 | 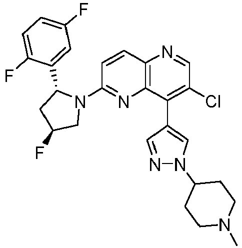 | Example 93 | 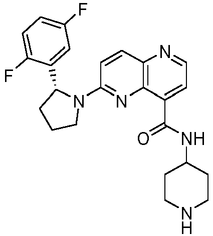 |
| Example 44 | 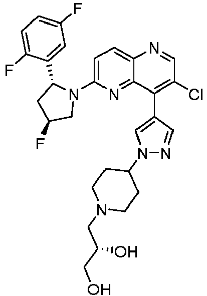 | Example 94 | 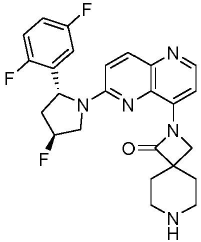 |
| Example 45 | 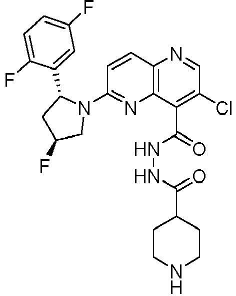 | Example 95 | 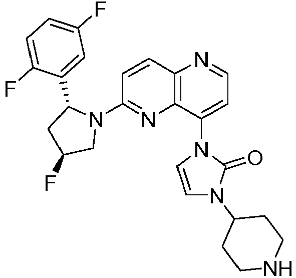 |
| Example 46 | 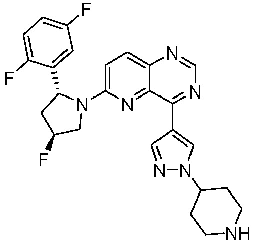 | Example 96 | 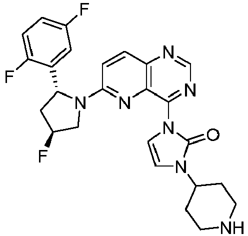 |
| Example 47 | 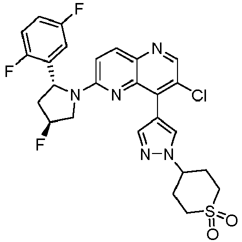 | Example 97 | 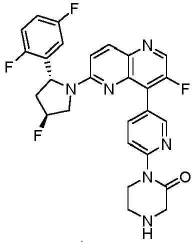 |
| Example 48 | 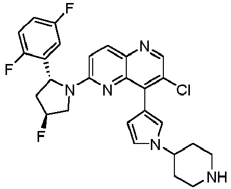 | Example 98 | 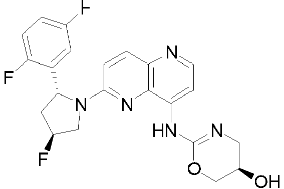 |
| Example 49 | 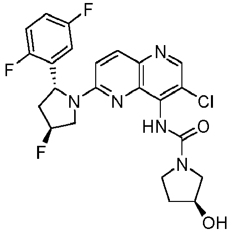 | Example 99 | 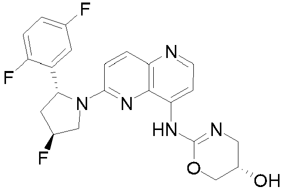 |
| Example 50 | 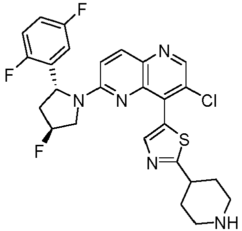 | Example 100 | 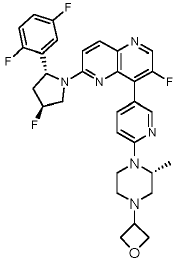 |
| Example 51 | 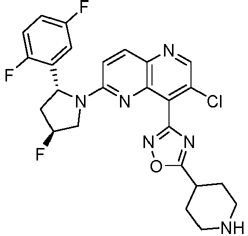 | Example 101 | 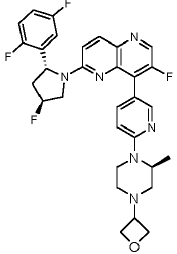 |
| Example 52 | 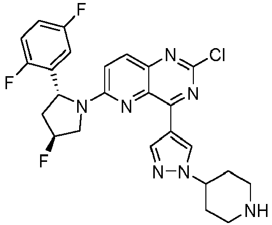 | | |

### Preparation of Compound of Formula I

The compound of the formula I of the present invention can be prepared by the following method: 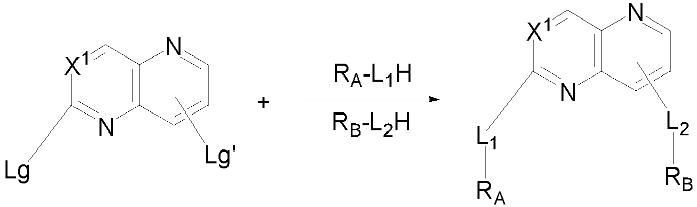 wherein LG and LG' are leaving groups, preferably Tf, fluorine, chlorine, bromine or iodine.

### PHARMACEUTICAL COMPOSITION AND ADMINISTRATION THEREOF

The compounds of the present invention possess outstanding activity of inhibiting TRK kinase. Therefore, the compound of the present invention, and the crystal forms, pharmaceutically acceptable inorganic or organic salts, hydrates or solvates thereof, and the pharmaceutical composition comprising the compound of the present invention as a main active ingredient can be used for preventing or treating diseases related to activity or expression of TRK kinase (e.g., cancers).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg of the compound of the invention per dose, preferably, 10-200 mg of the compound of the invention per dose. Preferably, the "dose" is a capsule or tablet.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

There is no special limitation on administration mode for the compound or pharmaceutical compositions of the present invention, and the representative administration mode includes (but is not limited to): oral, parenteral (intravenous, intramuscular or subcutaneous) administration.

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or a combination thereof.

Besides these inert diluents, the composition may also contain additives such as wetting agents, emulsifiers, and suspending agent, sweetener, flavoring agents and perfume.

In addition to the active compounds, the suspension may contain a suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds.

In the case of co-administration, the pharmaceutical composition can also include one or more other pharmaceutically acceptable compounds. The one or more other pharmaceutically acceptable compounds may be administered simultaneously, separately or sequentially with the compound of the present invention.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1-2000 mg, preferably 20-500mg. Of course, the particular dose should also depend on various factors, such as the route of administration, patient healthy status, which are well within the skills of an experienced physician.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

### Synthesis of Intermediate A:

### (R)-8-chloro-2-(2-(2,5-difluorophenyl)pyrrolidin-l-yl)-l,5-naphthyridin

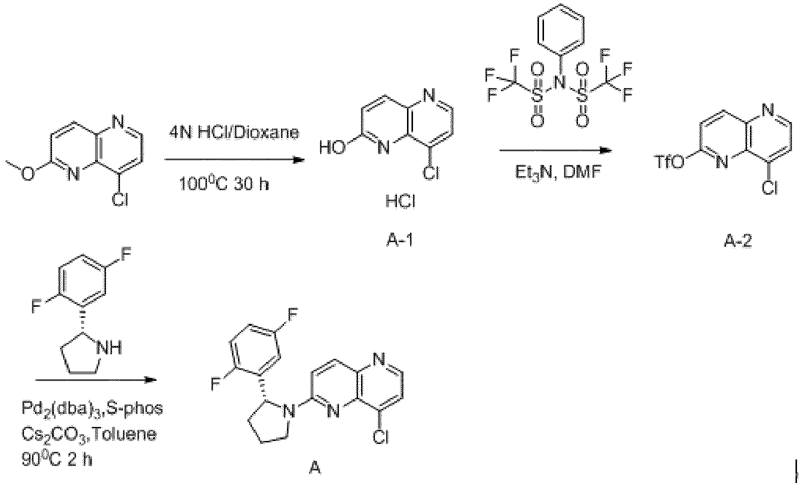

### 8-Chloro-[1,5]naphthyridin-2-ol hydrochloride

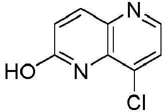

4 N hydrochloride in dioxane (60 mL) was added to 8-chloro-2-methoxy-1,5-naphthyridin (900 mg, 4.62 mmol). The mixture were heated to 100 °C and stirred for 30 hours. The reaction solution was concentrated to provide the title compound 8-chloro-1,5-naphthyridin-2-ol hydrochloride (1.17 g, yield 100%) as a white solid.

MS (ESI): m/z = 181 [M+H] +

### 8-Chloro-1,5-naphthyridin-2-yl trifluoromethanesulfonate

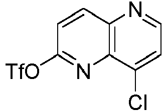

N-phenyl bis(trifluoromethanesulfonyl)imide (2.48 g, 6.93 mmol) was added to the solution of 8-chloro-1,5-naphthyridin-2-ol hydrochloride (1.17 g, 4.62 mmol) and triethylamine (3.2 ml, 23.1 mmol) in N,N-dimethylformamide (28ml), and the resulting mixture was stirred at room temperature for 1h. Water (100 mL) was added and the mixture was extracted with ethyl acetate twice (100 mL*2). The combined organic phase was dried and concentrated, and purified by column chromatography (petroleum ether: ethyl acetate = 2: 1) to afford the title compound 8-chloro-1,5-naphthyridin-2-yl trifluoromethanesulfonate. (1.4 g, 97.0%) as a white solid.

MS (ESI): m/z = 313 [M+H]⁺

### (R)-8-chloro-2-(2-(2,5-difluorophenyl)pyrrolidin-1-yl)-1,5-naphthyridin

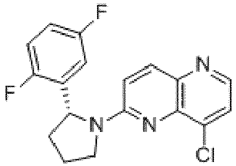

A mixture of 8-chloro-1,5-naphthyridin-2-yl trifluoromethanesulfonate (500 mg, 1,60 mmol), (R)-2-(2,5-difluorophenyl)pyrrole (293 mg, 1.60 mmol), cesium carbonate (1.04 g, 3.20 mmol), bis(dibenzylideneacetone)palladium (146 mg, 0.16 mmol) and 2-dicyclohexylphosphine-2',6'-dimethoxy-biphenyl (131 mg, 0.32 mmol) was stirred at 90°C under N₂ atmosphere for 2 hours. Water (100 mL) was added, and extracted with dichloromethane twice (100 mL*2). The combined organic phase was dried, concentrated, and purified by silica chromatography (petroleum ether: ethyl acetate = 4: 1) to afford the title compound (R)-8-chloro-2-(2-(2,5-difluorophenyl)pyrrolidin-l-yl)-l,5-naphthyridin (257 mg, yield of 46.5%) as a yellow oil.

MS (ESI): m/z = 346[M+H]+.

### Synthesis of intermediate B

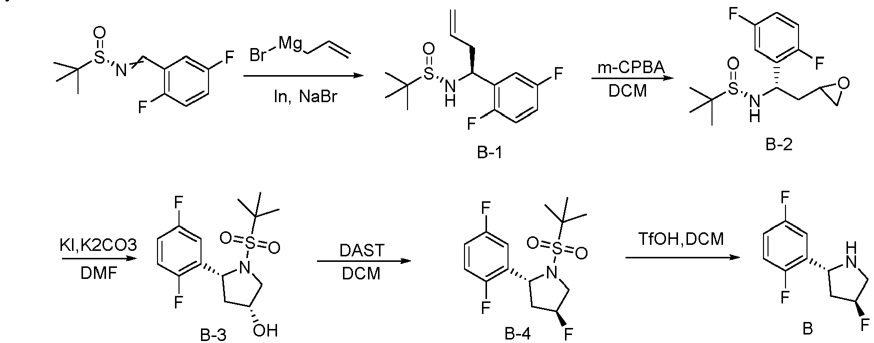

### (S)-N-((S)-1-(2,5-difluorophenyl)but-3-en-1-yl)-2-methylpropan-2-sulfenamide

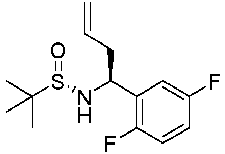

(R)-N-(2,5-difluorobenzyliden)-2-methylpropan-2-sulfinamide (30 g, 122.45 mmol) was added to an aqueous solution of saturated sodium bromide (480 mL) at room temperature. Indium (42 g, 367.35 mmol) was added, followed by the addition of allyl magnesium bromide (42 ml, 489.8 mmol). The mixture was stirred at room temperature for 6h. TLC showed that the reaction was completed, then the solution was quenched with saturated sodium bicarbonate and filtered. The filtrate was extracted with ethyl acetate, washed with saturated brine, dried with anhydrous sodium sulfate, and concentrated to afford (S)-N-((S)-1-(2,5-difluorophenyl)but-3-en-1-yl)-2-methylpropan-2-sulfenamide as yellow solid (35 g).

### (S)-N-((1S)-1-(2,5-difluorophenyl)-2-(oxiran-2-yl)ethyl)-2-methylpropan-2-sulfinylamid e

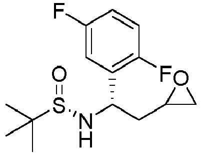

(S)-N-((S)-1-(2,5-Difluorophenyl)but-3-en-1-yl)-2-methylpropan-2-sulfenamide (35 g, 121.95 mmol) was dissolved in dichloromethane (800 mL), and 3-chloroperbenzoic acid (80 g, 365.85 mmol) was added in batches at room temperature and the resulting mixture was stirred at room temperature overnight. TLC showed that the reaction was completed, and the mixture was washed sequentially with saturated sodium bicarbonate, saturated sodium thiosulfate, saturated brine, and dried over anhydrous sodium sulfate, and the filtrate was concentrated to afford
(S)-N-((S)-1-(2,5-difluorophenyl)but-3-en-1-yl)-2-methylpropan-2-sulfenamide as yellow solid (31 g, yield: 79%).

### (3R,5R)-1-(tert-butylsulfonyl)-5-(2,5-difluorophenyl)pyrrolidin-3-ol

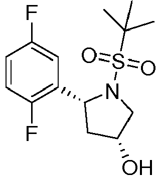

A mixture of (S)-N-((S)-1-(2,5-difluorophenyl)but-3-en-1-yl)-2-methylpropan-2-sulfenamide (31g, 97.18 mmol), potassium carbonate (40 g, 291.53 mmol) and potassium iodide (16 g, 97.18 mmol) in N,N-dimethylformamide (300 mL), then was stirred at 100°C for 1h. TLC showed that the reaction was completed. The reaction solution was cooled to room temperature and filtered. The filtrate was poured into water and extracted with ethyl acetate. The combined organic phase was washed with saturated brine, dried with anhydrous sodium sulfate, and the filtrate was concentrated and purified by silica chromatography(petroleum ether/ethyl acetate = 10/1-5/1) to afford (3R,5R)-1-(tert-butylsulfonyl)-5-(2,5-difluorophenyl)pyrrolidin-3-ol (7.5 g).

### (2R,4S)-2-(2,5-difluorophenyl)-4-fluorpyrrolidine

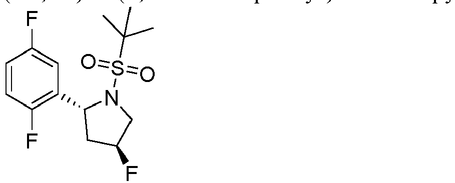

(3R,SR)-1-(tert-butylsulfonyl)-5-(2,5-difluorophenyl)pyrrolidin-3-ol (2.0 g, 6.27 mmol) was dissolved in dichloromethane (50 mL), cooled to -60 °C, then DAST (2 mL) was added to the mixture. The mixture was spontaneously warmed to room temperature and stirred overnight, LCMS showed that the reaction was completed. The reaction solution was diluted with dichloromethane, and slowly poured into ice water. The organic phase was separated, washed with saturated brine and dried with anhydrous sodium sulfate, then the filtrate was concentrated and purified by silica chromatography(petroleum ether/ethyl acetate=10/1) to afford (2R,4S)-1-(tert-butylsulfonyl)-2-(2,5-difluorophenyl)-4-fluorine asyellow solid (1.2 g, yield: 60%).

### (2R,4S)-2-(2,5-difluorophenyl)-4-fluoropyrrolidine

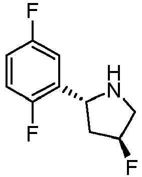

Trifluoromethanesulfonic acid (0.7 mL) was added dropwise to a mixture of (2R,4S)-1-(tert-butylsulfonyl)-2-(2,5-difluorophenyl)-4-fluorine (500 mg, 1.55 mmol) in dichloromethane (20 mL) at room temperature and the mixture was stirred at room temperature for 2h. The solvent was concentrated, and residue was washed with 2M sodium hydroxide and extracted with ethyl acetate, and the organic phase was separated, washed with saturated brine, dried over anhydrous sodium sulfate, the filtrate was concentrated and purified by silica chromatography (petroleum ether/ethyl acetate = 4/1) to afford (2R,4S)-2-(2,5-difluorophenyl)-4-fluoropyrroline as yellow solid (305 mg, yield: 99%).

### Synthesis of intermediate C

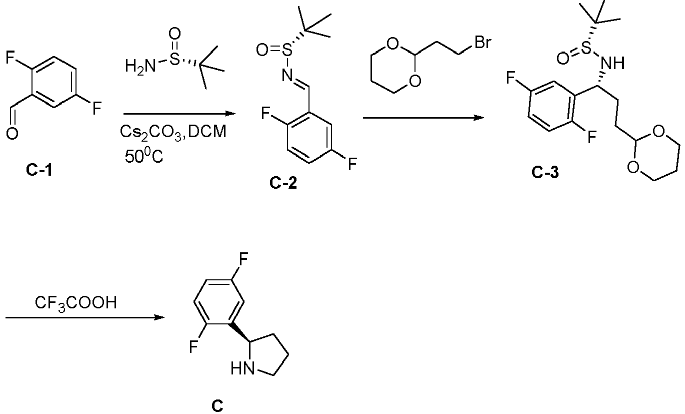

### (R)-N-(2,5-Difluorobenzylidene)-2-methylpropan-2-sulfinamide

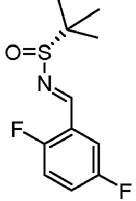

2,5-Difluorobenzaldehyde (5 g, 35.2 mmol) and (R)-2-methylpropan-2-sulfinamide (4.47 g, 36.9 mmol) were dissolved in dichloromethane (50 mL), and cesium carbonate (8.0 g, 24.6 mmol) was added at room temperature. The solution was warmed to 50 °C to react for 3h. TLC showed that the reaction was completed. The solution was filtered, the residue was washed with dichloromethane, and the filtrate was washed with brine, dried with Na₂SO₄, and the filtrate was concentrated to afford (R)-N-(2,5-difluorzylmethylene)-2-methylpropane-2-sulfamide as a yellow oily liquid (9g).

### (R)-N-((R)-1-(2,5-Difluorophenyl)-3-(1,3-dioxan-2-yl)propyl)-2-methylpropane-2-sulfi namide

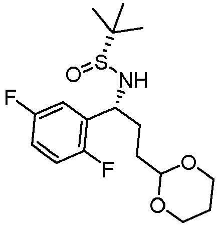

To a suspension of magnesium granules (2 g, 83.3 mmol) in dry tetrahydrofuran (72 mL) under nitrogen atomsphere, Dibal-H (0.1 mL, 1.5 m, 0.15 mmol) was added dropwise, and the mixture was allowed to react at 40°C for 0.5h. Then 2-(2-bromoethyl)-1,3-dioxane (14.3 g, 73.47 mmol) in tetrahydrofuran (40 ml) was slowly added to the system and the temperature was controlled at 40-50 °C, and upon addition, the system was kept at 40 °C and stirred for 1h. The reaction system was cooled to -30 °C, then
(R,E)-N-(2,5-difluorobenzylidene)-2-methylpropan-2-sulfinamide (9 g, 36.73 mmol) in tetrahydrofuran (40 mL) was added dropwise to the mixture, and the temperature was controlled at -30 °C -20 °C. After addition, the mixture was stirred at -30 °C for 2h. TLC showed that the reaction was completed, and the reaction was quenched with 10% aqueous citric acid solution. The temperature was controlled at 10 °C. After extracted with dichloromethane, the organic phase was washed with saturated brine, dried with Na₂SO₄ and the filtrate was concentrated to afford
(R)-N-((R)-1-(2,5-difluorophenyl)-3-(1,3-dioxan-2-yl)propyl)-2-methylpropane-2-sulfinamid e as colorless oily liquid (15.8 g).

### (R)-2-(2,5-difluorophenyl)pyrrolidine

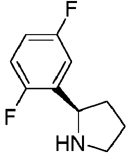

A mixture of (R)-N-((R)-1-(2,5-Difluorophenyl)-3-(1,3-dioxan-2-yl)propyl)-2-methylpropan-2-sulfinamid e (15.8 g, 43.76 mmol) in trifluoroacetic acid (32 mL) and water (8 mL) wasstirred at room temperature for 1h. Then anotherportions of trifluoroacetic acid (60 mL) and triethylsilane (15.2 g, 131.1 mmol) was added dropwise to and the mixture was allowed to react at room temperature for overnight. LCMS showed that the reaction was completed, and most of trifluoroacetic acidwasremoved, and the residue was dissolved in hydrochloric acid (1N, 100 mL) and stirred for 0.5 hours. The resulting mixture was extracted with methyl tert-butyl ether, and the organic phase was washed with hydrochloric acid (1N, 50 mL). The combined aqueous phase was adjusted to pH \around 11 with aqueous sodium hydroxide solution, then extracted with dichloromethane. The combined organic phase was washed with saturated brine and dried over anhydrous sodium sulfate, and the filtrate was concentrated to afford (R)-2-(2,5-difluorophenyl)pyrrolidine as oily liquid (6.7 g).

### Synthesis of intermediate D

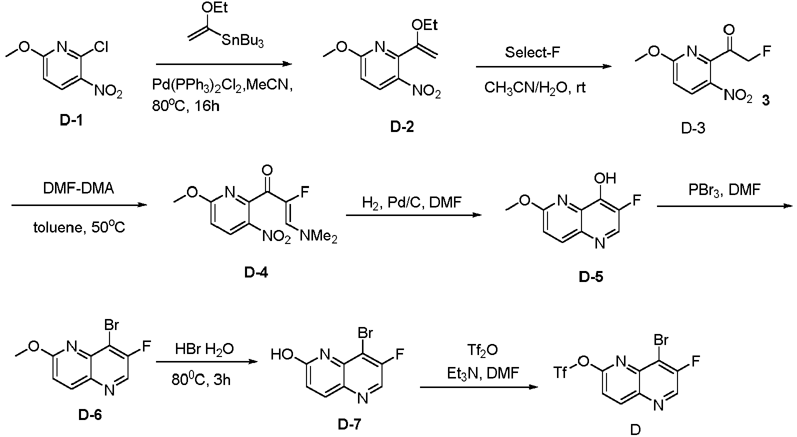

### 2-(1-ethoxyethylene)-6-methoxy-3-nitropyridine

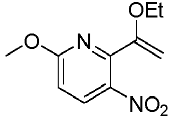

A mixture of 2-chloro-6-methoxy-3-nitropyridine (16.4 g, 86.8 mmol) acetonitrile (150 mL), tributyl(1-ethoxyethylene)tin (37.5 g, 103.9 mmol)andbis triphenylphosphine palladium dichloride (3.05 g, 4.3 mmol) was stirred at 80 °C under N₂ atmosphere for 16 hours. LCMS showed that the reaction was completed. After cooling down to to room temperature. the reaction mixture was poured into ice water/ethyl acetate (150 mL / 100 mL), and extracted with ethyl acetate twice (100 mL x 2). The combined organic layers were washed with brine (100 mL), dried over with anhydrous sodium sulfate and filtered. The filtrate was concentrated under reduced pressure, and purified by silica chromatography (petroleum ether / ethyl acetate = 10/1) to afford pale yellow liquid (29 g, yield 99%).

MS (ESI): m/z = 225 [M+H]+.

### 2-Fluoro-1-(6-methoxy-3-nitropyridin-2-yl)ethanone

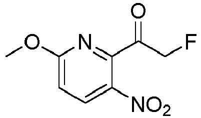

Selective Fluorine reagent (30.75 g, 86.8 mmol) was added to a mixture of 2-(1-ethoxyvinyl)-6-methoxy-3-nitropyridine (23 g, 66.7 mmol) in acetonitrile (100 mL) and water (50 mL).. The mixture was stirred at room temperature for 16 hours. After the reaction was completed, the reaction mixture was poured into ice water (100 mL) and extracted with ethyl acetate twice (100 mL x 2). The combined organic layer was washed with brine (100 mL), dried with anhydrous sodium sulfate and filtered. The filtrate was concentrated and purified by silica chromatography (petroleum ether / ethyl acetate = 6/1), and pulped with petroleum (150 mL) to afford a white solid (3)(13.76 g, yield 75%).

MS (ESI): m/z = 215 [M+H]+.

### 3-(6-Methoxy-3-nitropyridin-2-yl)-3-carbonylpropionitrile

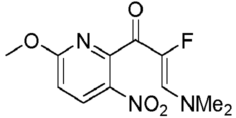

2-Fluoro-1-(6-methoxy-3-nitropyridin-2-yl)ethanone (9.76 g, 45.61 mmol) was dissolved in toluene (60 mL), N,N-dimethylformamide dimethyl acetal (30 mL) was added, and the reaction solution was stirred at 50 °C for 16 h. After cooling down to room temperature, and a large amount of solid was precipitated and filtered. The filter cake was washed with petroleum ether and dried to afford a yellow solid (10.61 g, yield 86%).

MS (ESI): m/z = 270 [M+H]+.

### 3-Fluoro-6-methoxy-1,5-naphthyridin-4-ol

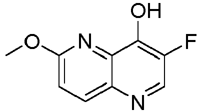

3-(6-Methoxy-3-nitropyridin-2-yl)-3-carbonylpropionitrile (10.61 g, 39.4 mmol) was dissolved in *N,N*-dimethylformamide (50 mL), and 10% Pd/C (3.2 g) was added. After replaced with hydrogen, the reaction solution was stirred under hydrogen atmosphere at 40 °C for 16 h. After the reaction was completed, the solid was filtered , and the filtrate was evaporated under reduced pressure to afford a yellow solid (8.76 g, yield 80%).

MS (ESI): m/z = 195 [M+H]+.

### 8-bromo-7-fluoro-2-methoxy-l,5-naphthyridine

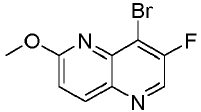

To a mixture of 3-Fluoro-6-methoxy-1,5-naphthyridin-4-ol (8.76 g, 44.9 mmol) in *N,N*-dimethylformamide (50 mL)was added dropwise phosphorus tribromide (14.73 g, 54.3 mmol) at 0 °C. The reaction mixture was stirred at room temperature for 2 h. The reaction mixture was poured into ice water (100 mL), neutralized with saturated sodium bicarbonate to pH ∼ 8, and extracted with ethyl acetate five times(40 mL x 5). The combined organic layer was dried with anhydrous sodium sulfate and filtered. The filtrate was concentrated and t purified by silica chromatography (petroleum ether / ethyl acetate = 6/1) to afford a white solid (**6**)(5.96 g, yield 52%).

MS (ESI): m/z = 257 [M+H]+.

### 8-bromo-7-fluoro-1,5-naphthyridin-2-ol

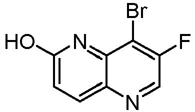

8-Bromo-7-fluoro-2-methoxy-1,5-naphthyridine (5.96 g, 23.2 mmol) was dissolved in hydrobromic acid solution (30 mL), and the reaction mixture was stirred at 80 °C for 3 h. After the reaction was completed, the mixture was neutralized with saturated sodium bicarbonate to pH ∼ 8, and filtered to afford an off-white solid (4.76 g, yield 85%).

### 8-bromo-7-fluoro-1,5-naphthyridin-2-yl trifluoromethanesulfonate

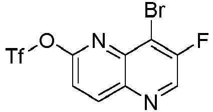

To a mixture of 8-bromo-7-fluoro-1,5-naphthyridin-2-ol (3.0 g, 12.4 mmol), pyridine (2.2 mL, 27.3 mmol) in dichloromethane (50 mL) was added dropwise of Tf₂O (2.5 mL, 14.8 mmol) at 0 °C.The resulting solution was stirred at room temperature for 1 h. After the reaction was completed, the pH of the mixture was adjusted with hydrochloride to ∼ 2, and extracted with dichloromethane (30 mL x 3), and the crude product was purified by silica chromatography (petroleum ether/ethyl acetate = 5/1) to afford a white solid (4.0 g, yield 86%).

MS (ESI): m/z = 375 [M+H]+.

### Synthesis of intermediate E

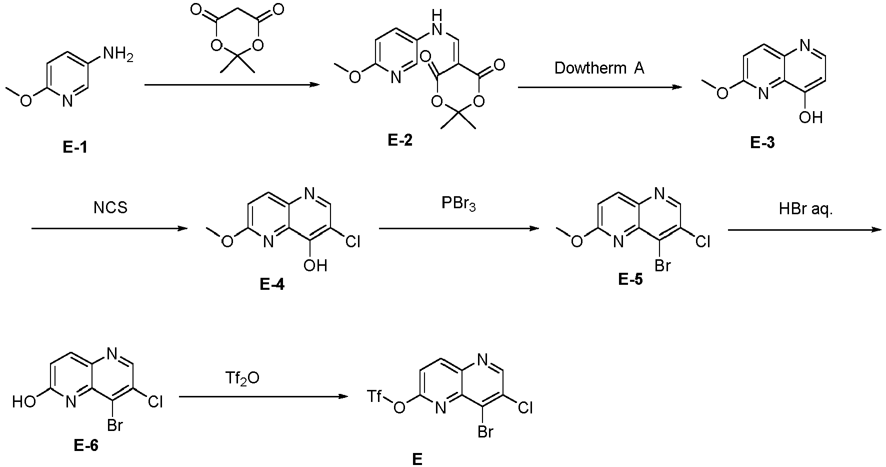

### 5-[(6-Methoxy-pyridin-3-ylamino)-methylene]-2,2-dimethyl-[1,3]dioxane-4,6-dione

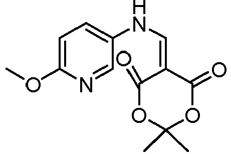

6-methoxypyridin-3-amine (25 g, 201.61 mmol) was dissolved in ethanol (150 mL), and isopropylidene malonate(31.9g, 221.77 mmol) and triethyl orthoformate (29.84 g, 201.61 mmol) were added, and the reaction solution was heated to reflux for 5 h. After the reaction was completed, the reaction mixture was cooled to room temperature, and a large amount of solid was precipitated and filtered. The filter cake was washed with ethanol and dried to afford a dark brown solid (90.4 g, yield 90%).

¹H-NMR (CDCl₃, 400 MHz): δ 8.51 (d, *J =* 14.4Hz, 1H), 8.13 (d, *J =* 2.8 Hz, 1H), 7.54-7.51 (m, 1H), 6.84 (d, *J=* 8.8 Hz, 1H), 3.95 (s, 3H), 1.75 (s, 6H).

### 6-Methoxy-[1,5] naphthyridin-4-ol

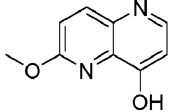

Diphenyl ether-biphenyl eutectic (170 mL) was heated to 190 °C,. 5-[(6-methoxy-pyridine-3-ylamino)-methylene]-2,2-dimethyl-[l,3]dioxan-4,6-dione (17 g, 61.5 mmol) was added to the above solution in batches, then the reaction solution was maintained at 190 °C and stirred for 0.5 h. After the reaction was completed, the reaction solution was cooled to room temperature, and diethyl ether (170 mL) was added, a large amount of solid was precipitated and filtered. The filter cake was washed with diethyl ether and dried to afford a brown solid (6.5 g, yield 60%).

MS (ESI): m/z = 177 [M+H]+.

### 3-chloro-6-methoxy-l,5-naphthyridin-4-ol

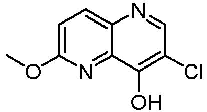

To a mixture of 6-Methoxy-[1,5]naphthyridin-4-ol (19.4 g, 110.23 mmol) in acetic acid (330 mL)N-chlorosuccinimide (16.87 g, 126.76 mmol) was added, and the reaction solution was stirred at 30 °C for 16 h. After the reaction solution was cooled to room temperature, and a large amount of solid was precipitated and filtered. The filter cake was washed with diethyl ether and n-heptane, and dried to obtain an off-white solid (10 g, yield 43%).

MS (ESI): m/z = 211 [M+H]+.

### 8-bromo-7-chloro-2-methoxy-1,5-naphthyridine

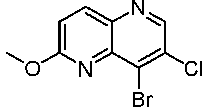

8-Bromo-7-chloro-2-methoxy-1,5-naphthyridine was synthesized using method similar to that for 8-bromo-7-fluoro-2-methoxy-1,5-naphthyridine by replacing the corresponding starting material (1.1 g, yield 84%).

MS (ESI): m/z = 273 [M+H]+.

### 8-bromo-7-chloro-1,5-naphthyridin-2-ol

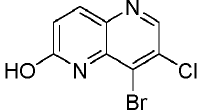

8-Bromo-7-chloro-1,5-naphthyridin-2-ol was prepared using method similar to that for 8-bromo-7-fluoro-1,5-naphthyridin-2-ol by replacing the corresponding starting material (1.6g, yield >100%).

MS (ESI): m/z = 261 [M+H]+.

### 8-bromo-7-chloro -1,5-naphthyridin-2-yl trifluoromethanesulfonate

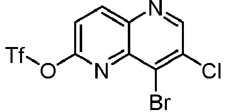

8-Bromo-7-chloro-1,5-naphthyridin-2-yl trifluoromethanesulfonate was prepared using method similar to that for 8-bromo-7-fluoro-1,5-naphthyridin-2-yl trifluoromethanesulfonate by replacing the corresponding starting material (13.3 g, yield 81%).
MS (ESI): m/z = 393 [M+H]+.

### Example 1:

### 3-((6-((R)-2-(2,5-difluorophenyl)pyrrolidin-1-yl)-1,5-naphthyridin-4-yl)amino)cyclopentane-1-ol

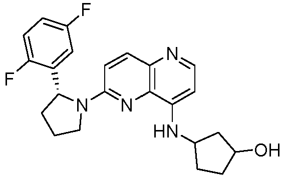

Bis(dibenzylideneacetone)palladium (18 mg, 0.020 mmol) and 2-dicyclohexylphosphin-2',6'-dimethoxy-biphenyl (16 mg, 0.039 mmol) was added to the suspension of (R)-8-chloro-2-(2-(2,5-difluorophenyl pyrrolidin-1-yl)-1,5-naphthyridine (68 mg, 0.197 mmol), 3-aminocyclopentan-l-ol hydrochloride (81 mg, 0.59 mmol), cesium carbonate (321 mg, 0.985 mmol) in toluene (8 mL). The resulting mixture washeated to 110 °C and stirred for 2 h under N₂ atmosphere. The mixture was concentrated and purified by reversed phase preparative chromatography to provide the title compound 3-((6-((R)-2-(2,5-difluorophenyl)pyrrolidin-1-yl)-1,5-naphthyridin-4-yl)amino)cyclopentan-1 -ol (10 mg, yield of 2.4%), as a yellow solid.

MS (ESI): m/z = 411 [M+H]+.

¹H NMR (400 MHz, CD₃OD) δ 8.09 (d, *J* = 7.8 Hz, 1H), 7.88 (d, J=9.6 Hz 1H), 7.21-7.14 (m, 2H), 7.00-6.93 (m, 1H), 6.90-6.80 (m, 1H), 6.75 (d, *J* = 7.0 Hz, 1H), 5.48-5.42 (m, 1H), 4.45-4.37 (m, 1H), 4.30-4.20 (m, 1H), 4.05-3.95 (m, 1H), 3.80-3.70 (m, 1H), 2.60-2.50 (m, 1H), 2.30-2.08 (m, 4H), 2.08-2.00 (m, 1H), 1.99-1.80 (m, 3H), 1.79-1.68 (m, 1H).

### Example 2:

### 3-((6-((2R,4S)-2-(2,5-difluorophenyl)-4-fluoropyrrolidin-l-yl)-l,5-naphthyridin-4-yl)amino) cyclopentan-l-ol

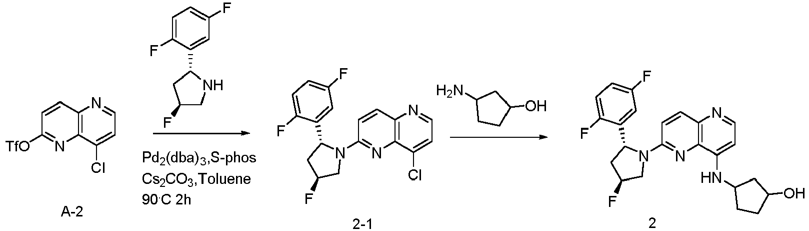

### 8-chloro-2-((2R,4S)-2-(2,5-difluorophenyl)-4-fluoropyrrolidin-l-yl)-l,5-naphthyridine

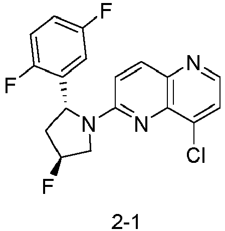

8-chloro-2-((2R,4S)-2-(2,5-difluorophenyl)-4-fluoropyrrolidin-l-yl)-l,5-naphthyridine (137 mg, yield 47.1 %) as a colorless oil was prepared using a method similar to that for (R)-8-chloro-2-(2-(2,5-difluorophenyl)pyrrolidin-1-yl)-1,5-naphthyridineby replacing the corresponding starting material.

MS (ESI): m/z = 364 [M+H]+.

### 3-((6-((2R,4S)-2-(2,5-difluorophenyl)-4-fluoropyrrolidin-l-yl)-l,5-naphthyridine-4-yl)a mino)cyclopentan-l-ol

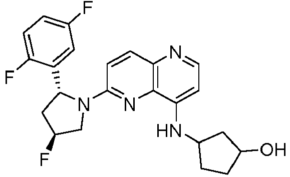

3-((6-((2R,4S)-2-(2,5-difluorophenyl)-4-fluoropyrrolidin-1-yl)-1,5-naphthyridin-4-yl)ami no)cyclopentan-1-ol (10 mg, yield 11.8 %) as a white oil was prepared using a method similar to that in example 1 by replacing the corresponding starting material.

MS (ESI): m/z = 429[M+H]+

¹H NMR (400 MHz, CD₃OD) δ 8.09 (d, *J=* 5.4 Hz, 1H), 7.88 (dd, *J=* 9.2, 2.3 Hz, 1H), 7.19 (m, 1H), 7.10-6.91 (m, 3H), 6.47 (d, *J* = 5.5 Hz, 1H), 5.52-5.37 (m, 2H), 4.39-4.33 (m, 1H), 4.22-4.10 (m, 2H), 4.01-3.93 (m, 1H), 2.93-2.81 (m, 1H), 2.45-2.24 (m, 2H), 2.23-2.06 (m, 2H), 1.96-1.80 (m, 2H), 1.76-1.52 (m, 2H).

### Example 3: (R)-1-(6-((R)-2-(2,5-difluorophenyl)pyrrolidin-1-yl)-1,5-naphthyridin-4-yl)pyrrolidin-3-ol

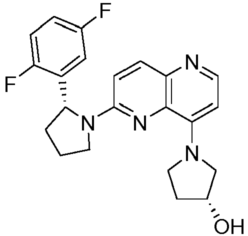

(R)-Pyrrolidin-3-ol hydrochloric acid (38mg, 0.433mmol) was added to a solution of (R)-8-chloro-2-(2-(2,5-difluorophenyl)pyrrolidin-1-yl)-1,5-naphthyridine (50mg, 0.144mmol) and N,N-diisopropylethylamine (93 mg, 0.72 mmol) in N,N-dimethylformamide (2 mL), the resulting mixture was heated to 110 °C and stirred for 16 h. The solution was concentrated and purified by reversed phase preparative chromatography to provide the title compound (R)-1-(6-((R)-2-(2,5-difluorophenyl)pyrrolidin-1-yl)-1,5-naphthyridin-4-yl)pyrrolidin-3-ol (12mg, yield 21.0%)as a yellow solid.

MS (ESI): m/z = 396.9 [M+H]+.

¹H NMR (400 MHz, CD₃OD) δ 8.00 (d, J = 5.6 Hz, 1H), 7.86 (d, *J=* 9.2 Hz, 1H), 7.15-7.09 (m,1H), 7.04-6.88 (m, 2H), 6.82-6.78 (m, 1H), 6.35 (d, *J=* 5.7 Hz, 1H), 5.53 (d, *J=* 8.0 Hz, 1H), 4.35-4.28 (m, 1H), 4.00-3.93 (m, 1H), 3.92-3.83 (m, 2H), 3.67-3.50 (m, 2H), 3.48-3.42 (m, 1H), 2.51-2.38 (m, 1H), 2.15-2.02 (m, 2H), 2.01-1.93 (m, 1H), 1.90-1.82 (m, 2H).

### Example 4:

### (R)-6-(2-(2,5-difluorophenyl)pyrrolidin-1-yl)-N,N-dimethyl-1,5-naphthyridin-4-amine

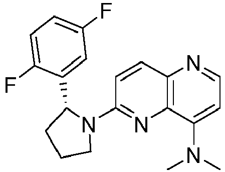

(R)-6-(2-(2,5-Difluorophenyl)pyrrolidin-1-yl)-N,N-dimethyl-1,5-naphthyridin-4-amine (16mg, yield 31.4%) as a yellow oil was prepared using method similar to that in example 3 by replacing the corresponding starting material .

MS (ESI): m/z = 355 [M+H]+.

1H NMR (400 MHz, CD₃OD) δ 8.10 (d, *J* = 5.5 Hz, 1H), 7.90 (d, *J* = 9.2 Hz, 1H), 7.15-7.05 (m, 1H), 7.00 (d, *J* = 9.4 Hz, 1H), 6.99-6.92 (m, 1H), 6.80-6.75 (m, 1H), 6.61 (d, *J* = 5.5 Hz, 1H), 5.51 (d, *J* = 8.4 Hz, 1H), 4.00-3.96 (m, 1H), 3.71-3.63 (m, 1H), 3.07 (s, 6H), 2.52-2.40 (m, 1H), 2.16-2.02 (m, 2H), 2.02-1.94 (m, 1H).

### Example 5: (S)-1-(6-((R)-2-(2,5-difluorophenyl)pyrrolidin-1-yl)-1,5-naphthyridin-4-yl)pyrrolidin-3-ol

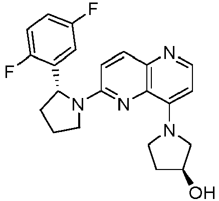

(S)-1-(6-((R)-2-(2,5-Difluorophenyl)pyrrolidin-1-yl)-1,5-naphthyridin-4-yl)pyrrolidin-3-ol was prepared (30mg, yield 52.6 %) as a yellow oil using method similar to that in example 3 by replacing the corresponding starting material.

MS (ESI): m/z = 397[M+H]+.

¹H NMR (400 MHz, CD₃OD) δ 8.00 (d, *J* = 5.6 Hz, 1H), 7.86 (d, *J* = 9.2 Hz, 1H), 7.12 (m, 1H), 7.01-6.89 (m, 2H), 6.80-6.74 (m,, 1H), 6.35 (d, *J* = 5.7 Hz, 1H), 5.49 (d, *J=* 8.1 Hz, 1H), 4.40-4.35 (m, 1H), 4.01-3.91 (m, 2H), 3.90-3.80 (m, 1H), 3.72-3.60 (m,3H), 2.52-2.38 (m, 1H), 2.12-1.88 (m, 5H).

### Example 6: (R)-2-((6-(2-(2,5-Difluorophenyl)pyrrolidin-1-yl)-1,5-naphthyridin-4-yl)amino)ethan-1-ol

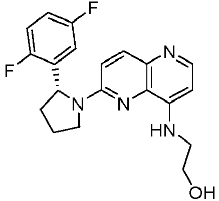

2-aminoethan-1-ol (26mg, 0.433mmol) was added to a solution of (R)-8-chloro-2-(2-(2,5-difluorophenyl)pyrrolidin-1-yl)-1,5-naphthyridine (50mg, 0.144mmol) and N,N-diisopropylethylamine (186 mg, 1.44mmol) in dimethylsulfoxide (2 mL), the resulting mixture was heated to 140 °C and stirred for 24 h. The solution was concentrated and purified by reversed phase preparative chromatography to provide (R)-2-((6-(2-(2,5-difluorophenyl)pyrrolidin-1-yl)-1,5-naphthyridin-4-yl)amino)ethan-1-ol (15 mg, yield of 28.1%)as a yellow solid.

MS (ESI): m/z = 371 [M+H]+.

¹H NMR (400 MHz, CD₃OD) δ 8.07 (d, *J=* 5.4 Hz, 1H), 7.83 (d, *J* = 9.2 Hz, 1H), 7.17-7.08 (m, 1H), 6.95-6.92 (m, 2H), 6.84-6.82 (m, 1H), 6.49 (d, *J=* 5.4 Hz, 1H), 5.42 (d, *J* = 5.2 Hz, 1H), 4.04-3.94 (m, 1H), 3.80-3.67 (m, 3H), 3.45-3.32 (m, 2H), 2.54-2.44 (m, 1H), 2.17-2.07 (m, 2H), 2.04-1.95 (m, 1H).

### Example 7:

### (R)-2-(2-(2,5-Difluorophenyl)pyrrolidin-1-yl)-8-(1-(piperidin-4-yl)-1H-pyrazol-4-yl)-1,5-nap hthyridine

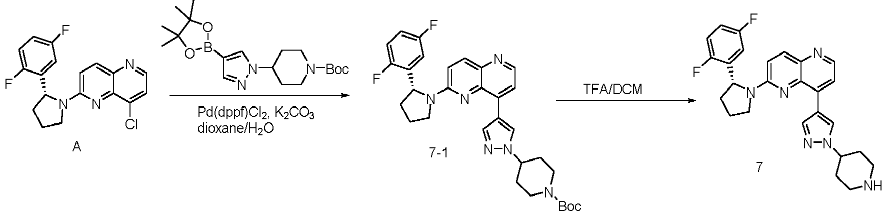

### Tert-butyl

### (R)-4-(4-(6-(2-(2,5-difluorophenyl)pyrrolidin-1-yl)-1,5-naphthyridin-4-yl)-1H-pyrazol-1-yl)p iperidin-1-carboxylate

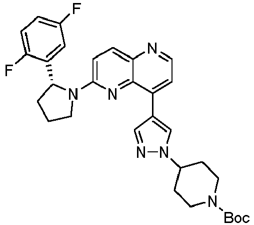

(R)-8-chloro-2-(2-(2,5-difluorophenyl)pyrrolidin-1-yl)-1,5-naphthyridine (50 mg, 0.14 mmol), tert-butyl
4-(4-(4,4,5,5-tetramethyl-1,3,2-dioxaborolan-2-yl)-1H-pyrazol-1-yl)piperidin-1-carboxylate (106 mg, 0.28 mmol), [1,1'-bis(diphenylphosphino)ferrocene]palladium dichloride (5.7 mg, 0.007 mmol) and potassium carbonate (38 mg, 0.28 mmol) were mixed with 1,4-dioxane (1 mL) and water (0.2 mL), the mixture was heated to 100 °C and stirred overnight under nitrogen atmosphere. Water (50 mL) was added and the mixture was extracted with ethyl acetate (50 mL*2). The combined organic phase was dried, filtered, concentrated and purified by column chromatography (petroleum ether: EtOAc=1: 2) to afford a yellow solid (70 mg, yield 89%).

MS (ESI): m/z = 561 [M+H]+.

### (R)-2-(2-(2,5-Difluorophenyl)pyrrolidin-1-yl)-8-(1-(piperidin-4-yl)-1H-pyrazol-4-yl)-1,5 -naphthyridine

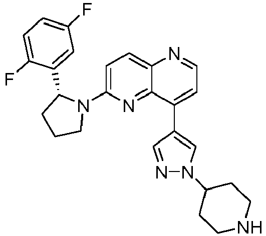

*Tert*-butyl (R)-4-(4-(6-(2-(2,5-difluorophenyl)pyrrolidin-1-yl)-1,5-naphthyridin-4-yl)-1H-pyrazol-1-yl)p iperidin-1-carboxylate (70 mg, 0.12 mmol) was dissolved in dichloromethane (5 mL), and cooled to 0 °C. Trifluoromethanesulfonic acid (0.5 mL) was added and the mixture was allowed to reactat room temperature for 1h. The solution was concentrated, and purified by reversed phase preparative chromatography to afford a white solid (48 mg, yield 87%).

MS (ESI): m/z = 461 [M+H]+.

¹H NMR (400 MHz,DMSO-*d*₆) δ 8.44 (d, *J* = 4.7 Hz, 1H), 8.41-7.87 (m, 3H), 7.77 (d, *J* = 3.9 Hz, 1H), 7.38-7.26 (m, 1H), 7.14-7.05 (m, 1H), 6.91-6.83 (m, 1H), 5.51 (s, 1H), 4.17-3.98 (m, 2H), 3.66 (s, 1H), 3.05 (d, *J=* 12.4 Hz, 2H), 2.61 (t, *J=* 12.1 Hz, 2H), 2.14-2.03 (m, 1H), 2.00-1.73 (m, 5H).

### Example 8:

### N-(6-((R)-2-(2,5-difluorophenyl)pyrrolidin-1-yl)-1,5-naphthyridin-4-yl)-2-((S)-3-hydroxypyr rolidin-1-yl)pyrimidin-5-carboxamide

### Example 9:

### (R)-6-(2-(2,5-Difluorophenyl)pyrrolidin-1-yl)-N-(4-methoxybenzyl)-1,5-naphthyridin-4-ami ne

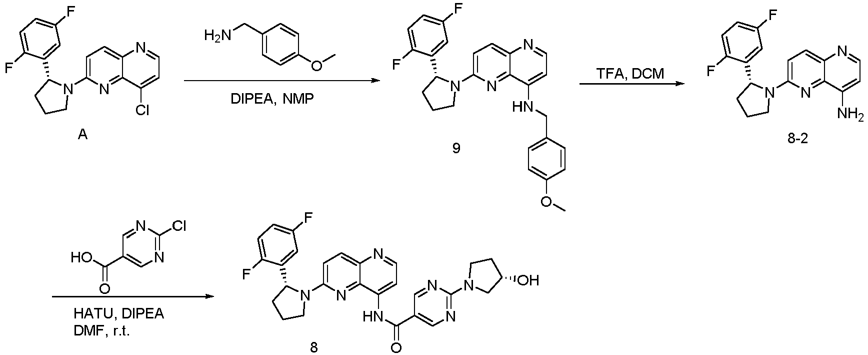

### (R)-6-(2-(2,5-Difluorophenyl)pyrrolidin-1-yl)-N-(4-methoxybenzyl)-1,5-naphthyridin-4-amine

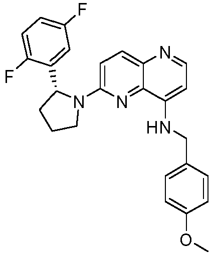

(R)-8-Chloro-2-(2-(2,5-difluorophenyl)pyrrolidin-1-yl)-1,5-naphthyridine (76 mg, 0.56 mmol), p-methoxybenzylamine (72 mg, 0.56 mmol), N,N-diisopropylethylamine (72 mg, 0.56 mmol) were mixed in *N*-methylpyrrolidone (2 mL), and the mixture was heated to 150 °C overnight. After cooled to room temperature,
(R)-6-(2-(2,5-difluorophenyl)pyrrolidin-1-yl)-N-(4-methoxybenzyl)-1,5-naphthyridin-4-amin e was obtained by reversed phase preparative chromatography (117 mg, yield 94%).

MS (ESI): m/z = 447 [M+H]+.

### (R)-6-(2-(2,5-Difluorophenyl)pyrrolidin-1-yl)-1,5-naphthyridin-4-amine

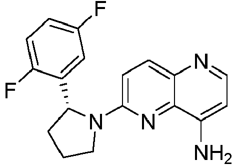

(R)-6-(2-(2,5-Difluorophenyl)pyrrolidin-1-yl)-1,5-naphthyridin-4-amine (83 mg, yield 98%) was prepared using method similar to that in example 7 by replacing the corresponding starting material.

MS (ESI): m/z = 327 [M+H]+.

### N-(6-((R)-2-(2,5-difluorophenyl)pyrrolidin-1-yl)-1,5-naphthyridin-4-yl)-2-((S)-3-hydrox ypyrrolidin-1-yl)pyrimidin-5-carboxamide

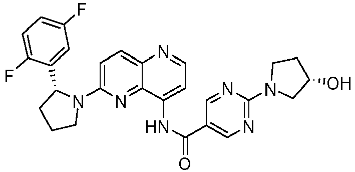

5-chloropyrazin-2-carboxylic acid (79 mg, 0.25 mmol), 2-(7-benzotriazole oxide)-*N,N,N',N'*-tetramethylurea hexafluorophosphate (144 mg, 0.38 mmol) and *N,N*-diisopropylethylamine (129 mg, 1.0 mmol) were added to a solution of (R)-6-(2-(2,5-difluorophenyl)pyrrolidin-1-yl)-1,5-naphthyridin-4-amine (83 mg, 0.25 mmol) in *N*, *N*-dimethylformamide(2 mL). The mixture was allowed to react at room temperature for 16 h, then (S)-pyrrolidine-3-ol hydrochloride (87 mg, 1.0 mmol) was added to and the mixture was allow to stir at room temperature for another 2h.
N-(6-((R)-2-(2,5-difluorophenyl)pyrrolidin-1-yl)-1,5-naphthyridine-4-yl)-2-((S)-3-hydroxypy rrolidin-1-yl)pyrimidine-5-carboxamide (70 mg, yield 54%) was obtained by reversed phase preparative chromatography, as a yellow solid.

MS (ESI): m/z = 518 [M+H]+.

¹H NMR (400 MHz,DMSO-*d*₆) δ 9.61 (s, 1H), 8.58 (s, 2H), 8.44 (d, *J* = 4.6 Hz, 1H), 8.28 (s, 1H), 8.06 (s, 1H), 7.43-6.92 (m, 3H), 6.90-6.80 (m, 1H), 5.78-5.53 (m, 1H), 5.04 (s, 1H), 4.41 (s, 1H), 3.79-3.51 (m, 4H), 2.01 (d, *J=* 44.0, 4H).

### Example 10:

### (R)-6-(2-(2,5-difluorophenyl)pyrrolidin-1-yl)-1,5-naphthyridin-4-carboxamide

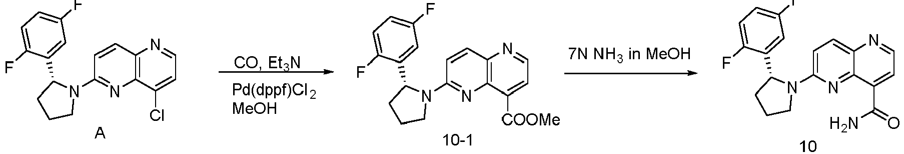

### Methyl (R)-6-(2-(2,5-difluorophenyl)pyrrolidin-1-yl)-1,5-naphthyridin-4-carboxylate

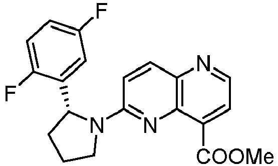

A mixture of (R)-8-chloro-2-(2-(2,5-difluorophenyl)pyrrolidin-1-yl)-1,5-naphthyridine (152 mg, 1.12 mmol), [1,1'-bis(diphenylphosphino)ferrocene]palladium dichloride (84 mg, 0.11 mmol) and triethylamine (339 mg, 3.36 mmol) in methanol (5 nL) was heated to 50 °C under CO atmosphere overnight. and the mixture was concentrated. and purification by silica chromatography afford methyl
(R)-6-(2-(2,5-difluorophenyl)pyrrolidin-1-yl)-1,5-naphthyridin-4-carboxylate (315 mg, yield 76%).

### (R)-6-(2-(2,5-Difluorophenyl)pyrrolidin-1-yl)-1,5-naphthyridin-4-carboxamide

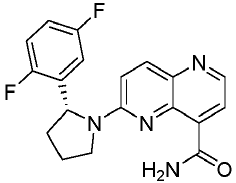

Methyl (R)-6-(2-(2,5-difluorophenyl)pyrrolidin-1-yl)-1,5-naphthyridin-4-carboxylate (40 mg, 0.11 mmol) was added to the solution of ammonium in methanol(2 mL, 7N), and the mixture was stirred at 80 °C under microwave for 2 h, and the reaction mixture was filtered to afford a white solid (20 mg, yield 51%).

MS (ESI): m/z = 355 [M+H]+.

¹H NMR (400 MHz,DMSO-*d*₆) δ 10.31 (s, 1H), 9.28 (s, 1H), 8.63 (d, *J* = 3.8 Hz, 1H), 8.25-8.02 (m, 2H), 7.74 (s, 1H), 7.44-6.74 (m, 4H), 5.38 (d, *J=* 8.1 Hz, 1H), 4.12-3.99 (m, 1H), 3.72-3.58 (m, 1H), 2.13-1.79 (m, 3H).

### Example 11:

### 2-((2R,4S)-2-(2,5-difluorophenyl)-4-fluoropyrrolidin-1-yl)-8-(1-(piperidin-4-yl)-1H-pyrizol-4-yl)-1,5-naphthyridine

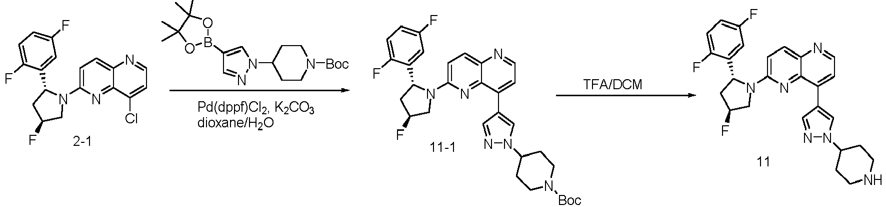

2-((2R,4S)-2-(2,5-difluorophenyl)-4-fluoropyrrolidin-1-yl)-8-(1-(piperidin-4-yl)-1H-pyri zol-4-yl)-1,5-naphthyridine (53 mg, yield 57%) was prepared using a method similar to that in example 7 by replacing the corresponding starting material.

MS (ESI): m/z = 479 [M+H]+.

¹H NMR (400 MHz,DMSO-*d*₆) δ 8.58 - 8.44 (m, 2H), 8.20 (s, 1H), 8.03 (d, *J* = 9.1 Hz, 1H), 7.77 (d, *J* = 4.8 Hz, 1H), 7.29 - 7.23 (m, 1H), 7.15 - 6.95 (m, 3H), 5.61-5.43 (m, 2H), 4.25 - 4.14 (m, 3H), 3.08 (d, *J* = 11.8 Hz, 2H), 2.98 - 2.85 (m, 1H), 2.64 (t, *J* = 12.0 Hz, 2H), 2.33 - 2.12 (m, 2H), 2.00 - 1.76 (m, 4H).

### Example 12:

### 2-((2R,4S)-2-(2,5-difluorophenyl)-4-fluoropyrrolidin-1-yl)-8-(1-(tetrahydro-2H-pyran-4-yl)-1H-pyrazol-4-yl)-1,5-naphthyridine

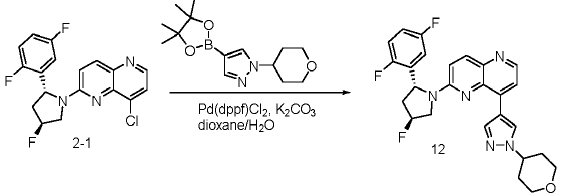

2-((2R,4S)-2-(2,5-difluorophenyl)-4-fluoropyrrolidin-1-yl)-8-(1-(tetrahydro-2H-pyran-4-yl)-1H-pyrazol-4-yl)-1,5-naphthyridine (54 mg, yield 56%) was prepared using method similar to that in example 7 by replacing the corresponding starting material.

MS (ESI): m/z = 480 [M+H]+.

¹H NMR (400 MHz, CD₃OD) δ 8.52 (s, 1H), 8.44 (d, *J* = 4.8 Hz, 1H), 8.23 (s, 1H), 8.02 (d, *J* = 9.2 Hz, 1H), 7.78 (d, *J* = 4.8 Hz, 1H), 7.15 (ddd, *J* = 21.4, 13.2, 6.6 Hz, 2H), 6.94 (dd, *J* = 18.0, 8.8 Hz, 2H), 5.62 (t, *J* = 7.9 Hz, 1H), 5.46 (d, *J* = 53.1 Hz, 1H), 4.45 (t*, J* = 11.5 Hz, 1H), 4.29 (dt, *J=* 12.6, 10.8 Hz, 2H), 4.14 (dd, *J=* 25.4, 7.3 Hz, 2H), 3.62 (t, *J=* 11.5 Hz, 2H), 3.01 (dd, *J* = 22.2, 11.7 Hz, 1H), 2.34-1.97 (m, 5H).

### Example 13:

### 2-((2R,4S)-2-(2,5-difluorophenyl)-4-fluoropyrrolidin-1-yl)-8-(1-(1-methylpiperidin-4-yl)-1H -pyrazol-4-yl)-1,5-naphthyridine

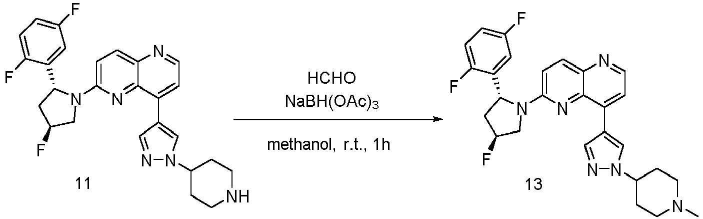

To a mixture of2-((2R,4S)-2-(2,5-difluorophenyl)-4-fluoropyrrolidin-1-yl)-8-(1-(1-methylpiperidin-4-yl) -1H-pyrazol-4-yl)-1,5-naphthyridine (60 mg, 0.125 mmol) in methanol (5 mL) and formaldehyde aqueous solution (0.5 mL)was added glacial acetic acid (0.05 mL). The reaction mixture was stirred at room temperature for 1h, and sodium triacetoxyborohydride (133 mg, 0.627 mmol) was added. The reaction mixture was stirred for 1 h at room temperature. After the starting material was consumed, the mixture was concentrated, and purified by reversed phase preparative chromatography to afford 2-((2R,4S)-2-(2,5-difluorophenyl)-4-fluoropyrrolidin-1-yl)-8-(1-(1-methylpiperidin-4-yl) -1H-pyrazol-4-yl)-1,5-naphthyridine (32 mg, yield 52.0%)as a yellow solid.

MS (ESI): m/z = 493 [M+H]+.

¹H NMR (400 MHz, DMSO-*d*₆) δ 8.62 - 8.45 (m, 2H), 8.21 (s, 1H), 8.03 (d, *J* = 9.1 Hz, 1H), 7.77 (d, *J=* 4.4 Hz, 1H), 7.29 - 7.24 (m, 1H), 7.15 - 6.95 (m, 3H), 5.61-5.42 (m, 2H), 4.30 - 4.02 (m, 3H), 2.98 - 2.83 (m, 3H), 2.34 - 2.10 (m, 4H), 2.11-1.80 (m, 6H).

### Example 14:

### 2-((2R,4S)-2-(2,5-difluorophenyl)-4-fluoropyrrolidin-1-yl)-8-(1-(1-isopropylpiperidin-4-yl))-1H-pyrazol-4-yl)-1,5-naphthyridine

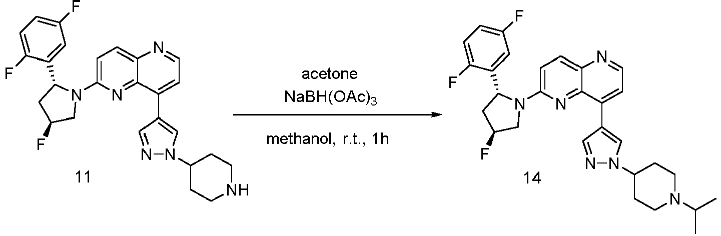

2-((2R,4S)-2-(2,5-difluorophenyl)-4-fluoropyrrolidin-1-yl)-8-(1-(1-isopropylpiperidin-4-yl))-1H-pyrazol-4-yl)-1,5-naphthyridine (18 mg, yield 16%) was prepared using method similar to that in example 13 by replacing the corresponding starting material.

MS (ESI): m/z = 521 [M+H]+.

¹H NMR (400 MHz, DMSO-*d*₆) δ 8.60-8.50 (s, 1H), 8.46 (d, *J=* 4.7 Hz,1H), 8.21 (s, 1H), 8.03 (d, *J* = 8.6 Hz, 1H), 7.77 (d, *J* = 4.5 Hz, 1H), 7.30-7.21 (m, 1H), 7.15 - 6.98 (m, 3H), 5.60-5.43 (m, 2H), 4.30-4.05 (m, 3H), 3.00 - 2.84 (m, 3H), 2.78-2.67 (m, 1H), 2.35 - 2.12 (m, 3H), 2.06-1.85 (m, 4H), 0.98 (d, *J* = 6.5 Hz, 6H).

### Example 15:

### (R)-2-(2-(2,5-Difluorophenyl)pyrrolidin-1-yl)-8-(5-fluoro-1-(piperidin-4-yl)-1H-pyrazole-4-yl)-1,5-naphthyridine

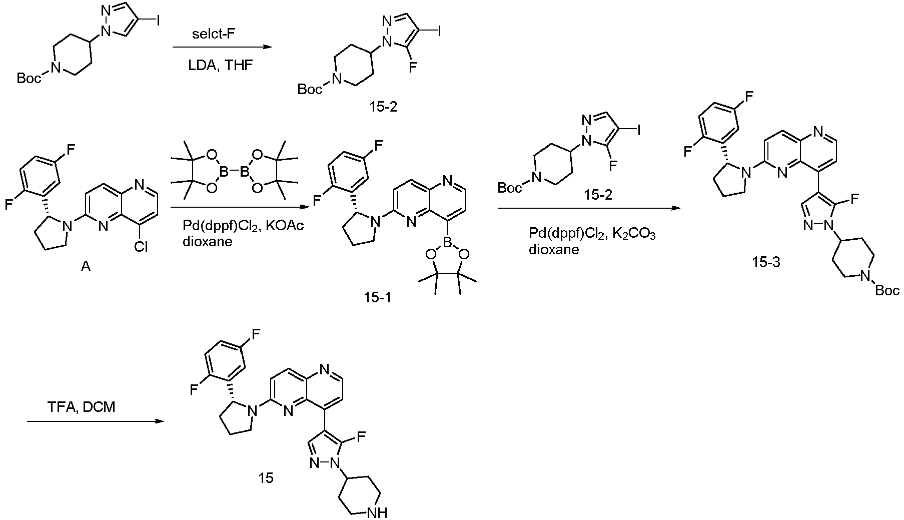

### Tert-Butyl 4-(5-fluoro-4-iodo-1H-pyrazol-1-yl)piperidin-1-carboxylate

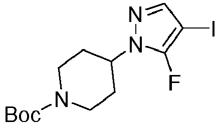

Tert-butyl 4-(4-iodo-1H-pyrazol-1-yl)piperidin-1-carboxylate (800 mg, 2.1 mmol) was dissolved in tetrahydrofuran (15 mL), and cooled to -78 °C. Under nitrogen atmosphere, LDA solution (2.1 mL, 4.2 mmol) was added and the mixture was stirred at this temperature for 30 minutes. SELECT-F reagent (2.6 g, 8.4 mmol) in tetrahydrofuran (10 mL) was added to and stirred for 1h. Saturated ammonium chloride was added to quench the reaction. Water (150 mL) was added and the mixture was extracted with ethyl acetate (150 mL*2). The combined organic phase was dried, filtered, concentrated and purified by silica chromatography (petroleum ether: EtOAc=1: 2) to afford a white solid (390 mg, yield 47%).

MS (ESI): m/z = 340 [M+H]+.

### (R)-2-(2-(2,5-Difluorophenyl)pyrrolidin-1-yl)-8-(4,4,5,5-tetramethyl-1,3,2-dioxaborolan-2-yl)-1,5-naphthyridine

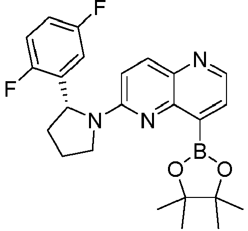

(R)-8-chloro-2-(2-(2,5-difluorophenyl)pyrrolidin-1-yl)-1,5-naphthyridine (100 mg, 0.29 mmol), 4,4,4',4',5,5,5',5'-octamethyl-2,2'-bi(1,3,2-dioxaborolane) (110 mg, 0.43 mmol), [1,1'-bis(diphenylphosphino)ferrocene]palladium dichloride (12 mg, 0.015 mmol) and potassium acetate (57 mg, 0.58 mmol) were mixed in 1,4-dioxane (2 mL) and the resulting mixture was heated to 100 °C overnight. The reaction mixture was concentrated and purified by column chromatography (ethyl acetate) to afford a brown solid (60 mg, yield 47%).

MS (ESI): m/z = 356 [M+H]+.

### Tert-butyl (R)-4-(4-(6-(2-(2,5-difluorophenyl)pyrrolidin-1-yl)-1,5-naphthyridine-4-yl) -5-fluoro-1H-pyrazol-1-yl)piperidin-1-carboxylate

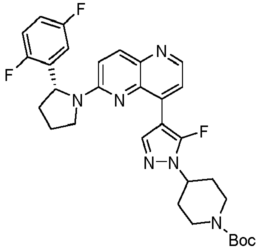

*Tert*-butyl(R)-4-(4-(6-(2-(2,5-difluorophenyl)pyrrolidin-1-yl)-1,5-naphthyridine-4-yl) -5-fluoro-1H-pyrazol-1-yl)piperidin-1-carboxylate (75 mg, yield 93%) was prepared using method similar to that in example 7 by replacing the corresponding starting material.

MS (ESI): m/z = 579 [M+H]+.

### (R)-2-(2-(2,5-Difluorophenyl)pyrrolidin-1-yl)-8-(5-fluoro-1-(piperidin-4-yl)-1H-pyrazole - 4-yl)-1,5-naphthyridine

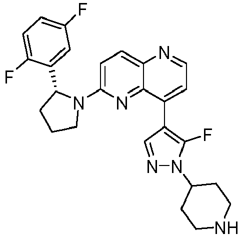

(R)-2-(2-(2,5-Difluorophenyl)pyrrolidin-1-yl)-8-(5-fluoro-1-(piperidin-4-yl)-1H-pyrazole - 4-yl)-1,5-naphthyridine (51 mg, yield 82%) was prepared using method similar to that in example 7 by replacing the corresponding starting material.

MS (ESI): m/z = 479 [M+H]+.

¹H NMR (400 MHz, DMSO-*d*₆) δ 8.48 (d, *J* = 4.6 Hz, 1H), 8.03 (s, 1H), 7.79 - 7.44 (m, 2H), 7.31 - 7.19 (m, 1H), 7.14 - 6.99 (m, 1H), 6.89 - 6.75 (m, 1H), 5.46 (s, 1H), 4.23 (s, 1H), 4.08-3.96 (m, 1H), 3.63 (s, 1H), 3.09 - 2.82 (m, 3H), 2.59 (t, *J* = 11.5 Hz, 2H), 2.43-2.32 (m, 1H), 2.12 - 1.99 (m, 1H), 1.99 - 1.70 (m, 6H).

### Example 16:

### 7-chloro-2-((2R,4S)-2-(2,5-difluorophenyl)-4-fluoropyrrolidin-1-yl)-8-(1-(piperidin-4-yl)-1H -pyrazol-4-yl)-1,5-naphthyridine

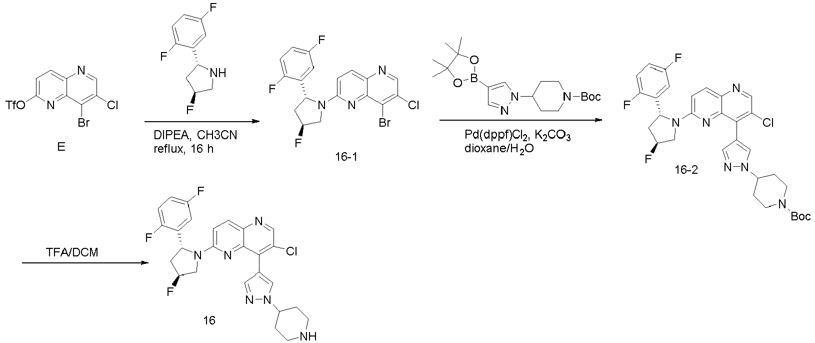

### 8-bromo-7-chloro-2-((2R,4S)-2-(2,5-difluorophenyl)-4-fluoropyrrolidin-1-yl)-1,5-naphth yridine

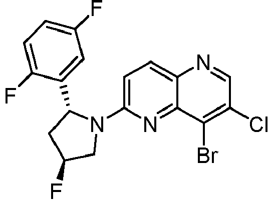

N, N-diisopropylethylamine (2.0 g, 15.34 mmol) was added to 8-bromo-7-chloro-1,5- naphthyridin-2-yl trifluoromethanesulfonate (3.0 g, 7.67 mmol) and (2R,4S)-2-(2,5-difluorobenzenyl)-4-fluoropyrrolidine (1.6 g, 8.05 mmol) in acetonitrile, the reaction mixture was allowed to react at 80 °C for 16 h. After the solvent was removed *in vacuo,* the crude product was purified by silica chromatography (ethyl acetate / petroleum ether = 2/1) to obtain
8-bromo-7-chloro-2-((2R,4S)-2-(2,5-difluorophenyl)-4-fluoropyrrolidin-1-yl)-1,5-naphthyrid ine (3.1, yield 91%)as a colorless oil.

MS (ESI): m/z = 442 [M+H]+.

### tert-butyl 4-(4-(3-chloro-6-((2R,4S)-2-(2,5-difluorophenyl)-4-fluoropyrolin-1-yl)-1,5-naphthyridin-4-yl )-1H-pyrazole-1-yl)piperidine-1-carboxylate

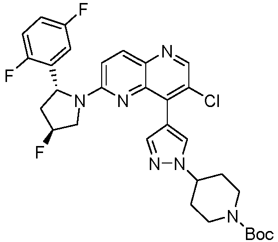

Tert-butyl 4-(4-(3-chloro-6-((2R,4S)-2-(2,5-difluorophenyl)-4-fluoropyrrolidin-1-yl)-1,5-naphthyridine-4-yl)-1H-pyrazol-1-yl)piperidine-1-carboxylate was prepared using method similar to that in example 7 by replacing the corresponding starting material.

MS (ESI): m/z = 613 [M+H]+.

### 7-chloro-2-((2R,4S)-2-(2,5-difluorophenyl)-4-fluoropyrrolidin-1-yl)-8-(1-(piperidin-4-yl) -1H-pyrazol-4-yl)-1,5-naphthyridine

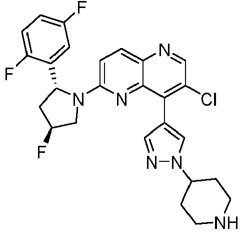

7-Chloro-2-((2R,4S)-2-(2,5-difluorophenyl)-4-fluoropyrrolidin-1-yl)-8-(1-(piperidin-4-yl )-1H-pyrazol-4-yl)-1,5-naphthyridine was prepared using method similar to that in example 7 by replacing the corresponding starting material.

MS (ESI): m/z = 513 [M+H]+.

¹H NMR (400 MHz, CD₃OD) δ 8.50 (s, 1H), 8.25 (s, 1H), 8.00 (d, J = 9.2 Hz, 2H), 7.10 (td, J = 9.2, 4.1 Hz, 1H), 7.03 (s, 1H), 6.93 (s, 1H), 6.84 (s, 1H), 5.55-5.31 (m, 2H), 4.45-4.22 (m, 2H), 4.09 (ddd, J = 35.7, 12.7, 3.0 Hz, 1H), 3.24 (s, 2H), 2.92 (s, 1H), 2.84 (t, J = 12.6 Hz, 2H), 2.31-2.11 (m, 3H), 2.04 (ddd, J = 24.5, 12.2, 3.6 Hz, 2H).

### Examples 17:

### 2-((R)-2-(2,5-difluorophenyl)pyrrolidin-1-yl)-8-(1-(2,2-dimethylpiperidin-4-yl)-1H-pyrazol-4-yl)-1,5-naphthyridine

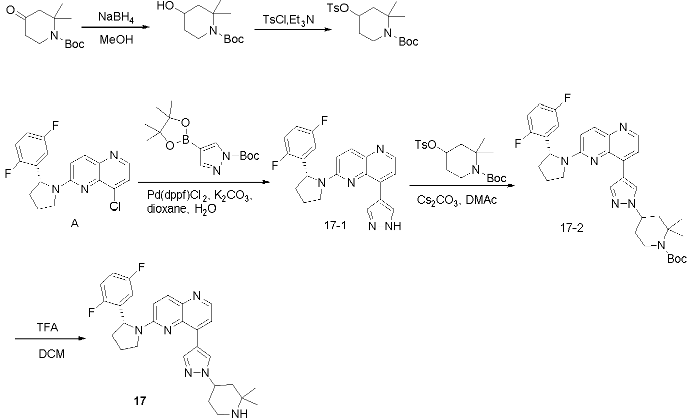

### Tert-butyl 4-hydroxy-2,2-dimethylpiperidin-1-carboxylate

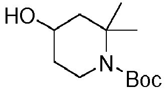

Sodium borohydride (1.0 g, 4.405mmol) was added to a mixture of *tert*-butyl 2,2-dimethyl-4-carbonylpiperidin-1-carboxylate (1.0 g, 4.405 mmol) in methanol (10 mL) under an ice bath, and the mixture was stirredat room temperature for 30 minutes. The reaction was quenched with water. After methanol was removed *in vacuo,* ethyl acetate was added and the organic phase was washed with water and dried over sodium sulfate. After filtration, the solvent was removed *in vacuo* to afford the title compound tert-butyl 4-hydroxy-2,2-dimethylpiperidin-1-carboxylate (790 mg, yield 78.3%) as colorless oil.

MS (ESI): m/z = 230 [M+H]+.

### Tert-butyl 2,2-dimethyl-4-(tosyloxy)piperidin-1-carboxylate

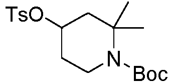

*p*-Toluenesulfonyl chloride (287 mg, 1.5 mmol), triethylamine (201 mg, 2.0 mmol) and 4-dimethylaminopyridine (24 mg, 0.2 mmol) were sequentially added to a solution of *tert*-butyl 4-hydroxy-2,2-dimethylpiperidin-1-carboxylate (229 mg, 1.0 mmol) in dichloromethane (10 mL). The mixture was reacted at 40 °C for 16 h, and diluted with dichloromethane. The organic phase was washed with water and saturated brine, dried with sodium sulfate, filtered, and the filtrate was concentrated. The residue was purified by silica chromatography (ethyl acetate/petroleum ether=1/4) to afford tert-butyl 2,2-dimethyl-4-(tosyloxy)piperidin-1-carboxylate (315 mg, yield 82.2%) as a pale yellow solid.

MS (ESI): m/z = 284 [M-Boc+H]+.

### (R)-2-(2-(2,5-Difluorophenyl)pyrrolidin-1-yl)-8-(1H-pyrazol-4-yl)-1,5-naphthyridine

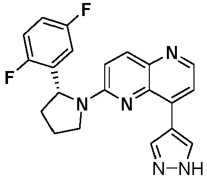

(R)-2-(2-(2,5-Difluorophenyl)pyrrolidin-1-yl)-8-(1H-pyrazol-4-yl)-1,5-naphthyridine (217 mg, yield 82%) was prepared using method similar to that in example 7 by replacing the corresponding starting material.

MS (ESI): m/z = 378 [M+H]+.

### Tert-butyl 4-(4-(6-((R)-2-(2,5-difluorophenyl)pyrrolidin-1-yl)-1,5-naphthyridine-4-yl) -1H-pyrazol-1-yl)-2,2-dimethylpiperidin-1-carboxylate

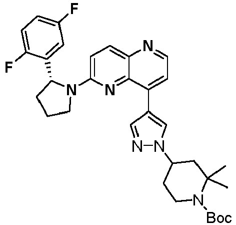

A mixture of
(R)-2-(2-(2,5-difluorophenyl)pyrrolidin-1-yl)-8-(1H-pyrazol-4-yl)-1,5-naphthyridine (136 mg, 0.36 mmol), *tert*-butyl 2,2-dimethyl-4-(tosyloxy)piperidine-1-carboxylate (138 mg, 0.36 mmol) and cesium carbonate (176 mg; 0.54 mmol)in *N,N*-dimethylacetamide (5 mL)was stirred for 3 h at 120 °C. After the reaction was completed, ethyl acetate was added. The organic phase was washed with water and brine, dried over sodium sulfate, and filtered. The solvent was removed *in vacuo.* The residue was purified by silica chromatography (ethyl acetate/petroleum ether=7/3) to afford the title compound tert-butyl
4-(4-(6-((R)-2-(2,5-difluorophenyl)pyrrolidin-1-yl)-1,5-naphthyridin-4-yl)-1H-pyrazol-1-yl)-2,2-dimethylpiperidin-1-carboxylate (60 mg, yield 29.3%) as a yellow solid.

MS (ESI): m/z = 589 [M+H]+.

### 2-((R)-2-(2,5-difluorophenyl)pyrrolidin-1-yl)-8-(1-(2,2-dimethylpiperidin-4-yl)-1H-pyra zol-4-yl)-1,5-naphthyridine

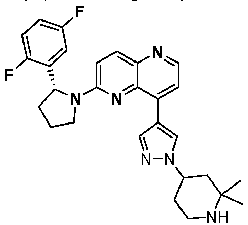

2-((R)-2-(2,5-difluorophenyl)pyrrolidin-1-yl)-8-(1-(2,2-dimethylpiperidin-4-yl)-1H-pyra zol-4-yl)-1,5-naphthyridine (33 mg, yield 66%) was prepared using method similar to that in example 7by replacing the corresponding starting material.

MS (ESI): m/z = 489 [M+H]+.

¹H NMR (400 MHz, CD₃OD) δ 8.51 (s, 1H), 8.41 (d, *J* = 4.8 Hz, 1H), 8.29-8.14 (m, 1H), 8.02 (s, 1H), 7.77 (s, 1H), 7.20 (dd, *J=* 9.6, 4.9 Hz, 2H), 6.98 (s, 1H), 6.77 (s, 1H), 5.61 (s, 1H), 4.67 (s, 1H), 4.08 (s, 1H), 3.76 (s, 2H), 3.42 (s, 2H), 2.54 (s, 1H), 2.20 (d, *J* = 9.4 Hz, 4H), 2.08 (d, *J* = 7.4 Hz, 2H), 1.54 (s, 3H), 1.47 (d, *J* = 5.5 Hz, 3H).

### Example 18:

### 2-((2R,4S)-2-(2,5-difluorophenyl)-4-fluoropyrrolidin-1-yl)-8-(1-(2,2-dimethylpiperidin-4-yl) -1H-pyrazol-4-yl)-1,5-naphthyridine

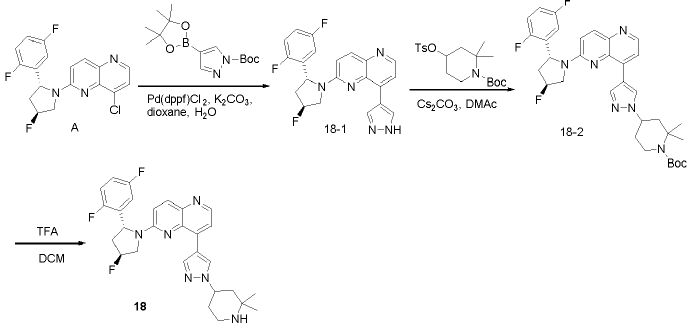

2-((2R,4S)-2-(2,5-difluorophenyl)-4-fluoropyrrolidin-1-yl)-8-(1-(2,2-dimethylpiperidin-4 -yl)-1H-pyrazol-4-yl)-1,5-naphthyridine was prepared using method similar to that in example 17by replacing the corresponding starting material.

MS (ESI): m/z = 507 [M+H]+.

¹H NMR (400 MHz, CD₃OD) δ 8.55 (s, 1H), 8.43 (d, *J* = 4.8 Hz, 1H), 8.22 (s, 1H), 8.01 (d, *J* = 9.3 Hz, 1H), 7.81-7.73 (m, 1H), 7.17-7.10 (m, 2H), 7.02 - 6.86 (m, 2H), 5.62 (s, 1H), 5.47 (d, *J* = 52.8 Hz, 1H), 4.57 (d, *J* = 3.8 Hz, 1H), 4.40 - 4.14 (m, 2H), 3.17-2.94 (m, 3H), 2.34-2.09 (m, 2H), 2.05 (d, *J* = 12.3 Hz, 1H), 1.89 (dt, *J* = 20.9, 10.5 Hz, 2H), 1.33 (d, *J=* 3.1 Hz, 3H), 1.26 (d, *J* = 4.8 Hz, 3H).

### Example 19:

### 4-(4-(6-((2R,4S)-2-(2,5-difluorophenyl)-4-fluoropyrrolidin-1-yl)-1,5-naphthyridin-4-yl)-1H-pyrazol-1-yl)piperidin-2-one

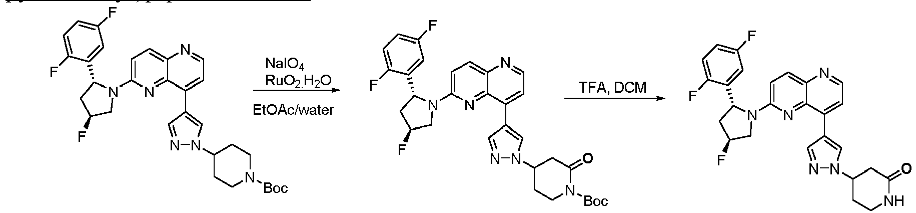

### Tert-butyl 4-(4-(6-((2R,4S)-2-(2,5-difluorophenyl)-4-fluoropyrrolidin-1-yl)-1,5-naphthyridin-4-yl)-1H-prazol-1-yl)-2-carbonylpiperidin-1-carboxlate

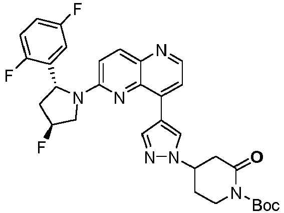

Sodium periodate (288 mg, 1.35 mmol) and ruthenium oxide (4 mg, 0.03 mmol) were added to a mixture of *tert*-butyl
4-(4-(6-((2R,4S)-2-(2,5-difluorophenyl)-4-fluoropyrrolidin-1-yl)-1,5-naphthyridin-4-yl)-1H-pyrazol-1-yl)piperidin-1-carboxylate (156 mg, 0.27 mmol) in ethyl acetate (8 mL) and water (2 mL). The mixture was stirred at 25 °C for 3h. After the starting material was consumed, water (50 mL) was added, and the mixture was extracted with ethyl acetate (50 mL*2). The combined organic phase was dried, concentrated, and purified by silica chromatography (ethyl acetate) to afford tert-butyl
4-(4-(6-((2R,4S)-2-(2,5-difluorophenyl)-4-fluoropyrrolidin-1-yl)-1,5-naphthyridin-4-yl)-1H-pyrazol-1-yl)piperidin-1-carboxylate (40 mg, yield 25%) as a white solid.

MS (ESI): m/z = 593 [M+H]+.

### 4-(4-(6-((2R,4S)-2-(2,5-difluorophenyl)-4-fluoropyrrolidin-1-yl)-1,5-naphthyridin-4-yl)-1H-pyrazol-1-yl)piperidin-2-one

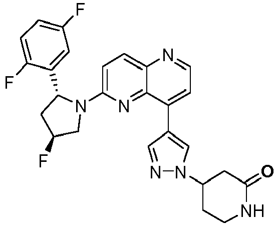

4-(4-(6-((2R,4S)-2-(2,5-difluorophenyl)-4-fluoropyrrolidin-1-yl)-1,5-naphthyridin-4-yl)-1H-pyrazol-1-yl)piperidin-2-one was prepared using method similar to that in example 7 by replacing the corresponding starting material.

MS (ESI): m/z = 493 [M+H]+.

¹H NMR (400 MHz, DMSO-*d*₆) δ 8.76 - 8.73 (m, 2H), 8.48 (d, *J* = 9.2 Hz, 1H), 8.38 (s, 1H), 8.04 (s, 1H), 7.87 (d, *J=* 4.7 Hz, 1H), 7.28-7.19 (m, 1H), 7.09-7.00 (m, 1H), 6.95-6.86 (m, 1H), 6.28 (d, *J=* 8.8 Hz, 1H), 5.74 - 5.54 (m, 1H), 4.25 - 4.15 (m, 1H), 3.07 (d, *J=* 9.8 Hz, 2H), 3.00 - 2.80 (m, 2H), 2.69-2.56 (m, 3H), 1.95-1.80 (m, 4H).

### Example 20:

### 2-((2R,4S)-2-(2,5-difluorophenyl)-4-fluoropyrrolidin-1-yl))-7-fluoro-8-(1-(piperidin-4-yl)-1 H-pyrizol-4-yl)-1,5-naphthyridine

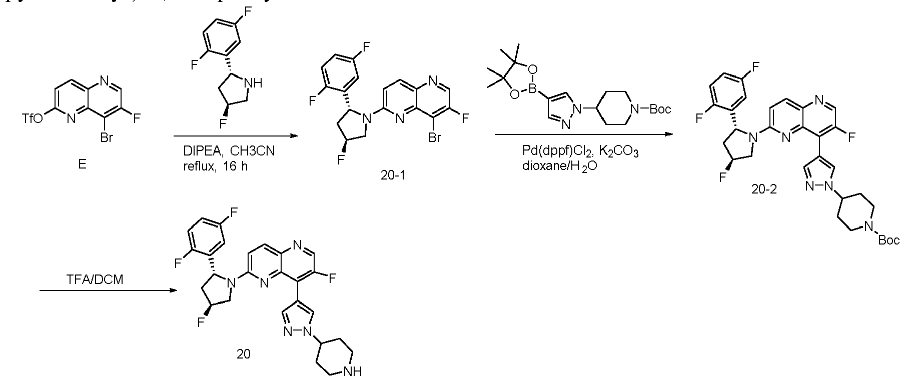

2-((2R,4S)-2-(2,5-difluorophenyl)-4-fluoropyrrolidin-1-yl))-7-fluoro-8-(1-(piperidin-4-yl )-1H-pyrizol-4-yl)-1,5-naphthyridine was prepared using method similar to that in example 16 by replacing the corresponding starting material.

MS (ESI): m/z = 493 [M+H]+.

¹H NMR (400 MHz, CD₃OD) δ 8.52 (s, 1H), 8.48 (d, *J=* 2.8 Hz, 1H), 8.31 (d, *J* = 9.32 Hz, 1H), 7.14 (td, *J* = 9.4, 4.3 Hz, 1H), 7.10-7.00 (m, 1H), 7.00-6.86 (m, 2H), 5.61 (t, *J* = 7.7 Hz, 1H), 5.46 (d, *J* = 52.8 Hz, 1H), 4.64-4.54 (m, 1H), 4.41-4.12 (m, 2H), 3.60-3.50 (m, 2H), 3.23-3.12 (m, 2H), 3.06-2.93 (m, 1H), 2.42-2.10 (m, 5H).

### Example 21:

### (R)-2-(2-(2,5-Difluorophenyl)pyrrolidin-1-yl))-7-fluoro-8-(1-(piperidin-4-yl)-1H-pyrazol-4-y l)-1,5-naphthyridine

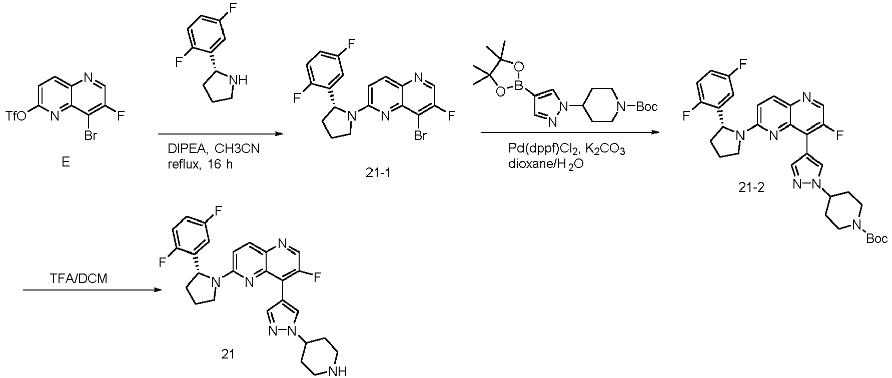

(R)-2-(2-(2,5-Difluorophenyl)pyrrolidin-1-yl))-7-fluoro-8-(1-(piperidin-4-yl)-1H-pyrazol -4-yl)-1,5-naphthyridine was prepared using method similar to that in example 21 by replacing the corresponding starting material.

MS (ESI): m/z = 479 [M+H]+.

¹H NMR (400 MHz, DMSO-*d*₆) δ 8.99-8.66 (m, 1H), 8.54 (d, *J=* 2.2 Hz, 1H), 8.46-7.77 (m, 3H), 7.37-7.24 (m, 1H), 7.18-7.03 (m, 1H), 6.91-6.80 (m, 1H), 5.70-5.06 (m, 2H), 4.31-3.96 (m, 2H), 3.80-3.53 (m, 1H), 3.04 (d, *J=* 12.4 Hz, 1H), 2.59 (t, *J=* 13.5 Hz, 1H), 2.14-1.64 (m, 7H).

### Example 22:

### 8-(1-(azetidine-3-yl)-1H-pyrazol-4-yl)-7-chloro-2-((2R,4S)-2-(2,5-difluorophenyl)-4-fluorop yrrolidin-1-yl)-1,5-naphthyridine

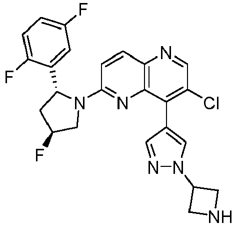

8-(1-(Azetidine-3-yl)-1H-pyrazol-4-yl)-7-chloro-2-((2R,4S)-2-(2,5-difluorophenyl)-4-flu oropyrrolidin-1-yl)-1,5-naphthyridine was prepared using method similar to that in example 21 by replacing the corresponding starting material.

MS (ESI): m/z = 485 [M+H]+.

¹H NMR (400 MHz, DMSO-*d*₆) δ 8.55 (s, 1H), 8.06-8.04 (m, 3H), 7.18-6.93 (m, 4H), 5.55-5.26 (m, 3H), 4.27-3.82 (m, 6H), 2.87-2.63 (m, 1H), 2.29-2.07 (m, 1H).

### Example 23:

### 7-chloro-2-((2R,4S)-2-(2,5-difluorophenyl)-4-fluoropyrrolidin-1-yl)-8-(1-(1-methylazetidin)-3-yl)-1H-pyrazol-4-yl)-1,5-naphthyridine

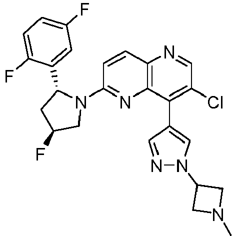

7-Chloro-2-((2R,4S)-2-(2,5-difluorophenyl)-4-fluoropyrrolidin-1-yl)-8-(1-(1-methylazeti din)-3-yl)-1H-pyrazol-4-yl)-1,5-naphthyridine was prepared using method similar to that in example 13 by replacing the corresponding starting material.

MS (ESI): m/z = 500 [M+H]+.

¹H NMR (400 MHz, CD₃OD) δ 8.50 (s, 1H), 8.48 (s, 1H), 8.04-7.95 (m, 3H), 7.11-7.03 (m, 2H), 6.91-6.87 (m, 1H), 6.77-6.75 (m, 1H), 5.53-5.21 (m, 3H), 4.27 - 4.02 (m, 6H), 2.96-2.85 (m, 1H), 2.75 (s, 3H), 2.26-2.08 (m, 1H).

### Example 24:

### (R)-7-chloro-2-(2-(2,5-difluorophenyl)pyrrolidin-1-yl)-8-(1-(piperidin-4-yl)-1H-pyrazol-4-yl)-1,5-naphthyridine

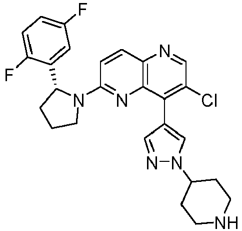

(R)-7-chloro-2-(2-(2,5-difluorophenyl)pyrrolidin-1-yl)-8-(1-(piperidin-4-yl)-1H-pyrazol-4-yl)-1,5-naphthyridine was prepared using method similar to that in example 21 by replacing the corresponding starting material.

MS (ESI): m/z = 495 [M+H]+.

¹H NMR (400 MHz, CD₃OD) δ 8.73 (s, 1H), 8.28-7.77 (m, 3H), 7.19-7.16 (m, 1H), 6.98-6.96 (m, 1H), 6.74-6.71 (m, 1H), 5.56-5.54 (m, 1H), 4.64-4.56 (m, 1H), 4.12-4.08 (m, 1H), 3.77-3.61 (m, 3H), 2.54-2.03 (m, 8H).

### Example 25:

### 7-chloro-2-((2R,4S)-2-(2,5-difluorophenyl)-4-fluoropyrrolidin-1-yl)-8-(1-(methylsulfonyl)-1 H-pyrazol-4-yl)-1,5-naphthyridine

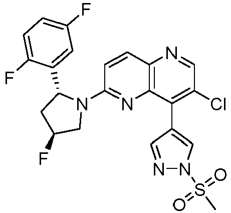

7-Chloro-2-((2R,4S)-2-(2,5-difluorophenyl)-4-fluoropyrrolidin-1-yl)-8-(1-(methylsulfony l)-1H-pyrazol-4-yl)-1,5-naphthyridine was prepared using method similar to that in example 21 by replacing the corresponding starting material.

MS (ESI): m/z = 508 [M+H]+.

### Example 26:

### (S)-N-(6-((2R,4S)-2-(2,5-difluorophenyl)-4-fluoropyrrolidin-1-yl)-1,5-naphthyridin-4-yl)-3-h ydroxypyrrolidin-1-carboxamide

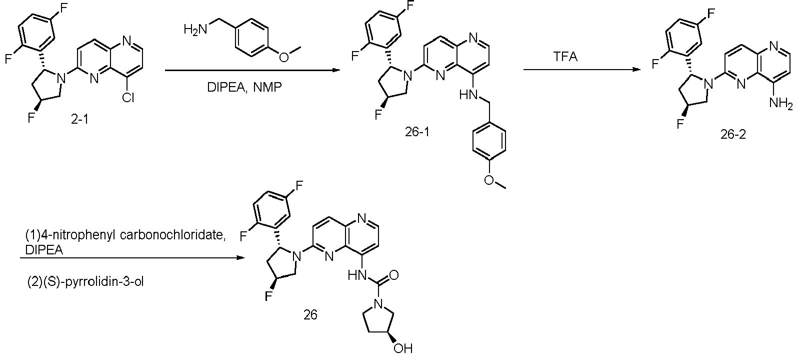

### 6-((2R,4S)-2-(2,5-difluorophenyl)-4-fluoropyrrolidin-1-yl)-1,5-naphthyridin-4-amine

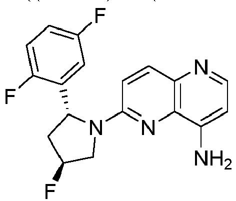

6-((2R,4S)-2-(2,5-difluorophenyl)-4-fluoropyrrolidin-1-yl)-1,5-naphthyridin-4-amine was prepared using method similar to that in example 8-2 by replacing the corresponding starting material.

### (S)-N-(6-((2R,4S)-2-(2,5-difluorophenyl)-4-fluoropyrrolidin-1-yl)-1,5-naphthyridin-4-yl) -3-hydroxypyrrolidin-1-carboxamide

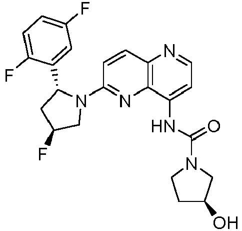

A mixture of 6-((2R,4S)-2-(2,5-difluorophenyl)-4-fluoropyrrolidin-1-yl)-1,5-naphthyridin-4-amine (80 mg, 0.233 mmol), *N*, *N*-Diisopropylethylamine (150 mg, 1.165 mmol) and *p*-nitrophenyl chloroformate (140 mg, 0.698 mmol) in dichloromethane (5 mL) was stirred at room temperature for 16h. (S)-pyrrolidin-3-ol (101 mg, 1.165 mmol) was added to the mixture, and the mixture was stirred for 1h. Dichloromethane and water were added, and the organic phase was washed with water and brine, dried over sodium sulfate, and filtered. The solvent was removed *in vacuo,* and the residue was purified by reverse phase column to obtain (S)-*N*-(6-((2R,4S)-2-(2,5-difluorophenyl)-4-fluoropyrrolidin-1-yl)-1,5 -naphthyridin-4-yl)-3-h ydroxypyrrolidin-1-carboxamide (64 mg, yield 60%) as white solid.

MS (ESI): m/z = 458 [M+H]+.

¹H NMR (400 MHz, CD₃OD) δ 8.34 (d, *J* = 5.3 Hz, 1H), 8.16 (d, *J* = 5.3 Hz, 1H), 8.01 (d, *J =* 9.2 Hz, 1H), 7.24-7.09 (m, 2H), 6.98 (ddd, *J =* 9.0, 7.4, 3.6 Hz, 1H), 6.86 (s, 1H), 5.63 (t, *J =* 7.8 Hz, 1H), 5.43 (d, *J =* 53.2 Hz, 1H), 4.49 (s, 1H), 4.32-4.05 (m, 2H), 3.65-3.35 (m, 4H), 3.08-2.94 (m, 1H), 2.31-1.98 (m, 3H).

### Example 27:

### (S)-N-(6-((R)-2-(2,5-difluorophenyl)pyrrolidin-1-yl)-1,5-naphthyridin-4-yl)-3-hydroxypyrrol idin-1-carboxamide

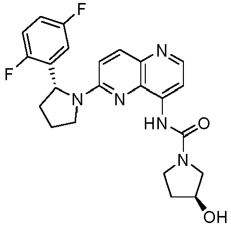

(S)-*N*-(6-((R)-2-(2,5-difluorophenyl)pyrrolidin-1-yl)-1,5-naphthyridin-4-yl)-3-hydroxypy rrolidin-1-carboxamide was prepared using method similar to that in example 26 by replacing the corresponding starting material.

MS (ESI): m/z = 440 [M+H]+.

¹H NMR (400 MHz, DMSO-*d*₆) δ 7.50 (d, *J =* 5.2 Hz, 1H), 7.32 (d, *J =* 5.2 Hz, 1H), 7.19 (d, *J =* 8.7 Hz, 1H), 6.54-6.22 (m, 2H), 6.22-6.12 (m, 1H), 5.99-5.90 (m, 1H), 4.76 (s, 1H), 3.66 (s, 1H), 3.19 (t, *J =* 8.3 Hz, 1H), 3.00-2.50 (m, 5H), 1.77-1.62 (m, 1H), 1.47-1.10 (m, 5H).

The compounds as shown in the following table were prepared using method similar to that in the examples by replacing the corresponding starting material:

| No. | Name of Compound | Structure | Structure Characterization |
|---|---|---|---|
| Exa mple 28 | (R)-8-(1-(azetidin -3-yl)-1H-pyrazol -4-yl)-2-(2-(2,5-d ifluorophenyl)pyr rolidin-1-yl)-7-fl uoro-1,5-naphthy ridine | 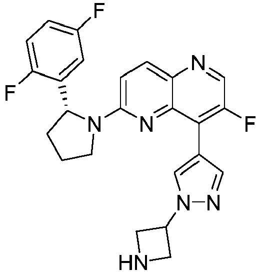 | MS (ESI): m/z = 451 [M+H]+. ¹H NMR (400 MHz, CD₃OD) δ 8.72-8.11 (m, 3H), 8.10-7.94 (m, 1H), 7.30-7.02 (m, 2H), 7.02-6.90 (m, 1H), 6.82-6.72 (m, 1H), 5.58 (s, 1H), 5.27 (s, 1H), 4.32-4.18 (m, 2H), 4.13-3.91 (m, 3H), 3.76 (s, 1H), 2.59-2.43 (m, 1H), 2.22-1.96 (m, 3H). |
| Exa mple 29 | 8-(1-(azetidin-3-y l)*-*1H-pyrazol-4-y l)-2-((2R,4S)-2-( 2,5-difluoropheny l)-4-fluoropyrroli din-1-yl)-7-fluoro -1,5-naphthyridin e | 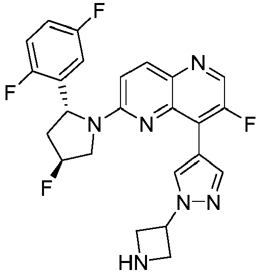 | MS (ESI): m/z = 469 [M+H]+. 1H NMR (400 MHz, CD₃OD) δ 8.64-8.41 (m, 2H), 8.30 (s, 1H), 8.03 (d, *J* = 9.5 Hz, 1H), 7.19-7.01 (m, 2H), 6.99-6.80 (m, 2H), 5.61 (t, *J* = 7.9 Hz, 1H), 5.56-5.32 (m, 2H), 4.44-4.11 (m, 4H), 4.06 (t, *J* = 8.6 Hz, 2H), 3.05-2.91 (m, 1H), 2.34-2.13 (m, 1H). |
| Exa mple 30 | (R)-7-chloro-2-(2 -(5-fluoro-2-meth oxypyridin-3-yl)p yrrolidin-1-yl)-8-(1-(piperidin-4-yl )-1H-pyrazol-4-yl )-1,5-naphthyridi ne | 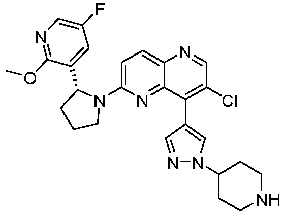 | MS (ESI): m/z = 509 [M+H]+. ¹H NMR (400 MHz, CD₃OD) δ 8.51 (s, 1H), 8.47 (s, 1H), 8.01 (s, 1H), 7.85 (s, 1H), 7.69-7.38 (bs, 1H), 7.22 (bs, 1H), 7.07 (d, *J* = 7.2 Hz, 1H), 5.43 - 5.25 (m, 1H), 4.55 - 4.40 (m, 1H), 4.07 (s, 3H), 4.03 - 3.95 (m, 1H), 3.75 - 3.60 (m, 1H), 3.60-3.49 (m, 2H), 3.20 - 3.12 (m, 2H), 2.50 - 2.20 (m, 5H), 2.12 - 1.88 (m, 3H) |
| Exa mple 31 | 7-chloro-2-((2R,4 S)-2-(2,5-difluoro phenyl)-4-fluorop yrrolidin-1-yl)-8-(1H-pyrazol-4-yl) -1,5-naphthyridin e | 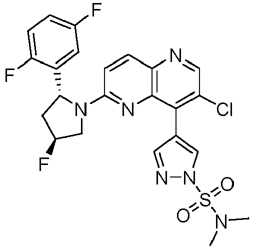 | MS (ESI): m/z = 537 [M+H]+. ¹H NMR (400 MHz, CD₃OD) δ 8.57 (s, 1H), 8.50 (s, 1H), 8.00 (d, *J* = 9.0 Hz, 1H), 7.98-7.67 (m, 1H), 7.14-6.98 (m, 2H), 6.97 - 6.88 (m, 1H), 6.83 (bs, 1H), 5.55-5.30 (m, 2H), 4.34 - 4.16 (m, 1H), 4.15 - 4.00 (m, 1H), 2.98 (s, 6H), 2.95-2.81 (m, 1H), 2.26-2.09 (m, 1H). |
| Exa mple 33 | (R)-7-chloro-2-(2 -(5-fluoro-2-meth oxyphenyl)pyrrol idin-1-yl)-8-(1-(p iperidin-4-yl)-1H -pyrazol-4-yl)-1,5 -naphthyridine | 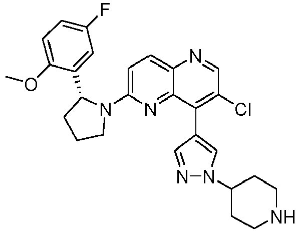 | MS (ESI): m/z = 508 [M+H]+. ¹H NMR (400 MHz, CD₃OD) δ 8.70 - 8.05 (m, 3H), 7.88 (bs, 1H), 7.03 - 6.99 (m, 1H), 6.95 - 6.87 (m, 1H), 6.65 - 6.58 (m, 2H), 5.45-5.28 (m, 1H), 4.43-4.25 (m, 1H), 4.05 - 3.95 (m, 1H), 3.93 (s, 1H), 3.82 - 3.65 (m, 1H), 3.25 - 3.16 (m, 2H), 2.84-2.72 (m, 2H), 2.47 - 2.32 (m, 1H), 2.20 - 2.10 (m, 2H), 2.07-1.87 (m, 5H). |
| Exa mple 35 | 1-(4-(3-chloro-6-( (2R,4S)-2-(2,5-di fluorophenyl)-4-f luoropyrrolidin-1 -yl)-1,5-naphthyri din-4-yl)-1H-pyra zol-1-yl)-2-methy lpropan-2-ol | 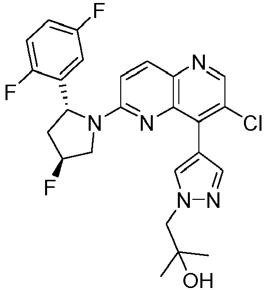 | MS (ESI): m/z = 502 [M+H]+. ¹H NMR (400 MHz, CD₃OD) δ 8.55-8.39 (m, 1H), 8.25 (s, 1H), 8.00 (d, *J* = 9.3 Hz, 1H), 7.87 (s, 1H), 7.16-6.98 (m, 2H), 6.98-6.88 (m, 1H), 6.84 (s, 1H), 5.54-5.31 (m, 2H), 4.40-3.99 (m, 4H), 2.99-2.83 (m, 1H), 2.29-2.09 (m, 1H), 1.22 (d, *J* = 5.4 Hz, 6H). |
| Exa mple 37 | 6-((2R,4S)-2-(2,5 -difluorophenyl)-4-fluoropyrrolidi n-1-yl)-4-(1-(pipe ridin-4-yl)-1H-py razol-4-yl)-1,5-na phthyridin-3 -carb oxamide | 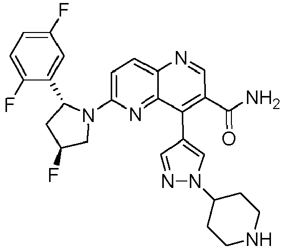 | MS (ESI): m/z = 522 [M+H]+. |
| Exa mple 39 | 3-(4-(3-chloro-6-( (2R,4S)-2-(2,5-di fluorophenyl)-4-f luoropyrrolidin- 1 -yl)-1,5-naphthyri din-4-yl)-1H-pyra zol-1-yl)-1-methy lcyclobutan-1-ol | 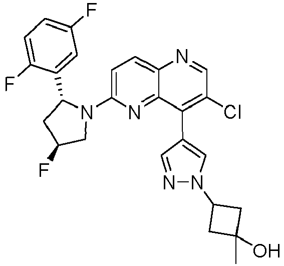 | MS (ESI): m/z = 514 [M+H]+. ¹H NMR (400 MHz, CD₃OD) δ 8.49 (s, 1H), 8.21 (s, 1H), 8.01 (d, *J* = 9.2 Hz, 1H), 7.94 (s, 1H), 7.12-6.98 (m, 2H), 6.96-6.86 (m, 1H), 6.79 (s, 1H), 5.54-5.31 (m, 2H), 4.59 (p, *J* = 8.0 Hz, 1H), 4.33-4.20 (m, 1H), 4.10 (ddd, *J* = 36.0, 12.7, 2.9 Hz, 1H), 2.99-2.82 (m, 1H), 2.70 (d, *J* = 8.1 Hz, 4H), 2.29-2.11 (m, 1H), 1.46 (s, 3H). |
| Exa mple 40 | (R)-3-(4-(4-(3-chl oro-6-((2R,4S)-2-(2,5-difluorophen yl)-4-fluoropyrrol idin-1-yl)-1,5-nap hthyridin-4-yl)-1 H-pyrazol-1-yl)pi peridin-1-yl)prop ane-1,2-diol | 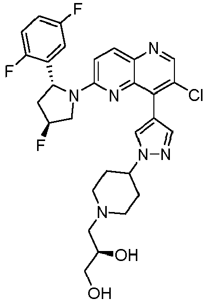 | MS (ESI): m/z = 587 [M+H]+. ¹H NMR (400 MHz, CD₃OD) δ 8.51 (s, 1H), 8.24 (s, 1H), 8.04 - 7.87 (m, 2H), 7.20 (td, *J* = 9.3, 4.4 Hz, 1H), 7.03 (s, 1H), 6.93 (s, 1H), 5.53-5.33 (m, 2H), 4.37-4.21 (m, 2H), 4.17-4.02 (m, 1H), 3.89-3.80 (m, 1H), 3.60-3.48 (m, 2H), 3.22 (d, *J* = 11.4 Hz, 2H), 2.99-2.84 (m, 1H), 2.65-2.51 (m, 2H), 2.51-2.34 (m, 2H), 2.31-2.11 (m, 4H). |
| Exa mple 41 | 7-chloro-2-((2R,4 S)-2-(2,5-difluoro phenyl)-4-fluorop yrrolidin-1-yl)-8-(2-(piperidin-4-yl )-2H-1,2,3-triazol -4-yl)-1,5-naphth yridine | 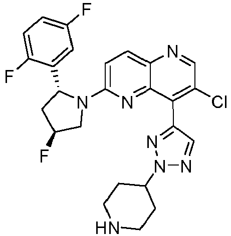 | MS (ESI): m/z = 514 [M+H]+. ¹H NMR (400 MHz, CD₃OD) δ 8.57 (s, 1H), 8.08 (d, *J* = 9.1 Hz, 1H), 7.61-7.42 (m, 1H), 7.23-7.02 (m, 2H), 6.97-6.86 (m, 1H), 6.77 (s, 1H), 5.47-5.30 (m, 2H), 4.78-4.68 (m, 1H), 4.29-4.05 (m, 2H), 4.05-3.95 (m, 1H), 3.26-3.20 (m, 2H), 2.93-2.81 (m, 2H), 2.33-2.02 (m, 5H). |
| Exa mple 42 | 2-(3-Chloro-6-((2 R,4S)-2-(2,5-difl uorophenyl)-4-flu oropyrrolidin-1-y 1)-1,5-naphthyridi n-4-yl)-5-(piperid in-4-yl)-1,3,4-oxa diazole | 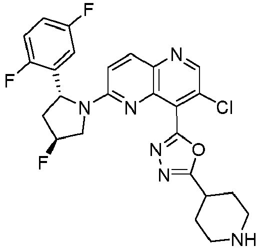 | MS (ESI): m/z = 515 [M+H]+. ¹H NMR (400 MHz, CD₃OD) δ 8.65 (s, 1H), 8.12 (d, *J* = 6.8 Hz, 1H), 7.26-6.74 (m, 4H), 5.46-5.33 (m, 2H), 4.20-4.02 (m, 2H), 3.43-3.34 (m, 1H), 2.95-2.70 (m, 3H), 2.25-1.93 (m, 5H). |
| Exa mple 43 | 7-Chloro-2-((2R, 4S)-2-(2,5-difluor ophenyl)-4-fluoro pyrrolidin-1-yl)-8 -(1-(1-methylpipe ridine-4-yl)-1H-pyrazol-4-yl)-1,5-naphthy ridine | 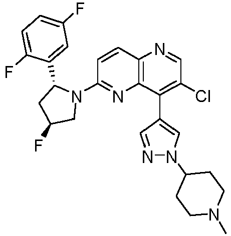 | MS (ESI): m/z = 527 [M+H]+. |
| Exa mple 44 | (S)-3-(4-(4-(3-chl oro-6-((2R,4S)-2-(2,5-difluorophen yl)-4-fluoropyrrol idin-1-yl)-1,5-nap hthyridin-4-yl)-1 H-pyrazol-1-yl)pi peridin-1-yl)prop ane-1,2-diol | 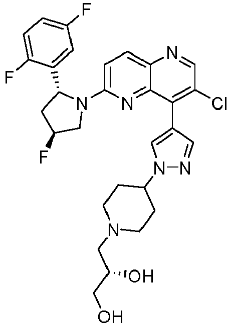 | MS (ESI): m/z = 587 [M+H]+. ¹H NMR (400 MHz, CD₃OD) δ 8.50 (s, 1H), 8.25 (s, 1H), 8.04 - 7.88 (m, 2H), 7.10 (td, *J* = 9.4, 4.4 Hz, 1H), 7.06-6.98 (m, 1H), 6.93 (s, 1H), 6.83 (s, 1H), 5.53-5.32 (m, 2H), 4.37-4.20 (m, 2H), 4.09 (ddd, *J* = 35.5, 12.7, 3.1 Hz, 1H), 3.87-3.79 (m, 1H), 3.53 (qd, *J* = 11.1, 5.3 Hz, 2H), 3.22-3.12 (m, 2H), 2.99-2.85 (m, 1H), 2.60-2.45 (m, 2H), 2.44-2.3413 (m, 6H). |
| Exa mple 45 | 3-chloro-6-((2R,4 S)-2-(2,5-difluoro phenyl)-4-fluorop yrrolidin-1-yl)-N' -(piperidin -4-carb onyl)-1,5 -naphthy ridin -4-carboxyli c acid hydrazide | 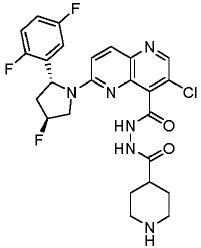 | MS (ESI): m/z = 533 [M+H]+. |
| Exa mple 47 | 4-(4-(3-chloro-6-( (2R,4S)-2-(2,5-di fluorophenyl)-4-f luoropyrrolidin-1 -yl)-1,5-naphthyri din-4-yl)-1H-pyra zol-1-yl)tetrahydr o-2H-thiopyran 1,1-dioxide | 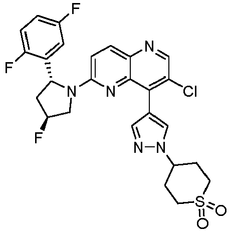 | MS (ESI): m/z = 562 [M+H]+. ¹H NMR (400 MHz, CD₃OD) δ 8.50 (s, 1H), 8.27 (s, 1H), 8.01 (d, *J* = 9.2 Hz, 1H), 7.88 (s, 1H), 7.11 (ddd, *J* = 24.0, 14.3, 9.6 Hz, 2H), 6.93 (s, 1H), 6.83 (s, 1H), 5.55-5.31 (m, 2H), 4.74-4.60 (m, 1H), 4.39-4.19 (m, 1H), 4.18-4.01 (m, 1H), 3.44-3.32 (m, 3H), 2.99-2.85 (m, 1H), 2.77-2.62 (m, 2H), 2.53 (d, *J* = 10.8 Hz, 2H), 2.19 (d, *J* = 38.9 Hz, 1H). |
| Exa mple 48 | 7-chloro-2-((2R,4 S)-2-(2,5-difluoro phenyl)-4-fluorop yrrolidin-1-yl)-8-(1-(piperidin-4-yl )-1H-pyrrol-3-yl) -1,5-naphthyridin e | 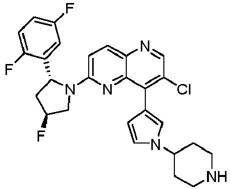 | MS (ESI): m/z = 512 [M+H]+. ¹H NMR (400 MHz, CD₃OD) δ 8.45 (s, 1H), 7.97 (d, *J* = 9.3 Hz, 1H), 7.42 (s, 1H), 7.16-7.06 (m, 1H), 6.95 (d, *J*= 8.7 Hz, 2H), 6.83 (s, 2H), 6.50 (s, 1H), 5.53-5.29 (m, 2H), 4.39-3.93 (m, 4H), 2.89 (t, *J* = 12.7 Hz, 3H), 2.35-2.08 (m, 3H), 2.06-1.87 (m, 2H). |
| Exa mple 49 | (S)-N-(3-Chloro-6-((2R,4S)-2-(2,5 -difluorophenyl)-4- fluoropyrrolidi n-1-yl)-1,5-napht hyridin-4-yl)-3-h ydroxypyrrolidin - 1-carboxamide | 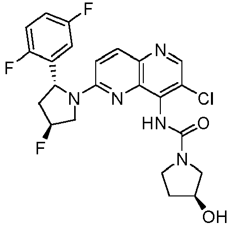 | MS (ESI): m/z = 493 [M+H]+. |
| Exa mple 50 | 5-(3-Chloro-6-((2 R,4S)-2-(2,5-difl uorophenyl)-4-flu oropyrrolidin-1-y 1)-1,5-naphthyridi n-4-yl)-2-(piperid in-4-yl)thiazole | 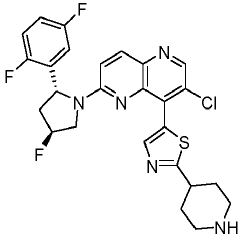 | MS (ESI): m/z = 530 [M+H]+. ¹H NMR (400 MHz, CD₃OD) δ 8.57 (s, 1H), 8.06 (d, *J* = 9.6 Hz, 2H), 7.05 (s, 2H), 6.91 (s, 1H), 6.75 (s, 1H), 5.55-5.31 (m, 2H), 4.40-3.94 (m, 3H), 3.23 (d, *J* = 12.5 Hz, 2H), 2.85 (t, *J* = 13.0 Hz, 3H), 2.21 (t, *J* = 14.6 Hz, 3H), 1.87 (dd, *J* = 23.4, 11.7 Hz, 2H). |
| Exa mple 52 | 6-((2R,4S)-2-(2,5 -difluorophenyl)-4-fluoropyrrolidi n-1-yl)-4-(1-(pipe ridin-4-yl)-1H-py razol-4-yl)pyrido[ 3,2-d]pyrimidine | 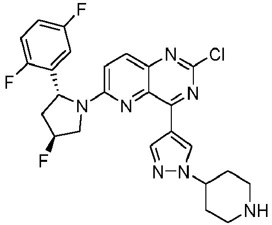 | MS (ESI): m/z = 514 [M+H]+. ¹H NMR (400 MHz, DMSO-*d*₆) δ 8.80 - 8.10 (m, 2H), 8.05 - 7.90 (m, 1H), 7.69-7.35 (m, 1H), 7.30 - 7.22 (m, 1H), 7.15 - 7.00 (m, 2H), 5.65 - 5.45 (m, 2H), 4.35 - 4.18 (m, 3H), 3.07 (d, J = 12.5 Hz, 2H), 3.00-2.85 (m, 1H), 2.65 - 2.55 (m, 2H), 2.34 - 2.10 (m, 1H), 2.00 - 1.75 (m, 4H). |
| Exa mple 53 | 6-((2R,4S)-2-(2,5 -difluorophenyl)-4-fluoropyrrolidi n-1-yl)-2-fluoro-4 -(1-(piperidin-4-y 1)-1H-pyrazol-4-y l)pyrido[3,2-d]py rimidine | 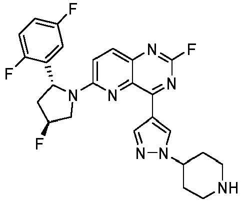 | MS (ESI): m/z = 498 [M+H]+. ¹H NMR (400 MHz, DMSO-*d*₆) δ 8.66 (s, 1H), 8.35 (s, 1H), 8.27 (s, 1H), 7.97 (d, *J* = *9.8* Hz, 1H), 7.55 - 7.32 (m, 1H), 7.32-7.21 (m, 1H), 7.15 - 7.00 (m, 2H), 5.65 - 5.40 (m, 2H), 4.40 - 4.10 (m, 3H), 3.13 (d, *J* = 9.1 Hz, 2H), 2.99-2.85 (m, 1H), 2.78-2.62 (m, 2H), 2.33 - 2.13 (m, 1H), 2.03 - 1.85 (m, 4H). |
| Exa mple 54 | 7-chloro-2-((2R,4 S)-2-(2,5-difluoro phenyl)-4-fluorop yrrolidin-1-yl)-8-(1-((R)-piperidin-3-yl)-1H-pyrazol-4-yl)-1,5-naphthy ridine | 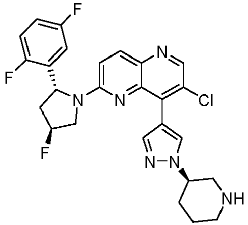 | MS (ESI): m/z = 513 [M+H]+. ¹H NMR (400 MHz, CD₃OD) δ 8.50 (s, 1H), 8.21 (s, 1H), 8.00 (d, J = 9.1 Hz, 1H), 7.94 (s, 1H), 7.11 (td, *J* = 9.6, 4.5 Hz, 1H), 7.04 (s, 1H), 6.93 (s, 1H), 6.82 (s, 1H), 5.53-5.30 (m, 2H), 4.41-4.22 (m, 2H), 4.18-4.02 (m, 1H), 3.37 (d, *J* = 13.1 Hz, 1H), 3.04 (dd, *J* = 12.2, 10.1 Hz, 2H), 2.94 (d, *J* = 20.4 Hz, 1H), 2.72 (dd, *J* = 18.0, 6.4 Hz, 1H), 2.33-2.05 (m, 3H), 1.98-1.88 (m, 1H), 1.78-1.66 (m, 1H). |
| Exa mple 55 | 7-chloro-2-((2R,4 S)-2-(2,5-difluoro phenyl)-4-fluorop yrrolidin-1-yl)-8-(1-((S)-piperidin-3-yl)-1H-pyrazol-4-yl)-1,5-naphthy ridine | 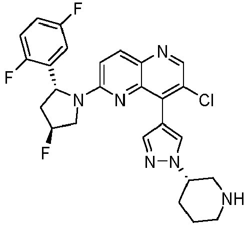 | MS (ESI): m/z = 513 [M+H]+. ¹H NMR (400 MHz, CD₃OD) δ 8.50 (s, 1H), 8.24 (s, 1H), 8.00 (d, *J* = 9.2 Hz, 1H), 7.94 (s, 1H), 7.10 (td, *J* = 9.6, 4.5 Hz, 1H), 7.03 (s, 1H), 6.93 (s, 1H), 6.83 (s, 1H), 5.53-5.32 (m, 2H), 4.35 (dd, *J* = 23.4, 13.0 Hz, 2H), 4.18-3.99 (m, 1H), 3.39 (d, *J* = 11.9 Hz, 1H), 3.11-3.01 (m, 2H), 2.98-2.84 (m, 1H), 2.77-2.67 (m, 1H), 2.32-2.02 (m, 3H), 1.92 (d, *J* = 14.0 Hz, 1H), 1.78-1.65 (m, 1H). |
| Exa mple 56 | 7-chloro-2-((2R,4 S)-2-(2,5-difluoro phenyl)-4-fluorop yrrolidin-1-yl)-8-(1-((R)-pyrrolidin -3-yl)-1H-pyrazol -4-yl)-1,5-naphth yridine | 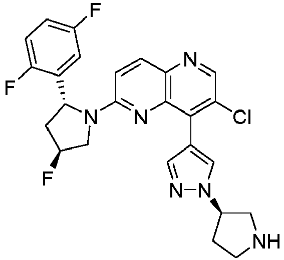 | MS (ESI): m/z = 499 [M+H]+. ¹H NMR (400 MHz, CD₃OD) δ 8.51 (s, 1H), 8.19 (s, 1H), 8.03 (d, *J* = 9.3 Hz, 1H), 7.90 (s, 1H), 7.19-7.01 (m, 2H), 7.01-6.89 (m, 1H), 6.81 (s, 1H), 5.47 (dd, *J* = 40.6, 32.0 Hz, 2H), 5.20 (s, 1H), 4.37-4.19 (m, 1H), 4.18-3.99 (m, 1H), 3.65-3.52 (m, 3H), 3.38-3.32 (m, 1H), 2.93 (d, *J* = 20.9 Hz, 1H), 2.53 (dt, *J* = 16.3, 8.0 Hz, 1H), 2.44-2.31 (m, 1H), 2.20 (d, *J* = 39.0 Hz, 1H). |
| Exa mple 57 | 7-chloro-2-((2R,4 S)-2-(2,5-difluoro phenyl)-4-fluorop yrrolidin-1-yl)-8-(1-((S)-pyrrolidin -3-yl)-1H-pyrazol -4-yl)-1,5-naphth yridine | 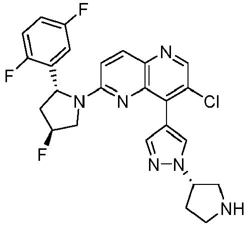 | MS (ESI): m/z = 499 [M+H]+. ¹H NMR (400 MHz, CD₃OD) δ 8.50 (s, 1H), 8.20 (s, 1H), 8.03 (d, *J* = 9.1 Hz, 1H), 7.95 (s, 1H), 7.15-7.03 (m, 2H), 6.92 (s, 1H), 6.80 (s, 1H), 5.53 (t, *J* = 8.1 Hz, 1H), 5.42 (d, *J* = 53.2 Hz, 1H), 5.18 (s, 1H), 4.31-4.19 (m, 1H), 4.18-4.02 (m, 1H), 3.63-3.51 (m, 3H), 3.34 (dd, *J* = 8.6, 5.3 Hz, 1H), 2.91 (s, 1H), 2.53 (dd, *J* = 14.0, 8.4 Hz, 1H), 2.35 (dd, *J* = 8.4, 5.2 Hz, 1H), 2.19 (d, *J* = 41.3 Hz, 1H). |
| Exa mple 58 | 6-((2R,4S)-2-(2,5 -difluorophenyl)-4-fluoropyrrolidi n-1-yl)-2-fluoro-4 -(1-(piperidin-4-y 1)-1H-pyrazol-4-y l)pyrido[3,2-d]py rimidine | 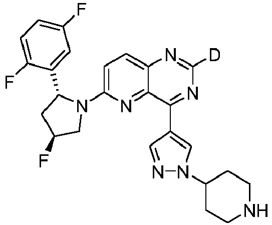 | MS (ESI): m/z = 481 [M+H]+. ¹H NMR (400 MHz, DMSO-*d*₆) δ 8.55 (bs, 1H), 8.32 (bs, 1H), 8.02 (d, *J* = 9.9 Hz, 1H), 7.55 - 7.20 (m, 2H), 7.15 - 6.95 (m, 2H), 5.65 - 5.45 (m, 2H), 4.32-4.15 (m, 3H), 3.11-2.83 (m, 3H), 2.65 - 2.54 (m, 2H), 2.35 - 2.14 (m, 1H), 1.95 - 1.72 (m, 4H). |
| Exa mple 59 | 4-(1-(azetidin-3-y lmethyl)-1H-pyra zol-4-yl)-6-((2R, 4S)-2-(2,5-difluor ophenyl)-4-fluoro pyrrolidin-1-yl)p yrido[3,2-d]pyrim idine HCOOH salt | 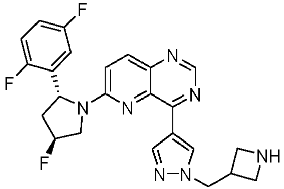 | MS (ESI): m/z = 466 [M+H]+. ¹H NMR (400 MHz, DMSO-*d*₆) δ 8.86 (s, 1H), 8.60 (s, 1H), 8.43 - 8.23 (m, 2H), 8.03 (d, *J* = 8.7 Hz, 1H), 7.45-7.20 (m, 2H), 7.15-7.00 (m, 2H), 5.65 - 5.44 (m, 2H), 4.50 - 4.33 (m, 2H), 4.35 - 4.15 (m, 2H), 3.88 - 3.72 (m, 2H), 3.75 - 3.62 (m, 2H), 3.28 - 3.10 (m, 1H), 3.00 - 2.82 (m, 1H), 2.35 - 2.10 (m, 2H). |
| Exa mple 60 | 5-(3-Chloro-6-((2 R,4S)-2-(2,5-difl uorophenyl)-4-flu oropyrrolidin-1-y 1)-1,5-naphthyridi n-4-yl)-2-(piperaz in-4-yl)thiazole | 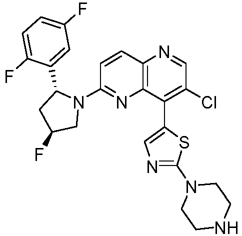 | MS (ESI): m/z =531 [M+H]+. ¹H NMR (400 MHz, CD₃OD) δ 8.49 (s, 1H), 8.00 (d, *J* = 9.3 Hz, 1H), 7.86 (s, 1H), 7.05 (d, *J* = 4.0 Hz, 2H), 6.90 (s, 1H), 6.79 (s, 1H), 5.58 (t, *J* = 7.8 Hz, 1H), 5.43 (d, *J* = 52.3 Hz, 1H), 4.35 (s, 1H), 4.14 (ddd, J= 16.1, 13.1, 3.5 Hz, 1H), 3.68-3.53 (m, 4H), 3.14-3.01 (m, 4H), 3.00-2.86 (m, 1H), 2.21 (d, *J* = 39.6 Hz, 1H). |
| Exa mple 61 | 4-(5-(3-chloro-6-( (2R,4S)-2-(2,5-di fluorophenyl)-4-f luoropyrrolidin-1 -yl)-1,5-naphthyri din-4-yl)thiazol-2 -yl)morpholine | 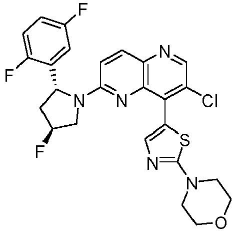 | MS (ESI): m/z = 532 [M+H]+. ¹H NMR (400 MHz, CD₃OD) δ 8.49 (s, 1H), 8.00 (d, *J* = 9.3 Hz, 1H), 7.87 (s, 1H), 7.05 (d, *J* = 4.3 Hz, 2H), 6.84 (d, *J* = 41.7 Hz, 2H), 5.58 (t, *J* = 8.2 Hz, 1H), 5.43 (d, *J* = 52.7 Hz, 1H), 4.35 (s, 1H), 4.21-4.06 (m, 1H), 3.91-3.80 (m, 4H), 3.61-3.50 (m, 4H), 3.01-2.87 (m, 1H), 2.29-2.10 (m, 1H). |
| Exa mple 62 | N-(3-Chloro-6-(( 2R,4S)-2-(2,5-dif luorophenyl)-4-fl uoropyrrolidin- 1 - yl)-1,5-naphthyri din-4-yl) piperidin -4-carbo xamide | 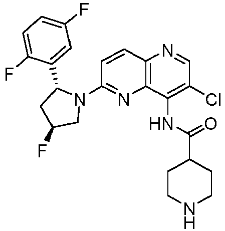 | MS (ESI): m/z = 491 [M+H]+. |
| Exa mple 64 | 6-((2R,4S)-2-(2,5 -difluorophenyl)-4-fluoropyrrolidi n-1-yl)-4-(1-(pipe ridin-4-yl)-1H-py razol-4-yl)pyrido[ 3,2-c]pyridazine | 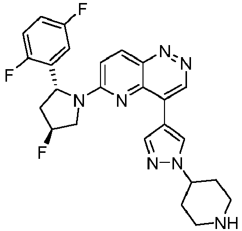 | MS (ESI): m/z = 480 [M+H]+. ¹H NMR (400 MHz, DMSO-d6) δ9.60 - 9.40 (m, 1H), 8.95 - 8.05 (m, 3H), 7.63 - 6.85 (m, 4H), 5.80 - 5.42 (m, 2H), 4.55 - 4.05 (m, 3H), 3.12 - 3.04 (m, 2H), 3.01 - 2.87 (m, 1H), 2.69 - 2.57 (m, 2H), 2.45 - 2.10 (m, 1H), 1.93 - 1.75 (m, 4H) |
| Exa mple 65 | (R)-1-(5-(3-chlor o-6-((2R,4S)-2-(2 ,5-difluorophenyl )-4-fluoropyrrolid in-1-yl)-1,5-napht hyridin-4-yl)pyri din-2-yl)pyrrolidi n-3-ol | 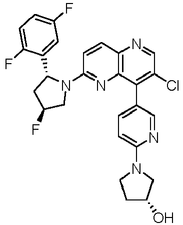 | MS (ESI): m/z = 526 [M+H]+. ¹H NMR (400 MHz, CD₃OD) δ 8.54 (s, 1H), 8.06 (d, *J* = 9.0 Hz, 1H), 7.97 (s, 1H), 7.39 (s, 1H), 7.13 (s, 1H), 6.93 (s, 1H), 6.80 (s, 1H), 6.60 (s, 1H), 6.50 (d, *J* = 8.7 Hz, 1H), 5.46-5.27 (m, 2H), 4.61 (s, 1H), 4.19-3.93 (m, 2H), 3.75-3.63 (m, 3H), 3.56 (d, *J* = 11.4 Hz, 1H), 2.81 (s, 1H), 2.28-2.17 (m, 1H), 2.16-1.98 (m, 2H), 1.26 (s, 1H). |
| Exa mple 66 | (S)-1-(5-(3-chlor o-6-((2R,4S)-2-(2 ,5-difluorophenyl )-4-fluoropyrrolid in-1-yl)-1,5-napht hyridin-4-yl)pyri din-2-yl)pyrrolidi n-3-ol | 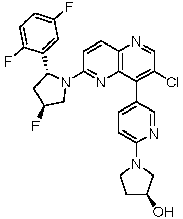 | MS (ESI): m/z = 526 [M+H]+. ¹H NMR (400 MHz, DMSO-*d*₆) δ 8.59 (s, 1H), 8.09 (d, *J* = 8.5 Hz, 1H), 7.93 (s, 1H), 7.35-6.83 (m, 4H), 6.69 (s, 1H), 6.33 (s, 1H), 5.43 (d, *J* = 53.4 Hz, 1H), 5.25 (t, *J* = 8.1 Hz, 1H), 5.01 (d, *J* = 3.5 Hz, 1H), 4.43 (s, 1H), 4.23-3.81 (m, 2H), 3.54 (dt, *J* = 11.0, 5.6 Hz, 3H), 3.39 (d, *J* = 10.6 Hz, 1H), 2.75 (s, 1H), 2.14-1.89 (m, 3H), 1.20 (s, 1H). |
| Exa mple 67 | 7-chloro-2-((2R,4 S)-2-(2,5-difluoro phenyl)-4-fluorop yrrolidin-1-yl)-8-(1-(piperidin-4-yl )-2H-1,2,3-triazol -4-yl)-1,5-naphth yridine | 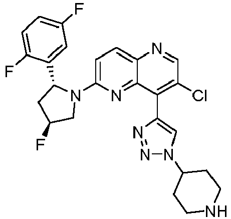 | MS (ESI): m/z = 515 [M+H]+. ¹H NMR (400 MHz, CD₃OD) δ 8.92 (bs, 1H), 8.81 (s, 1H), 8.35 (d, *J* = 9.2 Hz, 1H), 7.19-7.00 (m, 4H), 5.71 (t, *J* = 9.2Hz, 1H), 5.55 (d, *J* = 52.4 Hz, 1H), 5.09-4.84 (m, 1H), 4.41-4.33 (m, 2H), 3.67-3.64 (m, 2H), 3.37-3.29 (m, 2H), 3.10-3.02 (m, 1H), 2.59-2.45 (m, 5H). |
| Exa mple 68 | (S)-1-(3-(6-((2R, 4S)-2-(2,5-difluor ophenyl)-4-fluoro pyrrolidin-1-yl)p yrido[3,2-d]pyrim idin-4-yl)-1,2,4-o xadiazol-5-yl)pyr rolidin-3-ol | 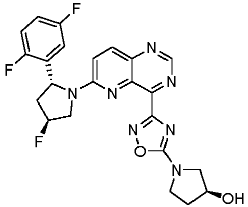 | MS (ESI): m/z = 484 [M+H]+. ¹H NMR (400 MHz, CD₃OD) δ 9.03 (s, 1H), 8.07 (s, 1H), 7.67-6.90 (m, 3H), 5.55-5.38 (m, 2H), 4.79 (s, 1H), 4.19-4.05 (m, 1H), 3.84-3.80 (m, 3H), 3.62-3.60 (m, 1H), 2.85-2.82 (m, 1H), 2.26-2.13 (m, 3H). |
| Exa mple 70 | (S)-1-(1-(6-((2R, 4S)-2-(2,5-difluor ophenyl)-4-fluoro pyrrolidin-1-yl)p yrido[3,2-d]pyrim idin-4-yl)-1H-pyr azol-4-yl)pyrrolid in-3-ol | 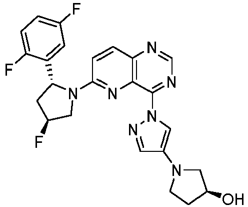 | MS (ESI): m/z = 482 [M+H]+ ¹H NMR (400 MHz, DMSO-*d*₆) δ 8.76 (s, 1H), 8.57 (s, 1H), 8.03 (d, *J* = 9.4 Hz, 1H), 7.63 (s, 1H), 7.30 - 7.05 (m, 4H), 5.64-5.41 (m, 2H), 4.93 (d, *J* = 4.1 Hz, 1H), 4.41-4.12 (m, 3H), 3.36 - 3.32 (m, 1H), 3.28-3.20 (m, 1H), 3.18-3.10 (m, 1H), 3.00-2.84 (m, 2H), 2.35 - 2.17 (m, 1H), 2.12 - 2.02 (m, 1H), 1.88-1.78 (m, 1H). |
| Exa mple 71 | (S)-1-(6-((2R,4S) -2-(2,5-difluorop henyl)-4-fluoropy rrolidin-1-yl)pyri do[3,2-d]pyrimidi ne-4-yl)pyrrolidin -3-ol | 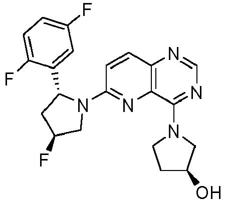 | MS (ESI): m/z = 416 [M+H]+ ¹H NMR (400 MHz, DMSO-*d*₆) δ 8.16 (s, 1H), 7.78 (d, *J* = 9.1 Hz, 1H), 7.28 - 7.22 (m, 1H), 7.13-6.94 (m, 3H), 5.54 - 5.38 (m, 2H), 4.90 - 4.78 (m, 1H), 4.52 - 4.35 (m, 1H), 4.26-4.04 (m, 3H), 3.72 - 3.50 (m, 2H), 2.87 - 2.75 (m, 1H), 2.20 - 2.02 (m, 1H), 1.98 - 2.83 (m, 1H), 1.80 - 1.65 (m, 2H). |
| Exa mple 72 | (S)-1-(5-(6-((2R, 4S)-2-(2,5-difluor ophenyl)-4-fluoro pyrrolidin-1-yl)p yrido[3,2-d]primi din-4-yl)pyrimidi n-2-yl)pyrrolidin-3-ol | 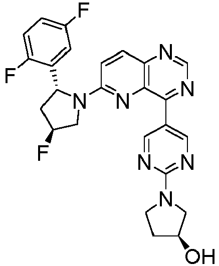 | MS (ESI): m/z = 494 [M+H]+ ¹H NMR (400 MHz, DMSO-*d*₆) δ 9.11 (bs, 2H), 8.93 (s, 1H), 8.06 (d, *J* = *8.3* Hz, 1H), 7.65 - 7.00 (m, 4H), 5.62 - 5.42 (m, 2H), 5.01 (d, *J* = 3.5 Hz, 1H), 4.45 - 5.38 (m, 1H), 4.32-4.10 (m, 2H), 3.77-3.51 (m, 4H), 2.95 - 2.80 (m, 1H), 2.30-2.10 (m, 1H), 2.09 - 1.98 (m, 1H), 1.98 - 1.88 (m, 1H). |
| Exa mple 73 | 3-(6-((2R,4S)-2-( 2,5-difluoropheny l)-4-fluoropyrroli din-1-yl)-3-fluoro -1,5-naphthyridin -4-yl)-5-(piperidi n-4-yl)-1,2,4-oxa diazole | 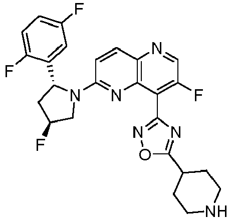 | MS (ESI): m/z = 499 [M+H]+ ¹H NMR (400 MHz, CD₃OD) δ 8.71 (s, 1H), 8.23 (d, *J* = 9.6 Hz, 1H), 7.26-6.86 (m, 4H), 6.57-5.32 (m, 2H), 4.29-4.22 (m, 2H), 3.69-3.55 (m, 4H), 2.94-2.88 (m, 1H), 2.55-2.49 (m, 2H), 2.27-2.05 (m, 3H). |
| Exa mple 74 | 2-(4-(4-(6-((2R,4 S)-2-(2,5-difluoro phenyl)-4-fluorop yrrolidin-1-yl)-3-fluoro-1,5-naphth yridin-4-yl)-1H-p yrazol-1-yl)piperi din-1-yl)acetamid e | 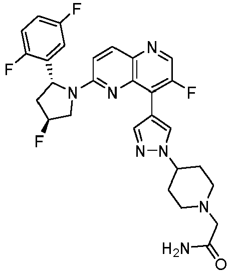 | MS (ESI): m/z = 554 [M+H]+ ¹H NMR (400 MHz, CD₃OD) δ 8.53 (s, 1H), 8.51 (s, 1H), 8.48 (s, 1H), 8.02 (d, *J* = 9.2Hz, 1H), 7.18-6.90 (m, 4H), 5.60 (t, *J* = 9.2 Hz, 1H), 5.46 (d, *J =* 53.2 Hz, 1H), 4.35-4.15 (m, 3H), 3.11-2.99 (m, 5H), 2.45-2.07 (m, 7H). |
| Exa mple 75 | (S)-1-(5-(6-((2R, 4S)-2-(2,5-difluor ophenyl)-4-fluoro pyrrolidin-1-yl)p yrido[3,2-d]pyrim idin-4-yl)pyridin-2-yl)pyrrolidin-3-ol | 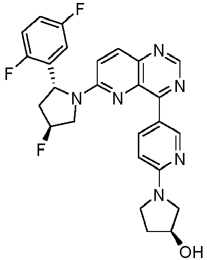 | MS (ESI): m/z = 493 [M+H]+ ¹H NMR (400 MHz, CD₃OD) δ 9.16 (s, 1H), 8.85 (s, 1H), 8.40 (s, 1H), 7.99 (d, *J* = 8.7 Hz, 1H), 7.29 (s, 1H), 7.10 (s, 1H), 6.92 (s, 2H), 6.50 (s, 1H), 5.50 (dd, *J* = 43.7, 30.5 Hz, 2H), 4.58 (s, 1H), 4.33 (s, 1H), 4.15 (ddd, *J* = 23.1, 12.9, 6.6 Hz, 1H), 3.75-3.61 (m, 3H), 3.56 (d, *J* = 11.1 Hz, 1H), 3.01-2.87 (m, 1H), 2.32-2.06 (m, 3H). |
| Exa mple 76 | 2-((2R,4S)-2-(2,5 -difluorophenyl)-4-fluoropyrrolidi n-1-yl)-8-(1-(pipe ridin-4-yl)-1H-py razol-4-yl)pyrimi do[5,4-d]pyrimidi ne | 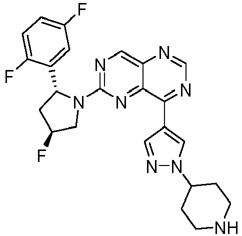 | MS (ESI): m/z = 481 [M+H]+ ¹H NMR (400 MHz, DMSO-*d*₆) δ 9.39 (s, 0.5 H), 9.14 (s, 0.5 H), 9.00 - 8.95 (m, 1.5H), 8.57 (m, 0.5H), 8.39 (s, 0.5H), 8.15 (s,0.5H), 7.35 - 7.18 (m, 1H), 7.17 - 7.02 (m, 2H), 5.67-5.37 (m, 2H), 4.61-4.03 (m, 3H), 3.13 - 2.80 (m, 3H), 2.68 - 2.55 (m, 2H), 2.40 - 2.11 (m, 2H), 2.10 - 1.73 (m, 4H). |
| Exa mple 77 | (S)-1-(3-(6-((2R, 4S)-2-(2,5-difluor ophenyl)-4-fluoro pyrrolidin-1-yl)-3 -fluoro-1,5-napht hyridin-4-yl)-1,2, 4-oxadiazol-5-yl) pyrrolidin-3-ol | 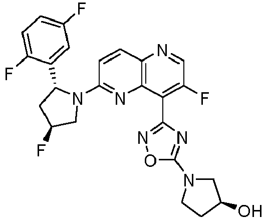 | MS (ESI): m/z = 501 [M+H]+ ¹H NMR (400 MHz, CD₃OD) δ 8.55 (s, 1H), 8.10 (d, *J* = 8.8 Hz, 1H), 7.22-6.75 (m, 4H), 5.50-5.34 (m, 2H), 4.60-4.57 (m, 1H), 4.15-4.06 (m, 2H), 3.82-3.75 (m, 3H), 3.59-3.50 (m, 1H), 2.85-2.76 (m, 1H), 2.27-2.10 (m, 3H). |
| Exa mple 78 | 6-((2R,4S)-2-(2,5 -difluorophenyl)-4-fluoropyrrolidi n-1-yl)-4-(4-(pipe ridin-4-yl)-1H-py razol-1-yl)pyrido[ 3,2-d]pyrimidine | 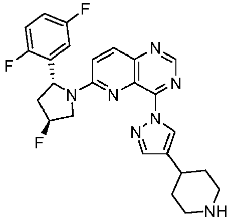 | MS (ESI): m/z = 480 [M+H]^{+ 1}H NMR (400 MHz, DMSO-*d*₆) δ 8.84 (s, 1H), 8.60 - 8.40 (m, 1H), 8.15 - 8.07 (m, 1H), 7.80 - 7.76 (m, 1H), 7.30 - 7.00 (m, 4H), 5.60 - 5.44 (m, 2H), 4.40 - 4.00 (m, 2H), 3.04 - 2.84 (m, 4H), 2.65 - 2.56 (m, 3H), 2.32 - 2.12 (m, 1H), 1.90 - 1.60 (m, 2H), 1.52 - 1.40 (m, 2H). |
| Exa mple 79 | (S)-N-(6-((2R,4S) -2-(2,5-difluorop henyl)-4-fluoropy rrolidin-1-yl)-1,5-naphthyridin-4-yl )-3-hydroxypyrro lidine-1-methylth ioamide | 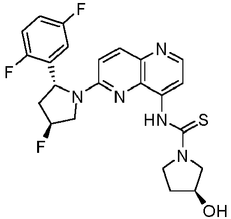 | MS (ESI): m/z = 474 [M+H]+. ¹H NMR (400 MHz, CD₃OD) δ 9.24 (d, *J* = 5.7 Hz, 1H), 8.36 (d, *J* = 5.4 Hz, 1H), 7.99 (d, *J* = 9.4 Hz, 1H), 7.29-6.80 (m, 4H), 5.63 (s, 1H), 5.44 (d, *J* = 53.2 Hz, 1H), 4.75-4.42 (m, 1H), 4.33 (brs, 1H), 4.15 (ddd, *J* = 34.6, 12.5, 3.2 Hz, 1H), 4.05-3.44 (m, 4H), 3.16-2.89 (m, 1H), 2.43-1.93 (m, 3H). |
| Exa mple 80 | 1-(3-(6-((2R,4S)-2-(2,5-difluoroph enyl)-4-fluoropyr rolidin-1-yl)pyrid o[3,2-d]pyrimidin -4-yl)-1,2,4-oxadi azol-5-yl )piperidi n-4-ol | 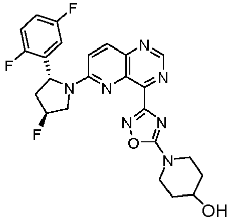 | MS (ESI): m/z = 498 [M+H]+. |
| Exa mple 81 | 3-(6-((2R,4S)-2-( 2,5-difluoropheny l)-4-fluoropyrroli din-1-yl)pyrido[3 ,2-d]pyrimidin-4-yl)-5-(piperidin-4 -yl)-1,2,4-oxadiaz ole | 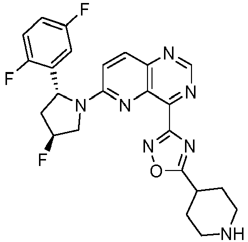 | MS (ESI): m/z = 482 [M+H]+. ¹H NMR (400 MHz, MeOD) δ 9.07 (s, 1H), 8.15-8.05 (m, 1H), 7.05-7.40 (m, 1H), 7.13-6.77 (m, 3H), 5.57-5.36 (m, 2H), 4.40-4.00 (m, 2H), 3.58-3.50 (m, 1H), 3.46-3.38 (m, 2H), 3.16-3.07 (m, 2H), 2.95-2.80 (m, 1H), 2.45-2.35 (m, 2H), 2.34-2.05 (m, 3H). |
| Exa mple 82 | (S)-1-(1-(6-((2R, 4S)-2-(2,5-difluor ophenyl)-4-fluoro pyrrolidin-1-yl)p yrido[3,2-d]pyrim idin-4-yl)-1H-pyr azol-4-yl)pyrrolid in-3-ol | 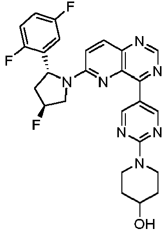 | MS (ESI): m/z = 509 [M+H]^{+ 1}H NMR (400 MHz, CD₃OD) δ 9.17 (s, 2H), 8.86 (s, 1H), 8.00 (d, *J* = 9.1 Hz, 1H), 7.33 (s, 1H), 7.13 - 7.04 (m, 1H), 6.97-6.88 (m, 2H), 5.57 - 5.37 (m, 2H), 4.67 - 4.50 (m, 2H), 4.37-4.07 (m, 2H), 3.97 - 3.88 (m, 1H), 3.50 - 3.40 (m, 2H), 3.01-2.87 (m, 1H), 2.33 - 2.12 (m, 1H), 2.04-1.91 (m, 2H), 1.63-1.46 (m, 2H). |
| Exa mple 83 | 2-((2R,4S)-2-(2,5 -difluorophenyl)-4-fluoropyrrolidi n-1-yl)-7-fluoro-8 -(1-(piperidin-4-y 1)-1H-1,2,3-triazo 1-4-yl)-1,5-naphth yridine | 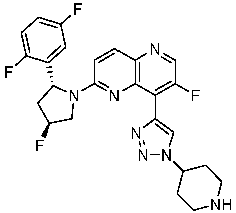 | MS (ESI): m/z =498 [M+H]+. ¹H NMR (400 MHz, CD₃OD) δ 8.56 (d, *J* = 2.1 Hz, 2H), 8.06 (d, *J* = 9.1 Hz, 1H), 7.11 (td, *J* = 9.5, 4.4 Hz, 2H), 6.90 (d, J = 28.3 Hz, 2H), 5.59 (s, 1H), 5.45 (d, *J* = 52.4 Hz, 1H), 4.73 (s, 1H), 4.32 (s, 1H), 4.16 (ddd, *J* = 35.7, 12.8, 3.2 Hz, 1H), 3.25 (s, 1H), 2.97 (dd, *J* = 22.2, 10.5 Hz, 1H), 2.88 (dd, *J* = 25.2, 13.0 Hz, 2H), 2.35-2.06 (m, 5H). |
| Exa mple 84 | 1-(3-(6-((2R,4S)-2-(2,5-difluoroph enyl)-4-fluoropyr rolidin-1-yl)-3-fl uoro-1,5-naphthy ridin-4-yl)-1,2,4-oxadiazol-5-yl)pi peridin-4-ol | 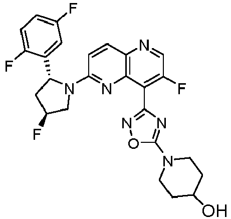 | MS (ESI): m/z =515 [M+H]+. ¹H NMR (400 MHz, CD₃OD) δ 8.55 (s, 1H), 8.10 (d, *J* = 8.8 Hz, 1H), 7.19 (s, 1H), 7.00 (s, 1H), 6.85 (d, *J* = 27.6 Hz, 2H), 5.47 (s, 1H), 5.40 (d, *J* = 39.8 Hz, 1H), 4.26-3.84 (m, 5H), 3.51 (ddd, *J* = 13.2, 9.3, 3.6 Hz, 2H), 2.80 (s, 1H), 2.15 (d, *J* = 41.2 Hz, 1H), 2.07-1.96 (m, 2H), 1.78-1.61 (m, 2H). |
| Exa mple 85 | 1-(5-(6-((2R,4S)-2-(2,5-difluoroph enyl)-4-fluoropyr rolidin-1-yl)pyrid o[3,2-d]pyrimidin e-4-yl)pyrimidin-2-yl)-3-methylpyr rolidin-3-ol | 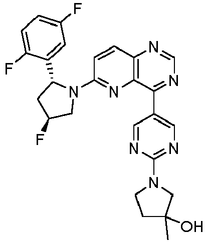 | MS (ESI): m/z =508 [M+H]+. ¹H NMR (400 MHz, CD₃OD) δ 9.27 (s, 2H), 8.88 (s, 1H), 7.99 (d, *J* = 9.1 Hz, 1H), 7.30 (s, 1H), 7.12-7.03 (m, 1H), 6.96 - 6.86 (m, 2H), 5.56 - 5.38 (m, 2H), 4.38 - 4.08 (m, 2H), 3.89-3.72 (m, 3H), 3.54 (dd, *J* = 11.9, 3.7 Hz, 1H), 3.01-2.86 (m, 1H), 2.33 - 2.15 (m, 1H), 2.13 - 2.02. (m, 2H), 1.49 (s, 3H). |
| Exa mple 86 | 6-((2R,4S)-2-(2,5 -Difluorophenyl)-4-fluoropyrrolidi n-1-yl)-4-(1-(pipe ridin-4-yl)-1H-1, 2,3-triazol-4-yl)p yrido[3,2-d]pyrim idine | 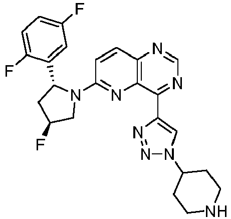 | MS (ESI): m/z = 482 [M+H]+. ¹H NMR (400 MHz, CD₃OD) δ 9.00 (s, 1H), 8.88 (s, 1H), 8.05 (d, *J* = 9.5 Hz, 1H), 7.39 (s, 1H), 7.24-7.14 (m, 1H), 7.02 - 6.92 (m, 2H), 5.75-5.67 (m, 1H), 5.58 - 5.42 (m, 1H), 4.80 - 4.70 (m, 1H), 4.44-4.17 (m, 2H), 3.41-3.31 (m, 2H), 3.12-2.92 (m, 3H), 2.38-2.15 (m, 5H). |
| Exa mple 87 | (S)-1-(5-(6-((2R, 4S)-2-(2,5-difluor ophenyl)-4-fluoro pyrrolidin-1-yl)p yrido[3,2-d]primi din-4-yl)pyrimidi n-2-yl)pyrrolidin-3-carboxylic acid | 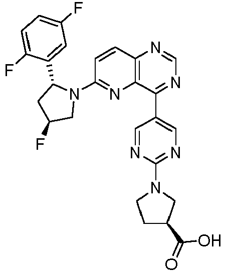 | MS (ESI): m/z = 522 [M+H]+. ¹H NMR (400 MHz, DMSO-*d*₆) δ 9.43 - 8.77 (m, 3H), 8.14 - 7.98 (m, 1H), 7.60 - 6.92 (m, 4H), 5.52-5.40 (m, 2H), 4.31 - 4.10 (m, 2H), 3.78 (d, *J* = 7.2 Hz, 2H), 3.72 - 3.56 (m, 2H), 3.18-3.12 (m, 1H), 2.95 - 2.80 (m, 1H), 2.29-2.10 (m, 3H). |
| Exa mple 88 | (R)-1-(5-(6-((2R, 4S)-2-(2,5-difluor ophenyl)-4-fluoro pyrrolidin-1-yl)p yrido[3,2-d]primi din-4-yl)pyrimidi n-2-yl)pyrrolidin-3-carboxylic acid | 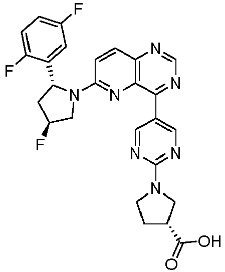 | MS (ESI): m/z = 522 [M+H]+. ¹H NMR (400 MHz, DMSO-*d*₆) δ 12.49 (s, 1H), 9.40 - 8.73 (m, 3H), 8.11 - 8.03 (m, 1H), 7.60 - 6.97 (m, 4H), 5.62 - 5.42 (m, 2H), 4.25 - 4.10 (m, 2H), 3.79 (d, *J* = 6.8 Hz, 2H), 3.71 - 3.58 (m, 2H), 3.25 - 3.18 (m, 1H), 2.93 - 2.80 (m, 1H), 2.30 - 2.10 (m, 3H). |
| Exa mple 89 | (R)-2-(2-(2-Chlor o-5-fluorophenyl) pyrrolidin-1-yl)-7 -fluoro-8-(1-(pipe ridin-4-yl)-1H-py razol -4-yl)-1,5-naphth yridine | 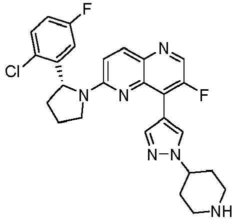 | MS (ESI): m/z = 495 [M+H]+. ¹H NMR (400 MHz, DMSO-*d*₆) δ 8.55 (s, 1H), 8.37-7.69 (m, 3H), 7.59 (dd, *J* = 8.8, 5.2 Hz, 1H), 7.36 (s, 1H), 7.16 (t, *J* = 7.1 Hz, 1H), 6.95 (d, *J* = 9.5 Hz, 1H), 5.45 (s, 1H), 4.41-3.43 (m, 4H), 3.04 (s, 2H), 2.61 (dd, *J* = 12.6, 9.4 Hz, 2H), 2.16-1.53 (m, 7H). |
| Exa mple 90 | (R)-2-(2-(2-Chlor o-5-fluoropyridin -3-yl)pyrrolidin-1 -yl)-7-fluoro-8-(1 -(piperidin-4-yl)-1H-pyrazol-4-yl)-1,5-naphthyridine | 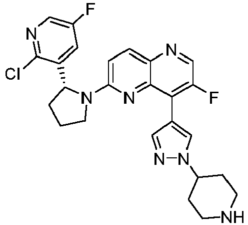 | MS (ESI): m/z = 496 [M+H]+. ¹H NMR (400 MHz, DMSO-*d*₆) δ 8.55 (s, 1H), 8.37 (s, 1H), 8.23-7.62 (m, 3H), 7.56 (d, *J* = 6.4 Hz, 1H), 7.29 (s, 1H), 5.43 (s, 1H), 4.14 (s, 2H), 3.65 (s, 1H), 3.03 (s, 2H), 2.62 (t, *J* = 11.9 Hz, 2H), 2.53 (s, 1H), 2.05 (t, *J* = 17.9 Hz, 2H), 1.98-1.56 (m, 5H). |
| Exa mple 91 | 6-((2R,4S)-2-(2,5 -Difluorophenyl)-4-fluoropyrrolidi n-1-yl)-4-(2-(pipe ridin-4-yl)pyrimi din-5-yl)pyrido[3 ,2-d]pyrimidine | 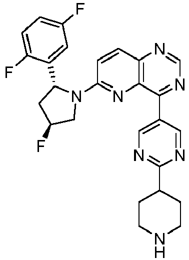 | MS (ESI): m/z = 492 [M+H]+. ¹H NMR (400 MHz, DMSO-*d*₆) δ 9.06 (s, 2H), 8.13 (s, 1H), 7.77-6.67 (m, 5H), 5.62-5.38 (m, 2H), 4.18 (d, *J* = 31.5 Hz, 2H), 3.10-2.74 (m, 4H), 2.62 (t, *J* = 11.5 Hz, 2H), 2.16 (d, *J* = 37.1 Hz, 2H), 1.92 (d, *J* = 12.5 Hz, 2H), 1.73 (dd, *J* = 23.0, 10.9 Hz, 2H). |
| Exa mple 92 | 7-(6-((2R,4S)-2-( 2,5-difluoropheny l)-4-fluoropyrroli din-1-yl)-1,5-nap hthyridin-4-yl)-2, 7-diazaspiro[3.5] nonane-1-one | 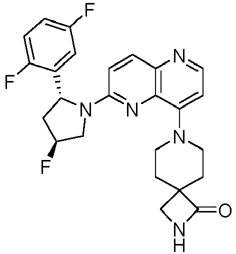 | MS (ESI): m/z = 468 [M+H]+ ¹H NMR (400 MHz, DMSO-d6) δ 8.26 (d, *J* = 5.1 Hz, 1H), 7.95 (d, *J* = 9.2 Hz, 1H), 7.76 (s, 1H), 7.27 (td, *J* = 9.5, 4.4 Hz, 1H), 7.13-7.05 (m, 1H), 7.04-6.95 (m, 2H), 6.79 (d, *J* = 5.1 Hz, 1H), 5.56-5.36 (m, 2H), 4.27-4.00 (m, 2H), 3.80-3.60 (m, 2H), 3.21-3.09 (m, 1H), 3.07-2.95 (m, 3H), 2.95-2.78 (m, 1H), 2.27-2.05 (m, 1H), 1.91-1.58 (m, 4H). |
| Exa mple 93 | (R)-6-(2-(2,5-Difl uorophenyl)pyrro lidin-1-yl)-N-(pip eridin-4-yl)-1,5-n aphthyridin-4-me thylamide | 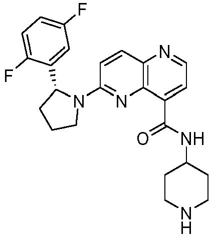 | MS (ESI): m/z =438 [M+H]+. ¹H NMR (400 MHz, CD₃OD) δ 8.64 (d, *J* = 4.6 Hz, 1H), 8.27 (d, *J* = 4.3 Hz, 1H), 8.03 (d, *J* = 9.3 Hz, 1H), 7.20 (td, *J* = 9.5, 4.3 Hz, 1H), 7.03 (ddd, *J* = 12.0, 8.2, 3.6 Hz, 1H), 6.90 (s, 1H), 6.82 (s, 1H), 5.45 (d, *J* = 7.1 Hz, 1H), 4.20-4.00 (m, 2H), 3.83 (dd, *J* = 17.1, 9.5 Hz, 1H), 3.19 (d, *J* = 9.6 Hz, 2H), 2.80 (t, *J* = 11.8 Hz, 2H), 2.61 (d, *J* = 7.2 Hz, 1H), 2.27-2.03 (m, 5H), 1.66 (ddd, *J* = 24.9, 12.5, 4.3 Hz, 2H). |
| Exa mple 94 | 2-(6-((2R,4S)-2-( 2,5-difluoropheny l)-4-fluoropyrroli din-1-yl)-1,5-nap hthyridin-4-yl)-2, 7-diazaspiro[3.5] nonane-1-one | 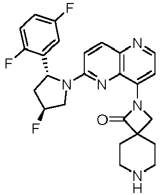 | MS (ESI): m/z =469 [M+H]⁺. ¹H NMR (400 MHz, DMSO-*d*₆) δ 8.38 (d, *J* = 5.0 Hz, 1H), 8.03 (d, *J* = 9.2 Hz, 1H), 7.96 (d, *J* = 5.0 Hz, 1H), 7.30 - 7.15 (m, 2H), 7.14-7.05 (m, 1H), 7.00 - 6.92 (m, 1H), 5.60 - 5.38 (m, 2H), 4.23 - 4.08 (m, 3H), 2.94 - 2.80 (m, 3H), 2.65-2.48 (m, 2H), 2.23 - 2.06 (m, 1H), 1.76-1.37 (m, 5H). |
| Exa mple 96 | 1-(6-((2R,4S)-2-( 2,5-difluoropheny l)-4-fluoropyrroli din-1-yl)pyrido[3 ,2-d]pyrimidin-4-yl)-3-(piperidin-4 -yl)-1,3-dihydro-2H-imidazol-2-on e | 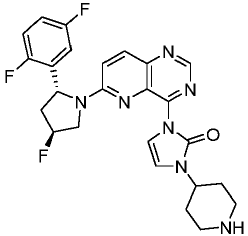 | MS (ESI): m/z = 496 [M+H]⁺. ¹H NMR (400 MHz, DMSO-*d*₆) δ 8.85 (s, 1H), 8.29 (s, 1H), 8.16 - 8.05 (m, 1H), 7.65 - 7.45 (m, 1H), 7.28 - 6.92 (m, 4H), 6.68 - 6.35 (m, 2H), 5.58 - 5.37 (m, 2H), 4.30-3.95 (m, 4H), 3.16 (d, *J* = 9.4 Hz, 2H), 2.85 - 2.70 (m, 3H), 2.26 - 2.05 (m, 1H), 1.88 - 1.75 (m, 3H). |
| Exa mple 97 | 1-(5-(6-((2R,4S)-2-(2,5-difluoroph enyl)-4-fluoropyr rolidin-1-yl)-3-fl uoro-1,5-naphthy ridin-4-yl)pyridin -2-yl)piperazin-2-one | 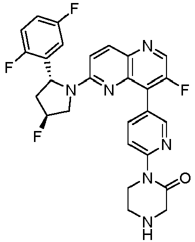 | MS (ESI): m/z = 523 [M+H]+. ¹H NMR (400 MHz, DMSO-*d*₆) δ 8.67 (s, 1H), 8.40 - 8.10 (m, 2H), 8.05 - 7.95 (m, 1H), 7.80 - 7.42 (m, 2H), 7.33-7.15 (m, 1H), 7.15 - 6.93 (m, 2H), 6.87-6.63 (m, 1H), 5.45 (d, *J* = 52.8 Hz, 1H), 5.28 (t, *J* = 8.3 Hz, 1H), 4.16-3.93 (m, 3H), 3.50 (s, 2H), 3.07 (t, *J* = 5.5 Hz, 2H), 2.98 - 2.68 (m, 2H), 2.20 - 1.95 (m, 1H), 1.67 - 1.49 (m, 1H). |
| Exa mple 100 | 2-((2R,4S)-2-(2,5 -difluorophenyl)-4-fluoropyrrolidi n-1-yl)-7-fluoro-8 -(6-((R)-2-methyl -4-(oxbutacyclo-3 -yl)piperazin-1-yl )pyridin-3-yl)-1,5 -naphthyridine | 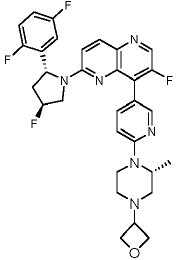 | MS (ESI): m/z = 579 [M+H]+. 1H NMR (400 MHz, CD₃OD) δ 8.49 (d, *J* = 1.6 Hz, 1H), 8.21 (s, 1H), 8.06 (d, *J* = 9.2 Hz, 1H), 7.65 - 7.55 (m, 1H), 7.15-6.65 (m, 5H), 5.47 - 5.30 (m, 2H), 4.76-4.67 (m, 3H), 4.65 - 4.55 (m, 2H), 4.26-3.96 (m, 3H), 3.54 - 3.46 (m, 1H), 3.27 - 3.23 (m, 1H), 2.95-2.71 (m, 3H), 2.26-2.01 (m, 3H), 1.36 (d, *J* = 6.7 Hz, 3H). |
| Exa mple 101 | 2-((2R,4S)-2-(2,5 -difluorophenyl)-4-fluoropyrrolidi n-1-yl)-7-fluoro-8 -(6-((S)-2-methyl -4-(oxbutacyclo-3 -yl)piperazin-1-yl )pyridin-3-yl)-1,5 -naphthyridine | 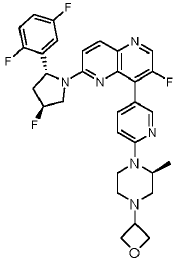 | MS (ESI): m/z = 579 [M+H]+. ¹H NMR (400 MHz, CD₃OD) δ 8.49 (d, *J* = 1.6 Hz, 1H), 8.22 (s, 1H), 8.07 (d, *J* = 8.9 Hz, 1H), 7.68 - 7.50 (m, 1H), 7.17 - 7.05 (m, 1H), 7.04 - 6.95 (m, 1H), 6.90 - 6.80 (m, 1H), 6.77 - 6.67 (m, 2H), 5.50 - 5.30 (m, 2H), 4.77-4.65 (m, 3H), 4.65 - 4.55 (m, 2H), 4.28-3.95 (m, 3H), 3.59-3.43 (m, 1H), 3.27 - 3.23 (m, 1H), 2.98-2.72 (m, 3H), 2.30-1.99 (m, 3H), 1.37 (d, J = 6.6 Hz, 3H). |

### Example 32:

### 2-(4-(4-(3-chloro-6-((2R,4S)-2-(2,5-difluorophenyl)-4-fluoropyrrolidin-1-yl)-1,5-naphthyridi n-4-yl)-1H-pyrazol-1-yl)piperidin-1-yl)ethan-1-ol

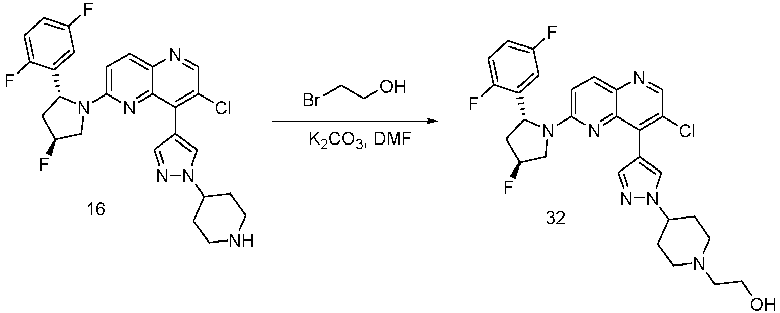

A mixture of 7-chloro-2-((2R,4S)-2-(2,5-difluorophenyl)-4-fluoropyrrolidin-1-yl)-8-(1-(piperidin-4-yl)-1H -pyrazol-4-yl)-1,5-naphthyridine (46 mg, 0.08 mmol), 2-bromoethane-1-ol (21 mg, 0.01 mmol) and potassium carbonate (35 mg, 0.25 mmol) in *N,N*-dimethylformamide (4 mL) was stirred at room temperature for 3 days. The reaction mixture was filtered, and the filtrate was concentrated and purified by reverse phase preparation column to afford 2-(4-(4-(3-chloro-6-((2R,4S)-2-(2,5-difluorophenyl)-4-fluoropyrrolidin-1-yl)-1,5-naphthyridi ne-4-yl)-1H-pyrazol-1-yl)piperidin-1-yl)ethan-1-ol (14.5 mg, yield 31%) as a white solid.

MS (ESI): m/z = 558 [M+H]+.

¹H NMR (400 MHz, CD₃OD) δ 8.50 (s, 1H), 8.25 (s, 1H), 8.04-7.80 (m, 2H), 7.10 (td, *J* = 9.3, 4.2 Hz, 1H), 7.05-6.98 (m, 1H), 6.93 (t, *J* = 8.3 Hz, 1H), 6.83 (s, 1H), 5.52-5.32 (m, 2H), 4.38-4.19 (m, 2H), 4.09 (ddd, *J* = 35.7, 12.7, 3.1 Hz, 1H), 3.71 (t, *J* = 6.0, Hz, 2H), 3.15 (d, *J* = 11.9 Hz, 2H), 2.97-2.85 (m, 1H), 2.61 (t, *J* = 6.0 Hz, 2H), 2.41-2.11 (m, 6H).

### Example 36:

### 6-((2R,4S)-2-(2,5-difluorophenyl)-4-fluoropyrrolidin-1-yl)-4-(1-(piperidin-4-yl)-1H-pyrazol-4-yl)-1,5-naphthyridin-3-carbonitrile

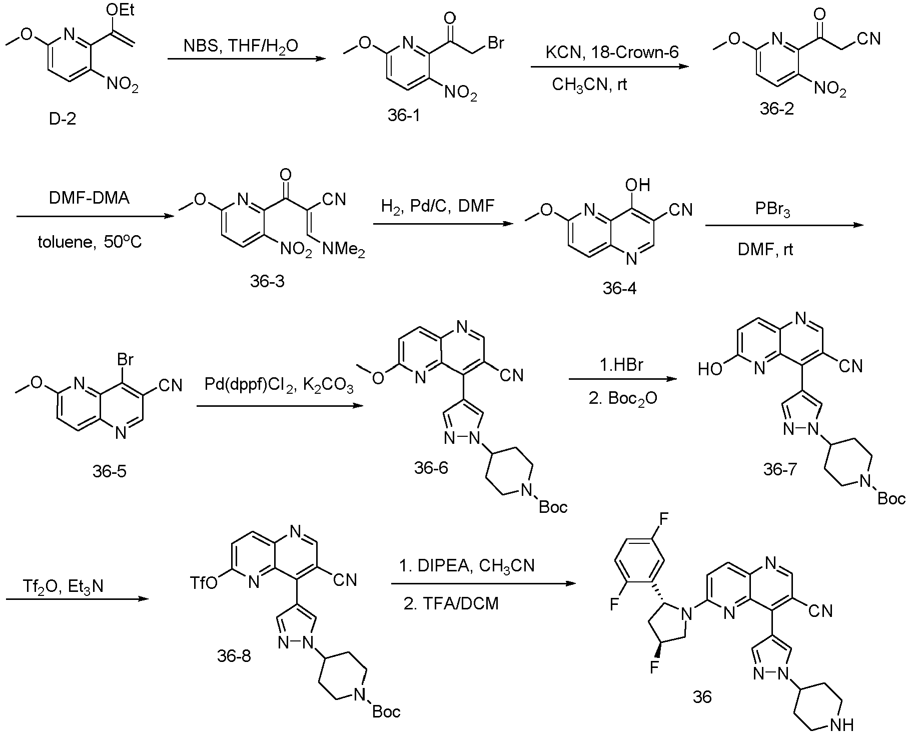

### 2-bromo-1-(6-methoxy-3-nitropyridin-2-yl)ethanone

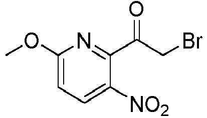

2-(1-Ethoxyvinyl)-6-methoxy-3-nitropyridine (9.5 g, 42.4 mmol) was dissolved in tetrahydrofuran (100 mL) and water (40 mL), and *N*-Bromosuccinimide (7.5 g, 42.4 mmol) was added. The reaction solution was stirred at room temperature for 16 h. After the reaction was completed, the reaction mixture was poured into ice water (200 mL), and extracted with ethyl acetate (100 mL x 2). The combined organic layer was washed with water (80 mL) and brine (80 mL), dried over with anhydrous sodium sulfate and filtered. The filtrate was dried under reduced pressure, and the crude product was purified by silica chromatography (petroleum ether / ethyl acetate = 6/1) to afford a yellow solid (3) (9.8 g, yield 85%).

MS (ESI): m/z = 275 [M+H]+.

### 3-(6-Methoxy-3-nitropyridin-2-yl)-3-carbonylpropionitrile

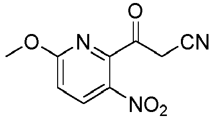

To a mixture of 2-bromo-1-(6-methoxy-3-nitropyridin-2-yl)ethanone (4.2 g, 15.2 mmol) in in toluene (40 mL) and acetonitrile (40 mL), 18-Crown-6-ether (8.1 g, 30.4 mmol) and potassium cyanide (1.98 g, 30.4 mmol) were added. The reaction mixture was stirred at room temperature for 20 minutes. After the reaction was completed, the reaction mixture was poured into ice water (80 mL), and extracted with ethyl acetate (100 mL). The organic layer was discarded. The aqueous layer was adjusted to pH=6-7 with acetic acid, and extracted with ethyl acetate twice (100 mL x 2). The combined organic layer was washed with water (80 mL), saturated brine (80 mL), and dried over anhydrous sodium sulfate and filtered. The filtrate was evaporated under reduced pressure, and the residue was purified by silica chromatography (petroleum ether/ethyl acetate = 3/1) to afford a black oil (1.1 g, yield 33%).

MS (ESI): m/z = 221 [M+H]+.

Intermediates 36-3, 36-4, 36-5 were prepared according to conditions similar to those for intermediate D.

### tert-Butyl 4-(4-(3-cyano-6-methoxy-1,5-naphthyridin-4-yl)-1H-pyrazol-1-yl)piperidin-1-carboxylate

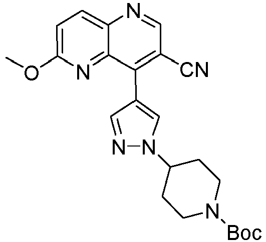

The target product was prepared according to conditions similar to that in example 16.

MS (ESI): m/z = 435 [M+H]+.

### tert-Butyl 4-(4-(3-cyano-6-hydroxy-1,5-naphthyridin-4-yl)-1H-pyrazol-1-yl)piperidin-1-carboxylate

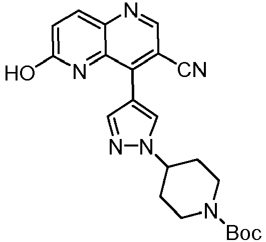

A mixture of *tert*-butyl 4-(4-(3-cyano-6-methoxy-1,5-naphthyridin-4-yl)-1H-pyrazol-1-yl)piperidin-1-carboxylate (300 mg, 0.69 mmol) in aqueous hydrobromic acid was stirred at 85 °C for 2 hours. The mixture was concentrated and the crude product was dissolved in dichloromethane (10 mL). Triethylamine (281 mg, 2.81 mmol) and di-*tert*-butyl dicarbonate (202 mg, 0.93 mmol) were added and was allowed to stir at room temperature for 1h. The resulting solution was concentrated and the crude product was purified by silica chromatography (petroleum ether / ethyl acetate = 1/3) to afford a colorless oil (170 mg) .

MS (ESI): m/z = 443 [M+H]+.

### tert-Butyl 4-(4-(3-cyano-6-(((trifluoromethyl)sulfonyl)oxo)-1,5-naphthyridin-4-yl)-1H-pyrazol-1-yl)pip eridin-1-carboxylate

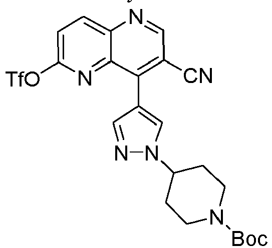

The target product was prepared according to conditions similar to those for intermediate D.

MS (ESI): m/z = 497 [M-56+H]+.

### 6-((2R,4S)-2-(2,5-difluorophenyl)-4-fluoropyrrolidin-1-yl)-4-(1-(piperidin-4-yl)-1H-pyra zol-4-yl)-1,5-naphthyridin-3-carbonitrile

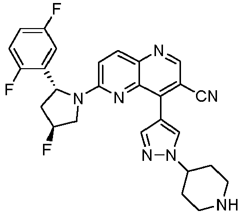

The target product was prepared according to conditions similar to that in example 16.

MS (ESI): m/z = 504 [M+H]+.

¹H NMR(400 MHz, DMSO-*d*₆) δ 8.75 (s, 1H), 8.10-7.95 (m ,3H), 7.23-7.06 (m, 4H), 5.57-5.44 (m, 2H), 4.35-4.09 (m, 3H), 3.10-2.89 (m, 2H), 2.84-2.82 (m, 1H), 2.73-2.62 (m ,2H), 2.29-1.85 (m, 5H).

### Example 38:

### 7-chloro-2-((2R,4S)-2-(2,5-difluorophenyl)-4-fluoropyrrolidin-1-yl)-8-(1-(3-methoxy-3-meth ylcyclobutyl)-1H-pyrazol-4-yl)-1,5-naphthyridine

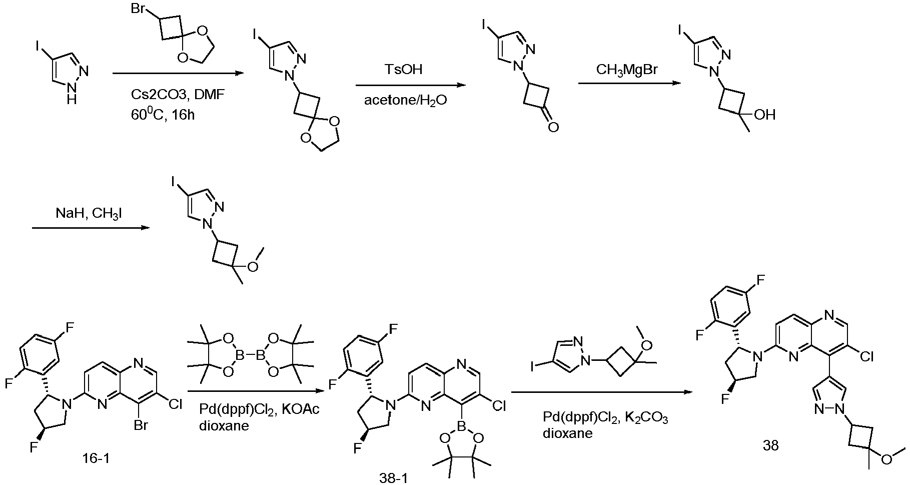

### 4-Iodo-1-(5,8-dioxaspiro[3.4]octae-2-yl)-1H-pyrazole

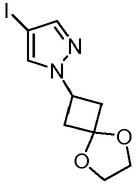

A mixture of 4-iodine-1H-pyrazole (1.67 g, 8.63 mmol), 2-bromo-5,8-dioxane[3.4]octane (2.0 g, 10.4 mmol) and cesium carbonate (5.64 g, 17.3 mmol) in *N,N*-dimethylformamide (20 mL) was heated to 60 °C and stirred at this temperature for overnight. After cooling down to room temperature, the reaction mixture was poured into ice water (80 mL), and extracted with ethyl acetate (100 mL x 2). The combined organic layer was washed with water (80 mL) and brine (80 mL), dried with anhydrous sodium sulfate and filtered. The filtrate was dried under reduced pressure, and the crude product was purified by silica chromatography (petroleum ether / ethyl acetate = 4/1) to afford a white solid (1.7 g, yield 64%).

### 3-(4-iodo-1H-pyrazol-1-yl)cyclobutan-1-one

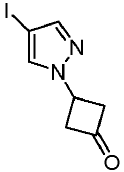

A mixture of 4-iodine-1-(5,8-dioxane[3.4]octan-2-yl)-1H-pyrazole (890 mg, 2.91 mmol) and TSOH.H₂O (110 mg, 0.58 mmol) in acetone (10 mL) and water (1 mL) was stirred to 50 °C for 2 days. The reaction solution was washed with saturated sodium bicarbonate, and extracted with ethyl acetate (100 mL x 2). The combined organic layer was washed with water (80 mL), brine (80 mL), dried over anhydrous sodium sulfate, and filtered. The filtrate was evaporated under reduced pressure to remove the solvent, and the crude product was purified by silica chromatography (petroleum ether / ethyl acetate = 2/1) to afford a colorless oil (691 mg, yield 90%).

### 3-(4-Iodo-1H-pyrazol-1-yl)-1-methylcyclobutan-1-ol

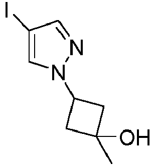

3-(4-Iodo-1H-pyrazol-1-yl)cyclobutan-1-one (691 mg, 2.64 mmol) in tetrahydrofuran (10 mL) solution was cooled to 0 °C, and the methyl Grignard reagent was added dropwise (3.1 mL, 1M in tetrahydrofuran) and the mixture was stirred at room temperature for 1h, The reaction solution was quenched with saturated ammonium chloride, and extracted with ethyl acetate (100 mL x 2). The combined organic layer was washed with water (80 mL), brine (80 mL), dried over anhydrous sodium sulfate, and filtered. The filtrate was evaporated under reduced pressure to remove the solvent, and the crude product was purified by silica chromatography (petroleum ether/ethyl acetate = 2/1) to afford a white solid (291 mg, yield 40%).

### /

### 4-Iodo-1-(3 -methoxy-3-methylcyclobutyl)-1H-pyrazole

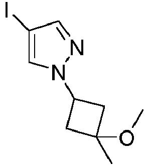

3-(4-Iodine-1H-pyrazol-1-yl)-1-methylcyclobutan-1-ol (120 mg, 0.43 mmol) was dissolved in *N*,*N*-dimethylformamide (5 ml), and sodium hydride (19 mg, 60%) was added at 0 °C, the mixture was stirred for 30 minutes. Iodide (123 mg, 0.86 mmol) was then added at room temperature for 1 h. The reaction solution was quenched with saturated ammonium chloride, and extracted with ethyl acetate (100 mL x 2). The combined organic layer was washed with water (80 mL), saturated brine (80 mL), dried over anhydrous sodium sulfate, and filtered. The filtrate was evaporated under reduced pressure to remove the solvent, and the crude product was purified by silica chromatography (petroleum ether/ethyl acetate = 4/1) to afford a white solid (100 mg, yield 79%).

### 7-Chloro-2-((2R,4S)-2-(2,5-difluorophenyl)-4-fluoropyrrolidin-1-yl)-8-(1-(3-methoxy-3-methylcyclobutyl)-1H-pyrazol-4-yl)-1,5-naphthyridine

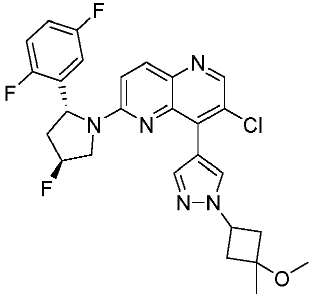

7-Chloro-2-((2R,4S)-2-(2,5-difluorophenyl)-4-fluoropyrrolidin-1-yl)-8-(1-(3-methoxy-3-methylcyclobutyl)-1H-pyrazol-4-yl)-1,5-naphthyridine was prepared using method similar to that in example 15 by replacing the corresponding starting material.

MS (ESI): m/z = 528 [M+H]+.

¹H NMR(400 MHz, CD₃OD) δ 8.50 (s, 1H), 8.22 (s, 1H), 8.05-7.87 (m, 2H), 7.14-6.96 (m, 2H), 6.90 (s, 1H), 6.79 (s, 1H), 5.56-5.32 (m, 2H), 4.72-4.60 (m, 1H), 4.33-4.19 (m, 1H), 4.10 (ddd, *J=* 36.6, 12.7, 3.2 Hz, 1H), 3.27 (s, 3H), 2.98-2.83 (m, 1H), 2.80-2.68 (m, 2H), 2.63-2.54 (m, 2H), 2.30-2.11 (m, 1H), 1.47 (s, 3H).

### Example 46:

### 6-((2R,4S)-2-(2,5-difluorophenyl)-4-fluoropyrrolidin-1-yl)-4-(1-(piperidin-4-yl)-1H-pyrazol-4-yl)pyrido[3,2-d]pyrimidine

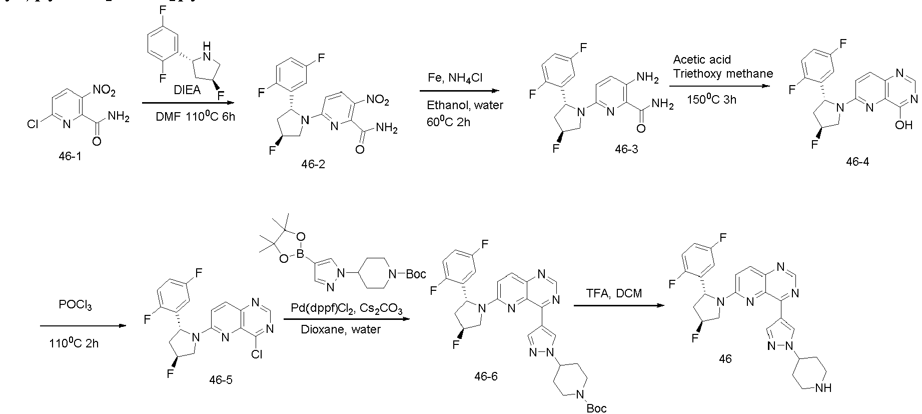

### 6-((2R,4S)-2-(2,5-difluorophenyl)-4-fluoropyrrolidin-1-yl)-3-nitropicolineamide

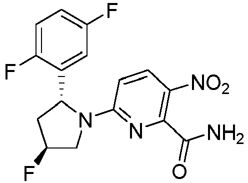

*N,N*-Diisopropylethylamine (2.1 g, 16.41 mmol) was added to a solution of 6-chloro-3-nitromethylpyridine amide (1.1 g, 5.47 mmol), (2R,4S)-2-(2,5-difluorophenyl)-4-fluoropyrrolidine (1.1 g, 5.47 mmol) in *N*, *N*-dimethylformamide (18 mL). The reaction solution was heated to 110 °C and stirred overnight. LCMS showed that the starting material was consumed. Ethyl acetate (50 mL) was added and washed with water (100 ml * 3). The organic phase was dried, concentrated, and purified by column chromatography (petroleum ether / ethyl acetate = 1/2) to afford 6-(2R,4S)-2-(2,5-difluorophenyl)-4-fluoropyrroline-1-yl)-3-nitropicolinamide (2.0 g, yield 99.8%) as a yellow solid.

MS (ESI): m/z = 367 [M+H]+.

### 3-Amino-6-((2R,4S)-2-(2,5-difluorophenyl)-4-fluoropyrrolidin-1-yl)picolinamide

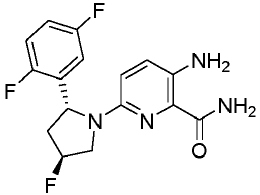

Iron powder (1.5 g, 27.3 mmol) and ammonium chloride (1.46 g, 27.3 mmol) were added to a solution of Example 726A 6-((2R,4S)-2-(2,5-difluorophenyl)-4-fluoropyrrolidin-1-yl)-3-nitropicolineamide (2.0 g, 5.46 mmol) in ethanol (40 mL) and water (10 mL). The reaction mixture was heated to 60 °C and stirred for 2h. LCMS showed that the starting material was consumed. The solution was concentrated, dichloromethane (100 mL) was added, dried, filtered, and concentrated to afford 3-amino-6-((2R,4S)-2-(2,5-difluorophenyl)-4-fluoropyrrolidin-1-yl)picolinamide (1.83 g, yield 99.7%) as a brown solid.

MS (ESI): m/z = 337 [M+H]+.

### 6-((2R,4S)-2-(2,5-difluorophenyl)-4-fluoropyrrolidin-1-yl)pyrido[3,2-d]pyrimidin-4-phe nol

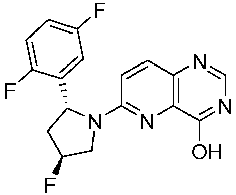

Glacial acetic acid (0.7 mL) was added to a solution of
3-amino-6-((2R,4S)-2-(2,5-difluorophenyl)-4-fluoropyrrolidin-1-yl)picolinamide (1.83 g, 5.44 mmol) in triethyl orthoformate (110 mL). The reaction mixture was heated to 150 °C and stirred for 3h. LCMS showed that the starting material was consumed. The reaction solution was concentrated, and purified by silica chromatography (petroleum ether / ethyl acetate = 1/2) to afford
6-((2R,4S)-2-(2,5-difluorophenyl)-4-fluoropyrrolidin-1-yl)pyrido[3,2-d]pyrimidin-4-phenol (1.07 g, yield 56.8%) as a brown solid.

MS (ESI): m/z = 347 [M+H]+.

### 4-chloro-6-((2R,4S)-2-(2,5-difluorophenyl)-4-fluoropyrrolidin-1-yl)pyrido[3,2-d]pyrimid ine

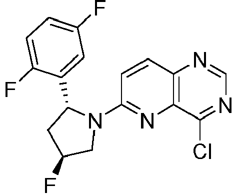

A solution of 6-(2R,4S)-2-(2,5-difluorophenyl)-4-fluoropyrrolidin-1-yl)pyridin [3,2-d]pyrimidin-4-ol (1.07 g, 3.09 mmol) in phosphoryl chloride (15 mL) was heated to 110 °C and stirred for 2h. LCMS showed that the starting material was consumed. The reaction solution was concentrated, and dichloromethane (10 mL) was added to dilute the mixture under ice bath, and then the diluted reaction solution was added into ice water (100 mL) dropwise. Dichloromethane (100 mL) was added, then washed with saturated sodium bicarbonate aqueous solution (80 mL*1) and water (80 mL*1). The organic phase was dried and concentrated to afford 4-chloro-6-((2R,
4S)-2-(2,5-difluorophenyl)-4-fluoropyrrolidin-1-yl)pyridin[3,2-d]pyrimidine (820 mg, yield 72.8%) as a brown solid.

MS (ESI): m/z = 365 [M+H]+.

### 6-((2R,4S)-2-(2,5-difluorophenyl)-4-fluoropyrrolidin-1-yl)-4-(1-(piperidin-4-yl)-1H-pyra zol-4-yl)pyrido[3,2-d]pyrimidine

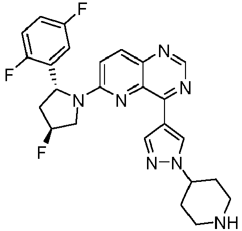

6-((2R,4S)-2-(2,5-difluorophenyl)-4-fluoropyrrolidin-1-yl)-4-(1-(piperidin-4-yl)-1H-pyra zol-4-yl)pyrido[3,2-d]pyrimidine was prepared according to conditions similar to those in example 7 by replacing the corresponding starting material.

MS (ESI): m/z = 480 [M+H]+.

¹H NMR(400 MHz, DMSO-*d*₆) δ 8.84 (s, 1H), 8.59 (s, 1H), 8.31 (s, 1H), 8.05 - 7.98 (m, 1H), 7.40 - 7.23 (m, 2H), 7.15 - 7.03 (m, 2H), 5.68 - 5.45 (m, 2H), 4.32 - 4.15 (m, 3H), 3.12 - 3.01 (m, 2H), 3.00 - 2.85 (m, 2H), 2.68 - 2.55 (m, 2H), 2.32 - 2.14 (m, 1H), 2.00 - 1.78 (m, 4H).

### Example 51:

### 3-(3-Chloro-6-((2R,4S)-2-(2,5-difluorophenyl)-4-fluoropyrrolidin-1-yl)-1,5-naphthyridine-4-yl)-5-(piperidin-4-yl)-1,2,4-oxadiazole

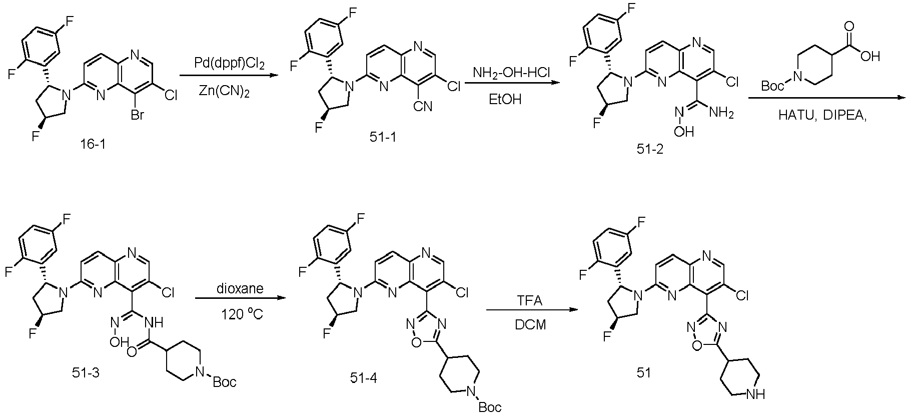

### 3-chloro-6-((2R,4S)-2-(2,5-difluorophenyl)-4-fluoropyrrolidin-1-yl)-1,5-naphthyridine-4-carbonitrile

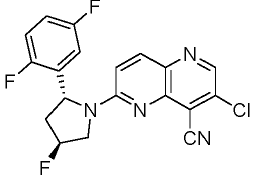

Zinc cyanide (133 mg, 1.14 mmol) and [1,1'-bis(diphenylphosphine)ferrocene]dichloride palladium dichloromethane complex (93 mg, 0.114 mmol) were added to a solution of 8-bromo-7-chloro-2-((2R,
4S)-2-(2,5-difluorophenyl)-4-fluoropyrrolidin-1-yl)-1,5-naphthyridine (503 mg, 1.14 mmol) in *N*, *N*-dimethylformamide (10 mL), the mixture was stirred at 120 °C under argon atomsphere for 16h, and cooled to room temperature. 30 mL of ethyl acetate was added, and organic phase was washed with water and brine, dried with sodium sulfate, filtered, and the filtrate was concentrated to remove the solvent. The residue was purified by silica chromatography (ethyl acetate: petroleum ether = 1: 1) to afford
3-chloro-6-((2R,4S)-2-(2,5-difluorophenyl)-4-fluoropyrrolidin-1-yl)-1,5-naphthyridine-4-car bonitrile (193 mg, yield is 43.6%) as a yellow solid.

MS (ESI): m/z = 389.0[M+H]+.

### (Z)-3-chloro-6-((2R,4S)-2-(2,5-difluorophenyl)-4-fluoropyrrolidin-1-yl)-N'-hydroxy-1,5-naphthyridin-4-carbooxamidine

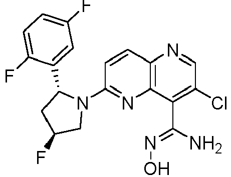

Hydroxylamine hydrochloride (52 mg, 0.746 mmol) and *N,N*-diisopropylethylamine (128 mg, 0.994 mmol) were added to a solution of
3-chloro-6-((2R,4S)-2-(2,5-difluorophenyl)-4-fluoropyrrolidin-1-yl)-1,5-naphthyridin-4-carb onitrile (193 mg, 0.497 mmol) in ethanol (5 mL), and was heated to 80 °C for 16 h. The solvent was removed under reduced pressure, and the residue was purified by silica chromatography (methanol: dichloromethane = 1: 20) to afford
(Z)-3-chloro-6-((2R,4S)-2-(2,5-difluorophenyl)-4-fluoropyrrolidin-1-yl)-N'-hydroxy-1,5-nap hthyridin-4-carbooxamidine (195 mg, yield 93.1%) as a yellow foamy solid.

MS (ESI): m/z =422.1 [M+H]+.

### tert-Butyl 4-(((Z)-(3-chloro-6-((2R,4S)-2-(2,5-difluorophenyl)-4-fluoropyrrolidin-1-yl)-1,5-naphthyridi n-4-yl)(oximino)methyl)carbamoyl)piperidin-1-carboxylate

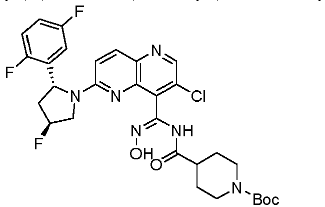

1-Boc-piperidin-4-carboxylic acid (106 mg, 0.463 mmol) was dissolved in *N,N*-dimethylformamide (5 mL), and
O-(7-azobenzotriazol-1-oxygen)-*N,N,N",N"*-tetramethylurea hexafluorophosphate (194 mg, 0.509 mmol, 1.1 equiv) and *N,N*-diisopropylethylamine (179 mg, 1.389 mmol) were added. After the mixture was allowed to react for 5 minutes at room temperature, example 758B (195 mg, 0.463 mmol) was added, and and the resulting solution was stirred for 5h at room temperature. Ethyl acetate was added, and the organic phase was washed with water and brine, dried with sodium sulfate, filtered, and the filtrate was concentrated to remove the solvent. The crude product afforded was used directly in the next step.

MS (ESI): m/z =633.2 [M+H]+.

### tert-Butyl 4-(3-(3-chloro-6-((2R,4S)-2-(2,5-difluorophenyl)-4-fluoropyrrolidin-1-yl)-1,5-naphthyridin-4 -yl)-1,2,4-oxadiazol-5-yl)piperidine-1-carboxylate

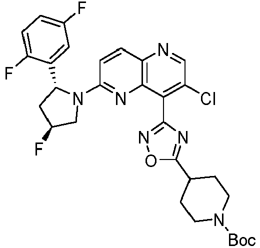

The crude product, *tert*-butyl 4-(((Z)-(3-chloro-6-((2R,4S)-2-(2,5-difluorophenyl)-4-fluoropyrrolidin-1-yl)-1,5-naphthyridi n-4-yl)(oximino)methyl)carbamoyl)piperidine-1-carboxylate was dissolved in dioxane (5 mL), and was heated to 120°C for 5h, then concentrated to remove solvent. The residue was purified by silica chromatography (ethyl acetate: petroleum ether = 1: 3) to afford the title compound (190 mg, yield of 66.7%) as a yellow solid.

MS (ESI): m/z =615.2 [M+H]+.

### 3-(3-Chloro-6-((2R,4S)-2-(2,5-difluorophenyl)-4-fluoropyrrolidin-1-yl)-1,5-naphthyridin -4-yl)-5-(piperidin-4-yl)-1,2,4-oxadiazole

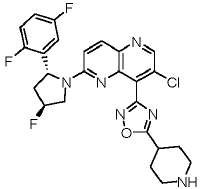

3-(3-Chloro-6-((2R,4S)-2-(2,5-difluorophenyl)-4-fluoropyrrolidin-1-yl)-1,5-naphthyridin -4-yl)-5-(piperidin-4-yl)-1,2,4-oxadiazole was prepared using a method similar to that in example 7 by replacing the corresponding starting material (94 mg, yield 59.1%, yellow solid).

MS (ESI): m/z =515.4 [M+H]+.

¹H NMR(400 MHz, CD₃OD) δ 8.61 (s, 1H), 8.11 (d, *J* = 8.7 Hz, 1H), 7.21 (s, 1H), 7.04 (s, 1H), 6.90 (s, 1H), 6.76 (s, 1H), 5.54-5.24 (m, 2H), 4.23-3.91 (m, 2H), 3.53-3.42 (m, 1H), 3.34 (d, *J* = 3.5 Hz, 2H), 3.01 (t, *J* = 12.1 Hz, 2H), 2.77 (s, 1H), 2.34 (t, *J* = 16.2 Hz, 2H), 2.23-1.92 (m, 3H).

### Example 63:

### 7-chloro-2-((2R,4S)-2-(2,5-difluorophenyl)-4-fluoropyrrolidin-1-yl)-8-(6-(piperidin-4-yl)pyri din-3-yl)-1,5-naphthyridine

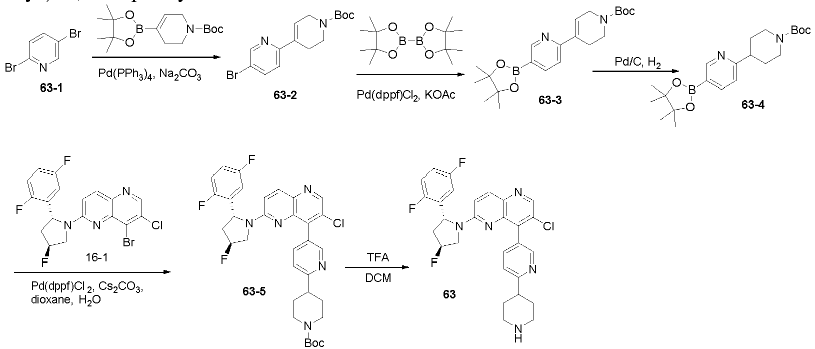

### tert-Butyl 5-bromo-3',6'-dihydro-[2,4'-bipyridin]-1'(2'H)-carboxylate

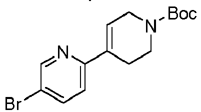

Sodium carbonate solution (7 mL, 14.0 mmol) and tetrakis (triphenylphosphine) palladium (0.817 g, 1.0 mmol) were added to mixture of 2,5-dibromopyridine (1.63 g, 10.0 mmol) and *tert*-butyl
4-(4,4,5,5-tetramethyl-1,3,2-dioxaborolan-2-yl)-3,6-dihydropyridin-1(2H)-carboxylate (3.40 g, 11.0 mmol) in dioxane (30 mL), and the mixture was heated to 100°C under argon for 16 h. The mixture was cooled to room temperature, 30 mL of ethyl acetate was added for dilution, and the organic phase was washed with water and brine, and dried with sodium sulfate. The mixture was filtered and concentrated to remove solvent. The residue was purified by silica chromatography (ethyl acetate: petroleum ether = 1: 5) to afford tert-butyl 5-bromo-3',6'-dihydro-[2,4'-bipyridin]-1'(2'H)-carboxylate (2.7 g, yield 79.1%) as a yellow oil.

MS (ESI): m/z =283 [M-56+H]+.

### tert-Butyl 5-(4,4,5,5-tetramethyl-1,3,2-dioxaborolan-2-yl)-3',6'-dihydro-[2,4'-bipyridin]-1'(2'H)-carbox ylate

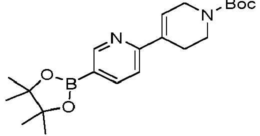

Pinacol diborate (1.52 g, 6.0 mmol), potassium acetate (1.47 g, 15.0 mmol) and [1,1'-bis(diphenylphosphine)ferrocene]palladium dichloride dichloromethane complex (0.41 g, 0.5 mmol) were added to a solution of *tert*-butyl
5-bromo-3',6'-dihydro-[2,4'-bipyridine]-1'(2'H)-carboxylate (1.69 g, 5.0 mmol) in dioxane (10 mL), and the mixture was heated to 100 °C under argon for 16 h. Ethyl acetate was added for dilution, and the organic phase was washed with water and brine, and dried with sodium sulfate. The mixture was filtered and the filtrate was concentrated to remove solvent. The residue was purified by silica chromatography (methanol: dichloromethane = 1: 20) to afford tert-butyl
5-(4,4,5,5-tetramethyl-1,3,2-dioxaborolan-2-yl)-3',6'-dihydro-[2,4'-bipyridine]-1'(2'H)-carbox ylate (1.49 g, yield 77.4%), as a black solid.

MS (ESI): m/z =305 [boric acid+H]+.

### tert-Butyl 4-(5-(4,4,5,5-tetramethyl-1,3,2-dioxaborolan-2-yl)pyridin-2-yl)piperidin-1-carboxylate

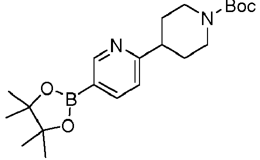

Palladium on carbon (100 mg) was added to a solution of *tert*-butyl 5-(4,4,5,5-tetramethyl-1,3,2-dioxaborolan-2-yl)-3',6'-dihydro-[2,4'-bipyridin)-1'(2'H)-carbox ylate (309 mg, 0.8 mmol) in methanol (10 mL), and the mixture was stirred under hydrogen atmosphere at room temperature for 2h. The mixture was filtered, and the filtrate was concentrated under reduced pressure to afford *tert*-butyl
4-(5-(4,4,5,5-tetramethyl-1,3,2-dioxaborolan-2-yl)pyridin-2-yl)piperidin-1-carboxylate (279 mg, yield is 90%) as a brown solid.

MS (ESI): m/z =307 [Boric acid+H]+.

### 7-Chloro-2-((2R,4S)-2-(2,5-difluorophenyl)-4-fluoropyrrolidin-1-yl)-8-(6-(piperidin-4-yl )pyridin-3-yl)-1,5-naphthyridine

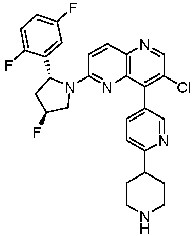

7-Chloro-2-((2R,4S)-2-(2,5-difluorophenyl)-4-fluoropyrrolidin-1-yl)-8-(6-(piperidin-4-yl )pyridin-3-yl)-1,5-naphthyridine was prepared using a method similar to that in example 16 (206mg, 92.4%, yellow solid).

MS (ESI): m/z =524 [M+H]+.

¹H NMR(400 MHz, CD₃OD) δ 8.59 (s, 1H), 8.38 (s, 1H), 8.11 (d, *J* = 9.0 Hz, 1H), 7.59 (s, 1H), 7.38 (s, 1H), 7.19 (s, 1H), 6.90 (d, *J* = 38.5 Hz, 2H), 6.55 (s, 1H), 5.49-5.17 (m, 2H), 4.20-3.92 (m, 2H), 3.59 (d, *J* = 12.6 Hz, 2H), 3.26 (s, 1H), 3.25-3.16 (m, 2H), 2.74 (s, 1H), 2.38-2.22 (m, 2H), 2.22-2.08 (m, 2H), 2.01 (s, 1H).

### Example 69:

### (S)-N-(6-((2R,4S)-2-(2,5-difluorophenyl)-4-fluoropyrrolidin-1-yl)-1,5-naphthyridin-4-yl)-3-h ydroxypyrrolidin-1-sulfonamide

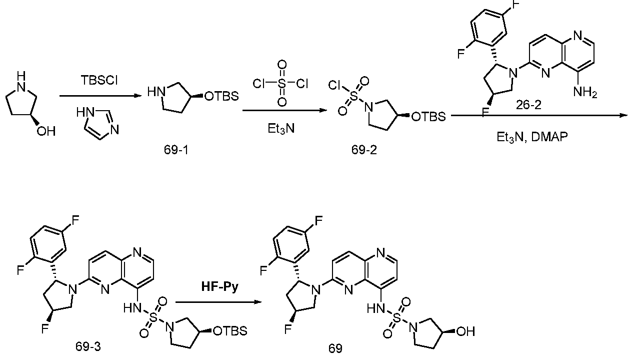

### (S)-3-((tert-butyldimethylsilyl)oxo)pyrrolidine

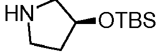

*tert*-Butyldimethylchlorosilane (2.16 g, 14.4 mmol) was added dropwise to a solution of (S)-pyrrolidin-3-ol (1.044 g, 12.0 mmol) and imidazole (1.632 g, 24.0 mmol) in dichloromethane (20 mL), and the mixture was stirred for 15h at room temperature, then saturated sodium bicarbonate was added and extracted with dichloromethane. The organic phase was dried over sodium sulfate, filtered and concentrated to afford 2.2 g product as a yellow oil, which was directly used in the next step.

MS (ESI): m/z = 202 [M+H]+.

### (S)-3-((tert-Butyldimethylsilyl)oxo)pyrrolidin-1-sulfonyl chloride

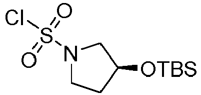

Under ice bath, sulfonyl chloride (2.68 g, 20 mmol) was added in batches to a solution of (S)-3-((tert-butyldimethylsilyl)oxo)pyrrolidine (2.01 g, 10 mmol),triethylamine (3.03 g, 30.0 mmol) in dichloromethane (20 mL), and for the resulting solution was allowed to stir for 1h in ice bath, and then at room temperature for 5h. Water was added to quench the reaction, extracted with dichloromethane, the combined organic phase was washed with water and brine, and dried with sodium sulfate. The mixture was filtered and concentrated, and the residue was purified by silica chromatography (ethyl acetate: petroleum ether = 1: 6) to afford the title compound (350 mg, yield 11.7%) as a yellow oil.

### (S)-3-((tert-butyldimethylsilyl)oxo)-N-(6-((2R,4S)-2-(2,5-difluorophenyl)-4-(fluoropyrro lidin-1-yl)-1,5-naphthalazin-4-yl)pyrrolidin-1-sulfonamide

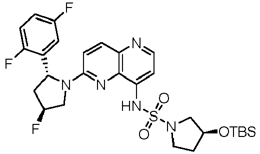

Triethylamine (101 mg, 5 mmol) and 4-dimethylaminopyridine (24 mg, 0.2 mmol) were added to a solution of (S)-3-((*tert*-butyldimethylsilyl)oxo)pyrrolidin-1-sulfonyl chloride (299 mg, 1 mmol) and
6-((2R,4S)-2-(2,5-difluorophenyl)-4-fluoropyrrolidin-1-yl)-1,5-naphthyridin-4-amine (69 mg, 0.2 mmol) in dichloromethane (5 mL), and the solution was heated tot 40 °C for 16 h. 10 ml of dichloromethane was added, and the organic phase was washed with water and saturated brine, dried with sodium sulfate, filtered and concentrated. The residue was purified by silica chromatography (ethyl acetate: petroleum ether = 1: 3) to afford titled compound (S)-3-((*tert*-butyldimethylsilyl)oxo)-N-(6-((2R,4S)-2-(2,5-difluorophenyl)-4-(fluoropyrrolidi n-1-yl)-1,5-naphthyridin-4-yl)pyrrolidin-1-sulfonamide (40 mg, yield 32.5%), as a yellow oil.

MS (ESI): m/z = 608 [M+H]+.

### (S)-N-(6-((2R,4S)-2-(2,5-difluorophenyl)-4-fluoropyrrolidin-1-yl)-1,5-naphthyridin-4-yl) -3-hydroxypyrrolidin-1-sulfonamide

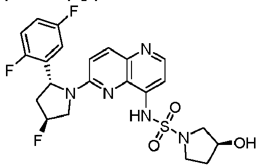

Hydrofluoric acid in pyridine (0.3 mL) was added to a solution of example 816C (40 mg, 0.065 mmol) in tetrahydrofuran (1mL) and the mixture was stirred at room temperature for 1h. The solvent was removed by rotatory evaporation, and the residue was purified by high performance liquid phase to afford the title compound 816
(S)-N-(6-((2R,4S)-2-(2,5-difluorophenyl)-4-fluoropyrrolidin-1-yl)-1,5-naphthyridin-4-yl)-3-h ydroxypyrrolidin-1-sulfonamide (25 mg, yield 78.4%) as a white solid.

MS (ESI): m/z = 494 [M+H]+.

¹H NMR (400 MHz, CD₃OD) δ 8.33 (d, *J* = 5.1 Hz, 1H), 8.01 (d, *J* = 9.2 Hz, 1H), 7.45 (d, *J* = 5.2 Hz, 1H), 7.28-7.12 (m, 2H), 7.04 (ddd, *J* = 8.9, 5.7, 3.2 Hz, 1H), 7.01-6.91 (m, 1H), 5.60-5.38 (m, 2H), 4.34-4.09 (m, 3H), 3.45-3.32 (m, 2H), 3.26-3.20 (m, 1H), 2.95-2.79 (m, 1H), 2.35-2.14 (m, 1H), 1.97-1.84 (m, 1H), 1.80 (s, 1H).

### Example 95:

### 1-(6-((2R,4S)-2-(2,5-difluorophenyl)-4-fluoropyrrolidin-1-yl)-1,5-naphthyridin-4-yl)-3-(pipe ridin-4-yl)-1,3-dihydro-2H-imidazol-2-one

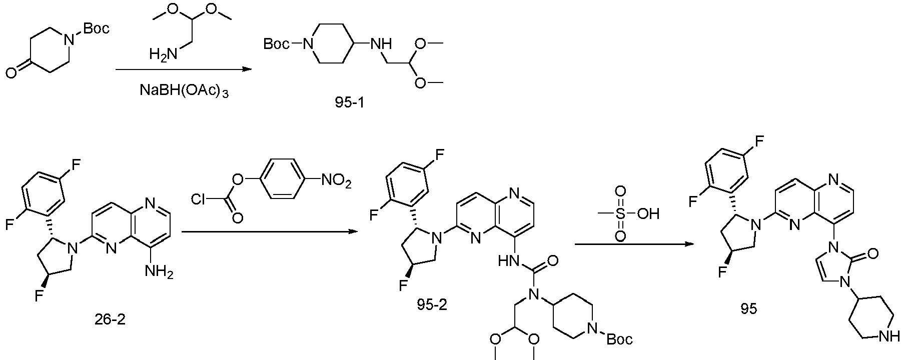

### tert-Butyl 4-((2,2-dimethoxyethyl)amino)piperidin-1-carboxylate

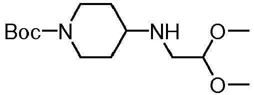

2,2-Dimethoxyethan-1-amine (1.47 g, 14 mmol) was added to a solution of *tert-*butyl 4-carbonylpiperidin-1-carboxylate (1.99 g, 10 mmol) in 1,2-dichloroethane (20 mL), and the resulting solution was heated to refluxed for 2 h, then cooled to room temperature, and sodium triacetoxyborohydride (3.18 g, 15 mmol) was added. The reaction mixture was stirred for 16 h at room temperature. The mixture was filtered, and filtrate was extracted with diluted hydrochloric acid. The aqueous phase was neutralized by an aqueous solution of sodium bicarbonate, extracted three times with dichloromethane, and the organic phase was dried over sodium sulfate. After concentration, the residue was purified by silica chromatography (methanol: dichloromethane = 1: 10) to afford *tert*-butyl
4-((2,2-dimethoxyethyl)amino)piperidin-1-carboxylate (1.2 g, yield 41.6%) as a colorless oil.

MS (ESI): m/z =289 [M+H]+.

### tert-Butyl 4-(3-(6-((2R,4S)-2-(2,5-difluorophenyl)-4-fluoropyrrolidin-1-yl)-1,5-naphthyridin-4-yl)-1-(2, 2-dimethoxyethyl)ureido)piperidin-1-carboxylate

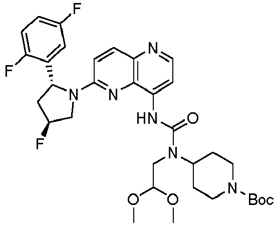

*N,N*-Diisopropylethylamine (150 mg, 1.165 mmol) and *p*-nitrophenyl chloroformate (104 mg, 0.513 mmol) was added to a solution of
6-((2R,4S)-2-(2,5-difluorophenyl)-4-fluoropyrrolidin-1-yl)-1,5-naphthyridin-4-amine (80 mg, 0.233 mmol) in dichloromethane (5 mL),the resulting mixture was stirred at room temperaturefor 16 h. tert-Butyl 4-((2,2-dimethoxyethyl)amino)piperidin-1-carboxylate (168 mg, 0.583 mmol) was added, and the mixture was stirred for another 1h at room temperature. Water and dichloromethane were added, and the organic phase was washed with water and brine, dried with sodium sulfate, and purified by silica chromatography (ethyl acetate: petroleum ether = 2: 1) to afford tert-butyl
4-(3-(6-((2R,4S)-2-(2,5-difluorophenyl)-4-fluoropyrrolidin-1-yl)-1,5-naphthyridin-4-yl)-1-(2, 2-dimethoxyethyl)ureido)piperidin-1-carboxylate (104 mg, yield 64.4%) as a colorless solid.

MS (ESI): m/z =659 [M+H]+.

### 1-(6-((2R,4S)-2-(2,5-difluorophenyl)-4-fluoropyrrolidin-1-yl)-1,5-naphthyridin-4-yl)-3-( piperidin-4-yl)-1,3-dihydro-2H-imidazol-2-one

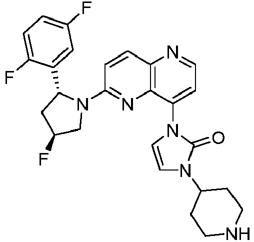

tert-Butyl 4-(3-(6-((2R,4S)-2-(2,5-difluorophenyl)-4-fluoropyrrolidin-1-yl)-1,5-naphthyridin-4-yl)-1-(2, 2-dimethoxyethyl)ureido)piperidin-1-carboxylate (104 mg, 0.158 mmol) was added to methanesulfonic acid (1 mL) and water (1 mL), and the mixture was heated to 100°C for 2h. The reaction solution was cooled to room temperature and neutralized by an aqueous solution of sodium carbonate. Acetate was added and the organic phase was washed with water and brine, dried with sodium sulfate, and concentrated by rotatory evaporation to obtain a residue which was purified by reverse phase column to afford
1-(6-((2R,4S)-2-(2,5-difluorophenyl)-4-fluoropyrrolidin-1-yl)-1,5-naphthyridin-4-yl)-3-(pipe ridin-4-yl)-1,3-dihydro-2H-imidazol-2-one (35 mg, yield 44.3%) as a white solid.

MS (ESI): m/z = 495 [M+H]+.

¹H NMR (400 MHz, CD₃OD) δ 8.55 (d, *J* = 5.0 Hz, 1H), 8.11 (d, *J* = 9.0 Hz, 1H), 7.81 (d, *J* = 4.9 Hz, 1H), 7.22 (s, 1H), 7.12 (td, *J* = 9.4, 4.2 Hz, 1H), 6.91 (d, *J* = 19.4 Hz, 2H), 6.82-6.63 (m, 1H), 6.57 (s, 1H), 5.58-5.31 (m, 2H), 4.12 (dd, *J* = 37.3, 12.5 Hz, 3H), 3.23 (d, *J=* 1.5 Hz, 1H), 2.87 (t, *J=* 11.2 Hz, 3H), 2.28-1.81 (m, 6H).

### Examples 98:

### (R)-2-((6-((2R,4S)-2-(2,5-difluorophenyl)-4-fluoropyrrolidin-1-yl)-1,5-naphthyridin-4-yl)amino)-5,6-dihydro-4H-1,3-oxazin-5-ol

### Example 99:

### (S)-2-((6-((2R,4S)-2-(2,5-difluorophenyl)-4-fluoropyrrolidin-1-yl)-1,5-naphthyridin-4-yl)amino-5,6-dihydro-4H-1,3-oxazin-5-ol

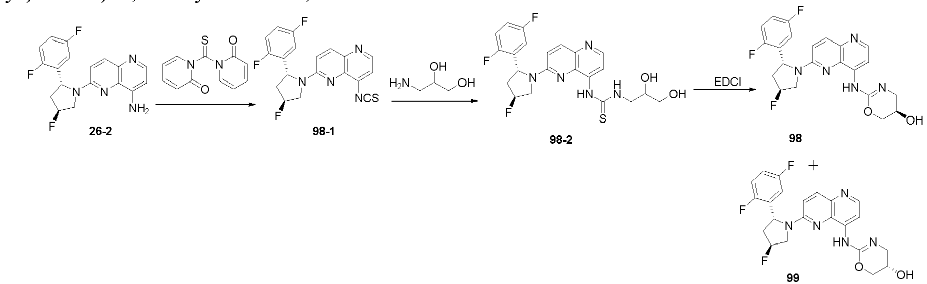

### 2-((2R,4S)-2-(2,5-difluorophenyl)-4-fluoropyrrolidin-1-yl)-8-isothiocyano-1,5-naphthyri dine

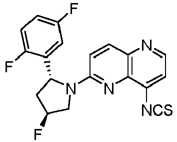

1,1'-Thiocarbonylbis(pyridin-2(1H)-one) (59 mg, 0.256 mmol) was added to a solution of 6-((2R,4S)-2-(2,5-difluorophenyl)-4-fluoropyrrolidin-1-yl)-1,5-naphthyridin-4-amine (80 mg, 0.233 mmol) in dichloromethane (5 mL) and the resulting solution was heated to 40°C for 3h, and the crude product was directly used in the next step.

MS (ESI): m/z = 387 [M+H]⁺.

### 1-(6-((2R,4S)-2-(2,5-difluorophenyl)-4-fluoropyrrolidin-1-yl)-1,5-naphthyridin-4-yl)-3-( 2,3-dihydroxypropyl)thiourea

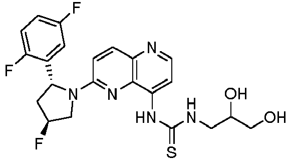

3-Aminopropan-1,2-diol (106 mg, 1.165 mmol) was added to the reaction mixture of example 882A, and reacted for 1h at room temperature. The solvent was removed by rotatory evaporation, and residue was purified by reverse phase column to afford 1-(6-((2R,4S)-2-(2,5-difluorophenyl)-4-fluoropyrrolidin-1-yl)-1,5-naphthyridin-4-yl)-3-(2,3-dihydroxypropyl)thiourea (80 mg, yield 71.7%) as a yellow solid.

MS (ESI): m/z = 478.1 [M+H]⁺.

### (R)-2-((6-((2R,4S)-2-(2,5-difluorophenyl)-4-fluoropyrrolidin-1-yl)-1,5-naphthyridin-4-yl)amino)-5,6-dihydro-4H-1,3-oxazin-5-ol

### (S)-2-((6-((2R,4S)-2-(2,5-difluorophenyl)-4-fluoropyrrolidin-1-yl)-1,5-naphthyridin-4-yl)amino)-5,6-dihydro-4H-1,3-oxazin-5-ol

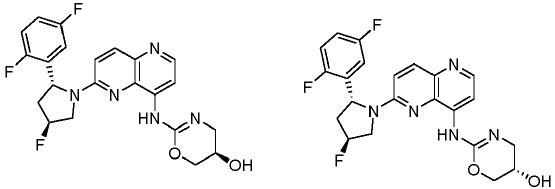

1-(3-Dimethylaminopropyl)-3-ethylcarbodiimide hydrochloride (80 mg, 0.418 mmol) and triethylamine (59 mg, 0.585 mmol) were added to a solution of
1-(6-((2R,4S)-2-(2,5-difluorophenyl)-4-fluoropyrrolidin-1-yl)-1,5-naphthyridin-4-yl)-3-(2,3-dihydroxypropyl)thiourea (80 mg, 0.167 mmol) in acetonitrile (3 mL), and for the mixture was heated to 40 °C for 16h. The mixture was cooled to room temperature, and ethyl acetate and water were added. The organic phase was washed with water and brine, and dried over sodium sulfate and concentrated. The residue was purified by preparative high performance liquid chromatography to afford
(R)-2-((6-((2R,4S)-2-(2,5-difluorophenyl)-4-fluoropyrrolidin-1-yl)-1,5-naphthyridin-4-yl)am ino)-5,6-dihydro-4H-1,3-oxazin-5-ol (14 mg, yield 18.5 %) as a white solid.

MS (ESI): m/z = 444 [M+H]⁺.

¹H NMR (400 MHz, CD₃OD) δ 8.32 (d, *J* = 5.3 Hz, 1H), 8.02-7.92 (m, 2H), 7.19-7.05 (m, 3H), 6.99-6.89 (m, 1H), 5.57-5.38 (m, 2H), 4.77 (t, *J* = 12.4 Hz, 1H), 4.29-4.08 (m, 2H), 3.98 (dd, *J=* 12.7, 9.4 Hz, 1H), 3.87-3.74 (m, 2H), 3.68 (dd, *J* = 12.4, 5.0 Hz, 1H), 2.94-2.80 (m, 1H), 2.38-2.18 (m, 1H).

Another isomer
(S)-2-((6-((2R,4S)-2-(2,5-difluorophenyl)-4-fluoropyrrolidin-1-yl)-1,5-naphthyridin-4-yl)ami no)-5,6-dihydro-4H-1,3-oxazin-5-ol (14 mg, yield 18.5%) was also obtained as a white solid.

MS (ESI): m/z = 444 [M+H]⁺.

¹H NMR (400 MHz, CD₃OD) δ 8.32 (d, *J* = 5.3 Hz, 1H), 7.98 (t, *J* = 7.7 Hz, 2H), 7.23 (td, *J* = 9.5, 4.3 Hz, 1H), 7.15-7.01 (m, 2H), 6.98-6.90 (m, 1H), 5.58-5.38 (m, 2H), 4.82-4.74 (m, 1H), 4.27-4.09 (m, 2H), 4.00 (dd, *J* = 12.8, 9.5 Hz, 1H), 3.83-3.65 (m, 3H), 2.94-2.80 (m, 1H), 2.37-2.16 (m, 1H).

### Biological test example 1 in vitro activity test on TRKA, TRKB, TRKC kinase

### Experimental Materials

Recombinant human TRKA, TRKB, TRKC proteins were purchased from Carna Biosciences. HTRF KinEASE TK kit was purchased from Cisbio Bioassays. Synergy Neo 2 of Biotek was used to read the plate.

### Experimental method

The tested compound was subjected to 3-fold serial dilution to reach a final concentration of 1 µM to 0.05 nM (10 concentrations), duplicates for each concentration; and the DMSO concentration in the detection reaction was 1%.

### TRKA enzyme reaction:

0.2 ng/µL TRKA protein kinase, 1 µM TK Substrate-biotin polypeptide substrate, 14.68 µM ATP, 1×enzymatic buffer, 5 mM MgCl₂, and 1 mM DTT. The detection plate was White Proxiplate384 -Plus plate (PerkinElmer), and incubated at room temperature for 40 min, and the assay volume was 10 µL.

### TRKB enzyme reaction:

0.037 ng/µL TRKB protein kinase, 1 µM TK Substrate-biotin polypeptide substrate, 4.77 µM ATP, 1×enzymatic buffer, 5 mM MgCl₂, and 1 mM DTT. The detection plate was White Proxiplate 384-Plus plate (PerkinElmer), and incubated at room temperature for 50 min, and the assay volume was 10 µL.

### TRKC enzyme reaction:

0.037 ng/µL TRKC protein kinase, 1 µM TK Substrate-biotin polypeptide substrate, 25.64 µM ATP, 1×enzymatic buffer, 5 mM MgCl₂, and 1 mM DTT. The detection plate was White Proxiplate 384-Plus plate (PerkinElmer), and incubated at room temperature for 40 min, and the assay volume was 10 µL.

### Detection step:

10 µL of detection reagent was added to the plate (containing 0.125 µM SA-XL665 and 5 µL 1×TK-Antibody) and incubated overnight at room temperature, and Synergy Neo 2 was used to read the plate.

### Data analysis

The 665/620 Ratio was converted according to the following formula into inhibition rate (%) = (1-Ratioₜₑₛₜ/Ratioₘₐₓ)×100%. Ratioₘₐₓ was a positive control without tested compound, and Ratiotest was the value of each concentration of different compounds. IC50 (nM) data was obtained by 4 parameter curve fitting (see Table 1).

### Biological Test Example 2 In vitro activity test on mutant TRKA (G595R), TRKA (G667C) and TRKC (G623R)

### Experimental Materials

The recombinant human TRKA (G595R), TRKA (G667C) and TRKC (G623R) proteins were purchased from SignalChem. HTRF kinEASE TK kit was purchased from CisbioBioassays. Synergy Neo 2 of Biotek was used to read the plate.

### Experimental method

The tested compound was subjected to 4-fold serial dilution to reach a final concentration of 1 µM to 0.004 nM (10 concentrates), duplicates for each concentration; and 1% DMSO was present in the detection reaction.

### TRKA (G595R) enzyme reaction:

0.12 ng/µL TRKA (G595R) kinase, 1 µM TK Substrate-biotin polypeptide substrate, 4.5 µM ATP, 1×enzymatic buffer, 5 mM MgCl₂, and 1 mm DTT. The detection plate was White Proxiplate384 -Plus plate (PerkinElmer), and incubated at room temperature for 30min, and the assay volume was 10 µL.

### TRKA (G667C) enzyme reaction:

0.026 ng/µL TRKA (G667C) kinase, 1 µM TK Substrate-biotin polypeptide substrate, 5.5 µm ATP, 1×enzymatic buffer, 5 mM MgCl₂, and 1 mM DTT. The detection plate was White Proxiplate 384-Plus plate (PerkinElmer), and incubated at room temperature for 30 min, the assay volume was 10 µL.

### TRKC (G623R) enzyme reaction:

1.0 ng/µL TRKC (G623R) kinase, 1 µM TK Substrate-biotin polypeptide substrate, 62.9 µM ATP, 1×enzymatic buffer, 5 mM MgCl₂, and 1 mM DTT. The detection plate was White Proxiplate 384-Plus plate (PerkinElmer), and incubated at room temperature for 50 min, and the assay volume was 10 µL.

### Detection step:

10 µL of detection reagent was added to the plate (containing 0.125 µM SA-XL665 and 5 µL 1×TK-Antibody) and incubated overnight at room temperature, and Synergy Neo 2 was used to read the plate.

### Data analysis

The value of 665/620 Ratio minus the value of negative control wells without enzyme, then the obtained value was converted according to the following formula into inhibition rate (%) = (1-Ratioₜₑₛₜ/ Ratioₘₐₓ)×100%. Ratioₘₐₓ was a positive control without tested compound, and Ratiotest was the value of each concentration of different compounds. IC50 (nM) data was obtained by 4 parameter curve fitting (see Table 1).

**Table 1**

| Compound | TRKA(n M) | TRKB (nM) | TRKC (nM) | TRKA (G595R) (nM) | TRKA (G667C) (nM) |
|---|---|---|---|---|---|
| Example 1 | <10 | <50 | <10 | <50 | |
| Example 2 | <10 | <10 | <10 | <10 | <10 |
| Example 3 | <150 | <500 | <500 | | |
| Example 4 | <10 | <10 | <10 | | |
| Example 5 | <100 | <500 | <150 | | |
| Example 6 | <100 | <500 | <500 | | |
| Example 7 | <50 | <100 | <50 | <1 | <1 |
| Example 8 | <10 | <1 | <10 | | |
| Example 9 | <50 | <50 | <50 | | |
| Example 10 | <1 | <1 | <1 | <1 | <100 |
| Example 11 | <1 | <1 | <1 | <1 | <1 |
| Example 12 | <1 | <1 | <1 | <1 | <1 |
| Example 13 | <1 | <1 | <1 | <1 | <10 |
| Example 14 | <1 | <10 | <1 | <1 | <50 |
| Example 15 | <1 | <1 | <1 | <1 | <10 |
| Example 16 | <1 | <1 | <1 | <10 | <1 |
| Example 17 | <1 | <10 | <10 | <10 | <50 |
| Example 18 | <1 | <1 | <1 | <10 | <10 |
| Example 19 | <10 | <500 | <500 | | |
| Example 20 | <1 | <1 | <1 | <1 | <1 |
| Example 21 | <1 | <1 | <1 | <1 | <10 |
| Example 22 | <1 | <1 | <1 | <1 | <1 |
| Example 23 | <1 | <1 | <10 | <1 | <1 |
| Example 24 | <1 | <1 | <10 | <1 | <10 |
| Example 25 | <10 | <1 | <1 | <1 | <10 |
| Example 26 | <1 | <1 | <1 | <1 | <1 |
| Example 27 | <1 | <1 | <1 | <1 | <50 |
| Example 28 | <10 | <10 | <10 | <10 | <10 |
| Example 29 | <1 | <1 | <1 | <1 | <1 |
| Example 30 | <1 | <10 | <10 | <1 | <10 |
| Example 31 | <1 | <10 | <10 | <10 | <100 |
| Example 32 | <1 | <1 | <1 | <1 | <1 |
| Example 33 | <1 | <10 | <50 | <10 | <10 |
| Example 34 | <1 | <100 | <50 | <50 | <10 |
| Example 35 | <1 | <1 | <1 | <10 | <10 |
| Example 36 | <1 | <1 | <1 | <10 | <10 |
| Example 37 | <1 | <10 | <10 | <10 | <50 |
| Example 38 | <1 | <10 | <10 | <50 | <50 |
| Example 39 | <1 | <1 | <1 | <10 | <10 |
| Example 40 | <1 | <1 | <1 | <1 | <1 |
| Example 41 | <1 | <1 | <1 | <10 | <1 |
| Example 42 | <1 | <1 | <1 | <10 | <10 |
| Example 43 | <1 | <1 | <1 | <10 | <1 |
| Example 44 | <1 | <1 | <1 | <10 | <1 |
| Example 45 | | | | | |
| Example 46 | <1 | <1 | <1 | <1 | <1 |
| Example 47 | <1 | <1 | <1 | <10 | <1 |
| Example 48 | <1 | <1 | <1 | <10 | <10 |
| Example 49 | <1 | <1 | <10 | <10 | <10 |
| Example 50 | <1 | <10 | <10 | <10 | <10 |
| Example 51 | <1 | <1 | <1 | <1 | <1 |
| Example 52 | | | | | |
| Example 53 | | | | | |
| Example 54 | <10 | <10 | <10 | <10 | <10 |
| Example 55 | <10 | <500 | <500 | | |
| Example 56 | <1 | <10 | <1 | <10 | <10 |
| Example 57 | <1 | <10 | <1 | <1 | <10 |
| Example 58 | <1 | <10 | <1 | <1 | <1 |
| Example 59 | <1 | <50 | <1 | <10 | <10 |
| Example 60 | <1 | <10 | <1 | | |
| Example 61 | <10 | <50 | <10 | <50 | <10 |
| Example 62 | <1 | | | | |
| Example 63 | <1 | <10 | <10 | <10 | |
| Example 64 | <1 | | | | |
| Example 65 | <1 | <10 | <10 | <10 | <10 |
| Example 66 | <1 | <10 | <10 | <10 | <10 |
| Example 67 | <1 | <1 | <1 | <10 | <10 |
| Example 68 | <1 | <10 | <10 | <10 | |
| Example 69 | <1 | <1 | <1 | <1 | <10 |
| Example 70 | <1 | <1 | <1 | <10 | <10 |
| Example 71 | <1 | <1 | <1 | <1 | <10 |
| Example 72 | <1 | <1 | <1 | <1 | <10 |
| Example 73 | <1 | <10 | <10 | <10 | <10 |
| Example 74 | <1 | <1 | <1 | <10 | <10 |
| Example 75 | <1 | <1 | <1 | <1 | <10 |
| Example 76 | <1 | <10 | <10 | <10 | |
| Example 77 | <1 | <10 | <10 | <1 | <50 |
| Example 78 | <1 | <1 | <1 | <1 | <10 |
| Example 79 | <1 | <1 | <1 | <1 | <10 |
| Example 80 | <1 | <10 | <10 | <10 | |
| Example 81 | <1 | <10 | <10 | <10 | |
| Example 82 | <1 | <10 | <1 | <1 | <10 |
| Example 83 | <1 | <10 | <10 | <10 | |
| Example 84 | <1 | <10 | <1 | | |
| Example 85 | <1 | <1 | <1 | <10 | |
| Example 86 | <1 | <10 | <1 | <10 | |
| Example 87 | <1 | <1 | <1 | <1 | |
| Example 88 | <1 | <1 | <1 | <1 | |
| Example 89 | <1 | <10 | <10 | <1 | |
| Example 90 | <1 | <10 | <10 | <1 | |
| Example 91 | <1 | <1 | <1 | <10 | |
| Example 92 | | | | | |
| Example 93 | | | | | |
| Example 94 | <1 | <1 | <1 | <1 | |
| Example 95 | <1 | <10 | <1 | <1 | |
| Example 96 | <1 | <10 | <10 | <10 | |
| Example 97 | <1 | <10 | <1 | | |
| Example 98 | <1 | <1 | <1 | <1 | <1 |
| Example 99 | <1 | <1 | <10 | <1 | <1 |

### Biological Test Example 3: KM12-LUC cell proliferation experiment

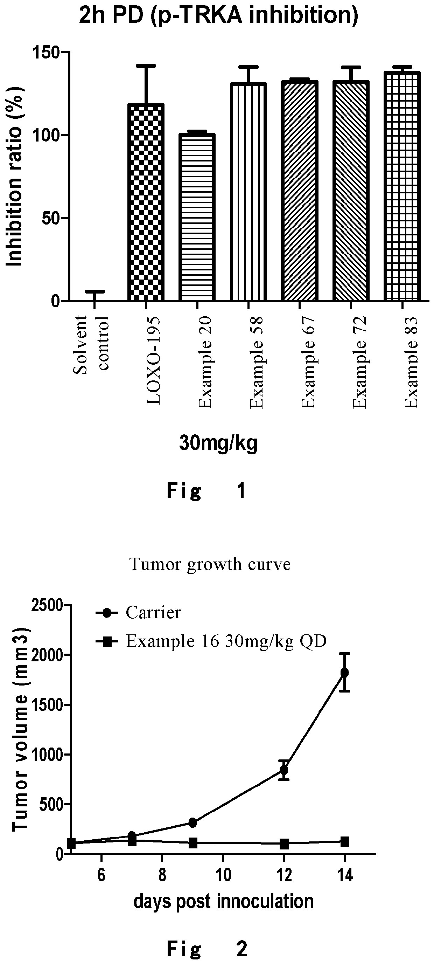

Human colon cancer cell line KM12-LUC (LUC, stably expressing Luciferase) expressing TPM3-NTRK1 fusion gene was used to evaluate cellular activity of the compounds cellular level. The TRK fusion gene in KM12-LUC cells makes it independent on the stimulation of extracellular growth factor, sustainably self-activate and activate the downstream signal pathway associated with cell proliferation such as MAPK-ERK, PI3K-AKT, or the like. Therefore, inhibition of TRK activity in KM12-LUC cells can significantly inhibit the proliferation of cells. The method was as follows: On the first day, the cells were seeded into 384-well plates at 2,000 cells/well; on the second day, different concentrations of test compounds were added; and on the 5^{th} day, CellTiter-Glo (Promega) was added to detect cellular potency, and 72 hours cell proliferation inhibition rate was calculated. Statistical analysis was carried out by Prism5 and the inhibition rate of the test compound were calculated, as shown in Figure 1.

The results show that, the compounds of the invention can effectively inhibit proliferation of KM12-LUC cells.

### Biological Test Example 4: Detection of TRK kinase activity on cellular level by ELISA

NIH-3T3 cell line stably expressing ΔTRKA or ΔTRKA(G595R) was constructed by plasmid transfection.

On the first day, cells were seeded into a 96-well cell culture plate, 10000 cells/well in the medium (DMEM + 10% FBS). On the second day, different concentrations of test compounds were added to treat cells for 2 hours, then the cell culture plate was placed on ice; and the supernatant was removed and washed with pre-cooled PBS once. The cells were lysed with NP40 lysis buffer containing protease and phosphatase inhibitor, transferred to an antibody pre-coated plate, and sealed to incubate overnight at 4 °C. The remaining steps were proceeded according to the method provided in the ELISA kit (eg, as described in R&D DYC2578-2), results were shown in Table 2.

The results show that the compounds of the invention can inhibit TRKA phosphorylation level of ΔTRKA/NIH-3T3 cells or ΔTRKA(G595R)/NIH-3T3 cells.

**Table 2**

| Compound | KM12-LUC IC₅₀ (nM) | ATRKA(G595R) IC₅₀ (nM) |
|---|---|---|
| Example 7 | <10 | <10 |
| Example 58 | <10 | <10 |
| Example 63 | <10 | <10 |
| Example 65 | <10 | <10 |
| Example 97 | <10 | |

### Biological Test Example 5: In vivo efficacy test of small molecular inhibitors of the invention for treating tumor

The mouse model of the subcutaneous inoculated tumor was established to examine inhibitory effects of the compounds on tumor growth. Methods were as follows:
ΔTRKA(G595R)/3T3 cells (5×10⁶) were subcutaneously injected to the dorsal part of the mouse. The tumor volume was monitored by measuring the diameter with a caliper, and calculated by the following formula: length × (width²)/2. When the tumor size was between 150 and 200 mm², the mice were randomly selected to accept the diluent, the compound to be tested, the dosage of which was 30 mg/kg. The compound to be tested was administered once a day for 14 days. After the last administration, the weight of mice was weighed, and tissue and blood were collected 2 hours after administration. The tumor inhibition rate was calculated, the concentration of the tested compound in tumor and blood samples were detected, and the phosphorylation level of TRKA and downstream signal molecules, such as ERK or AKT were detected. The results are shown in FIG. 2. The results showed that the tumor volume of the mice maintained at a lower level when the compounds of the invention were administrated.

The results show that the compounds of the invention can effectively inhibit tumor growth in tumor-bearing mice.

### Biological Test Example 6: Pharmacokinetics experiment of small molecular inhibitors of the present invention in mice

Tested compounds were administered to ICR mice via intravenous administration (IV) and para-oral (PO) administration, and blood samples were taken at different time points. The concentration of the tested article in mouse plasma was measured by LC-MS/MS, and relevant parameters were calculated. Specifically, a desired amount of a compound to be tested was taken and formulated in 5% DMSO + 10% Solutol + 85% injection water to form a solution at desired concentration for intravenous administration or para-oral administration. Animals were about 6-8 weeks old when the administration experiment started. Blood collection time for intravenous administration: 0.083h, 0.25h, 0.5h, 1h, 2h, 4h, 8h, and 24h after administration. Blood collection time for oral administration: 0.25h, 0.5h, 1h, 2h, 4h, 6h, 8h and 24h after administration. The biological sample analysis method and sample detection method were established. The pharmacokinetic parameters were calculated using Phoenix Winnonlin 7.0 software according to plasma concentration data at different time points, such as AUC(0-t), AUC(0-∞), T1/2, Cmax, Tmax, and MRT.

**Mouse pharmacokinetics (5 mg / kg, p.o.)**

| parameter | unit | Example 16 | Example 20 |
|---|---|---|---|
| Cₘₐₓ | ng/mL | 714 | 448 |
| AUC₀₋₂₄ₕᵣ | hr*ng/mL | 3229 | 2705 |
| T_{1/2} | hr | 2.34 | 2.82 |
| F | % | 107 | 144 |

The results show that the compounds of the invention have excellent pharmacokinetics properties.

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A compounds of Formula I: 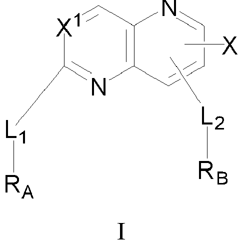 wherein,
X is H, halogen, D, CN or -CONH₂;
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or a substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is selected from the group consisting of a substitution or unsubstituted -(X⁴)_{z}-, wherein each X⁴ is independently selected from the group consisting of a substituted or unsubstituted C₁-C₈ alkylene, -O-, -C(=O)-, -S-, -S(=O)-, -S(=O)₂-, -NH-, -CONH-, -NHCO-, -NHCS-, -NHCONH-, -NHS(=O)-, -NHS(=O)₂-;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 heteroatoms selected from N, S or O, substituted or unsubstituted 5-10 membered heterocyclic group comprising 1-3 heteroatoms selected from N, S or O (including a monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amido (-C(=O)-N(Rc)₂ or -NH-C(=O)(Rc), Rc is H or C1-C5 alkyl), C1-C6 alkyl-(C1-C6 amido), 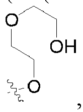
or a substituted or unsubstituted group selected from the group consisting of a C1-C6 alkyl, C3-C8 cycloalkyl, C1-C6 amido, C6-C10 aryl, 5-10 membered heteroaryl comprising 1-3 heteroatoms selected from N, S, or O, 3-12 membered heterocyclic ring group comprising 1-3 heteroatoms selected from N, S or O (including a monocyclic, bicyclic, spiro or bridged ring), - (CH₂) -C6-C10 aryl, -(CH₂)-(5-10 membered heteroaryl comprising 1-3 heteroatoms selected from N, S, or O), wherein the substituent is selected from the group consisting of a halogen, C1-C6 alkoxy, halogenated C1-C6 alkyl, halogenated C1-C6 alkoxy, halogenated C3-C8 cycloalkyl, methyl sulfuryl, -S (=O)₂NH₂, oxo (=O), -CN, hydroxyl, -NH₂, carboxyl, C1-C6 amido (-C(=O)-N(Rc)₂ or -NH-C(= O)(Rc), Rc is H or C1-C5 alkyl), C1-C6 alkyl-(C1-C6 amido), 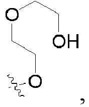 C1-C6 alkyl , C3-C8 cycloalkyl, C1-C6 amine group, C6-C10 aryl, 5-10 membered heteroaryl comprising 1-3 heteroatoms selected from N, S, or O, 3-12 membered heterocyclic ring group comprising 1-3 heteroatoms selected from N, S or O (including a monocyclic, bicyclic, spiro or bridged ring), -(CH₂)-C6-C10 aryl, -(CH₂)-(5-10 membered heteroaryl comprising 1-3 heteroatoms selected from N, S, or O); 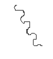
is the connection site of the group;
with the proviso that compounds of formula I are chemical stable structures.

2. The compound of claim 1, wherein L₁ is selected from the group consisting of: 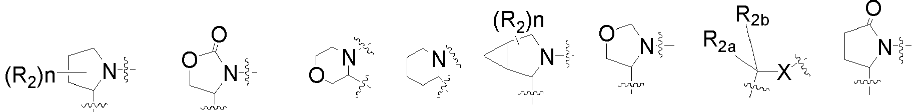
n is selected from the group consisting of 0, 1, 2 and 3;
R₂, R₂ₐ and R_{2b} are each independently selected from the group consisting of H, OH, halogen, substituted or unsubstituted C₁-C₈ alkyl;
X is selected from the group consisting of NH, O, -CONH-, -NHCO-, S, -S(= O)₂-, -NHS(=O)-, -NHS(=O)₂-;
R_{A} is 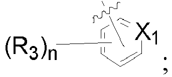 wherein the 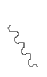 is the connection site of R_{A} and L₁;
L₂ is 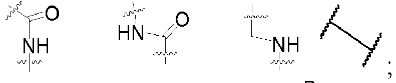
R_{B} is 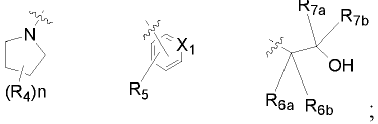 wherein the 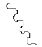 is connection site of R_{B} and L₂;
R₃ is selected from the group consisting of H, halogen, C1-C6 alkoxy, halogenated C1-C6 alkyl, halogenated C1-C6 alkoxy;
R₄ and R₅ are each independently selected from the group consisting of H, OH, halogen, C1-C6 alkyl-OH, C₁-C₆ alkoxy, C₁-C₆ alkyl amine group, C₁-C₆ alkyl amido, -(C₁-C₆ alkyl)-NH-(C₁-C₆alkyl), -(C₁-C₆ alkyl amido)-(C₁-C₆ alkyl);
R₆ₐ, R_{6b}, R₇ₐ, R_{7b} are each independently selected from the group consisting of H, OH, halogen; or R₆ₐ, R_{6b}, R₇ₐ, R_{7b} together with carbon atoms to which they are connected form a 5-12 membered heterocyclic group comprisingh 1-3 heteroatoms selected from N, S or O.

3. The compound of claim 1, wherein the compound is of the structure of the following formula II: 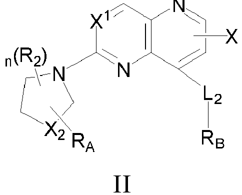 wherein the X₂ is selected from the group consisting of C=O, -CH₂-, O and NH.

4. The compound of claim 1, wherein the compound is of the structure of formula IIIa:

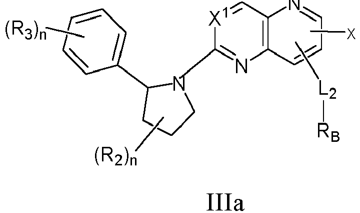

5. The compound of claim 1, wherein the compound is selected from the following group:
| Compound | Structure | Compound | Structure |
|---|---|---|---|
| Example 1 | 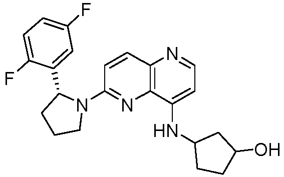 | Example 40 | 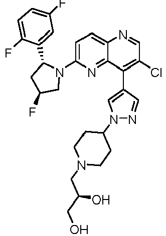 |
| Example 2 | 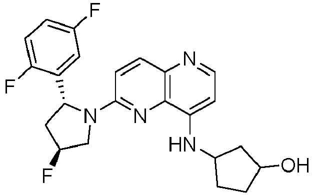 | Example 41 | 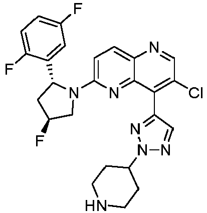 |
| Example 3 | 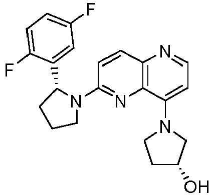 | Example 43 | 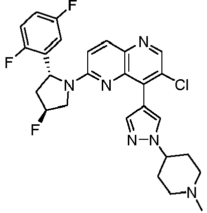 |
| Example 4 | 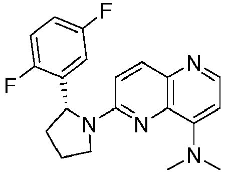 | Example 44 | 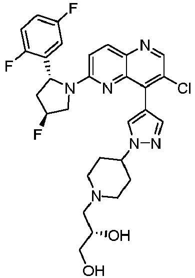 |
| Example 5 | 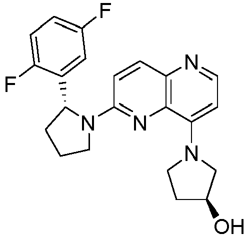 | Example 45 | 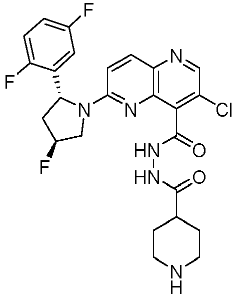 |
| Example 6 | 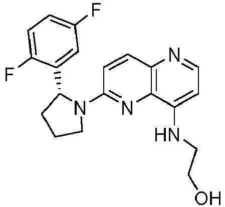 | Example 47 | 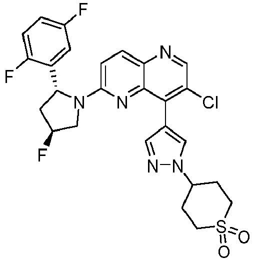 |
| Example 7 | 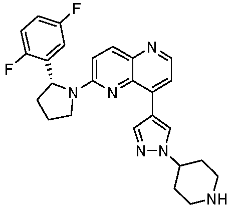 | Example 48 | 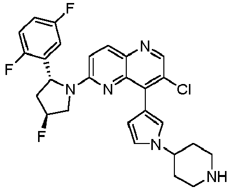 |
| Example 8 | 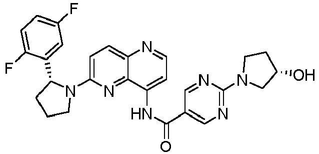 | Example 49 | 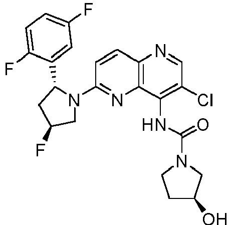 |
| Example 9 | 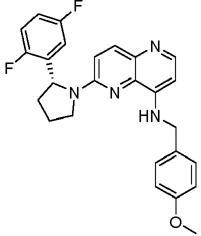 | Example 50 | 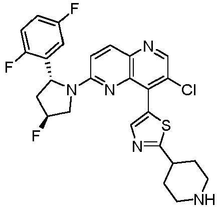 |
| Example 10 | 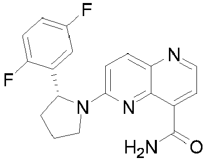 | Example 51 | 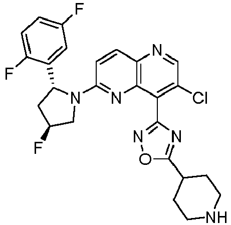 |
| Example 11 | 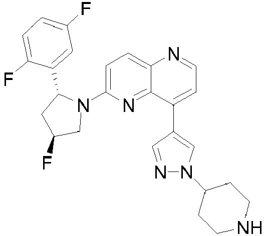 | Example 54 | 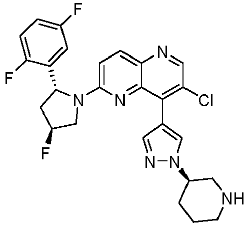 |
| Example 12 | 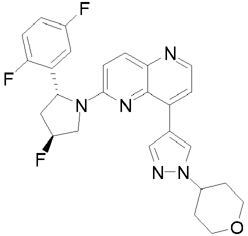 | Example 55 | 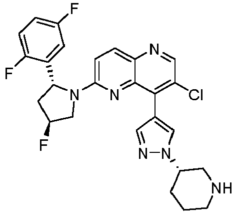 |
| Example 13 | 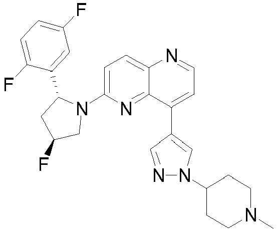 | Example 56 | 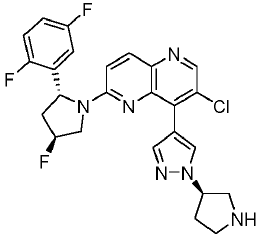 |
| Example 14 | 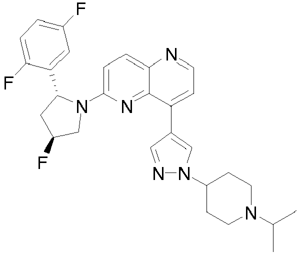 | Example 57 | 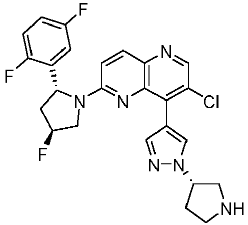 |
| Example 15 | 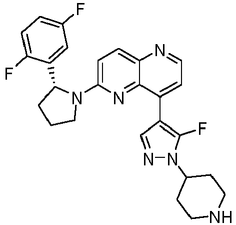 | Example 60 | 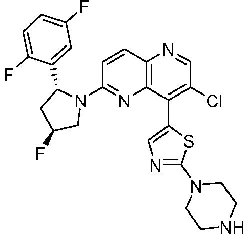 |
| Example 16 | 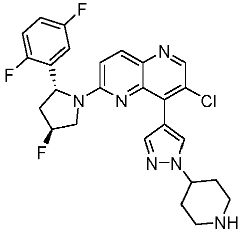 | Example 61 | 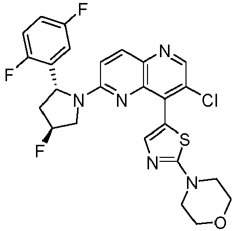 |
| Example 17 | 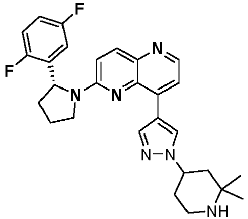 | Example 62 | 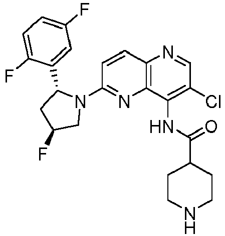 |
| Example 18 | 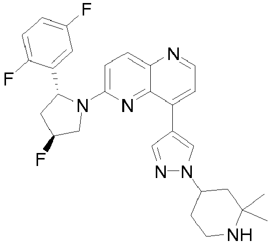 | Example 63 | 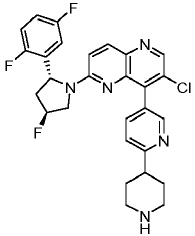 |
| Example 19 | 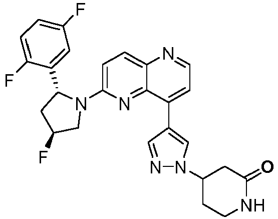 | Example 65 | 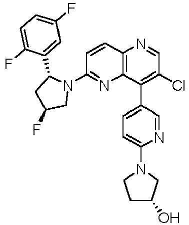 |
| Example 20 | 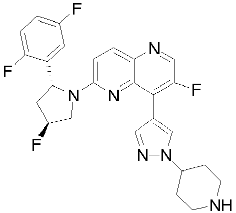 | Example 66 | 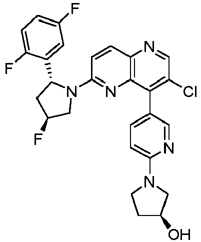 |
| Example 21 | 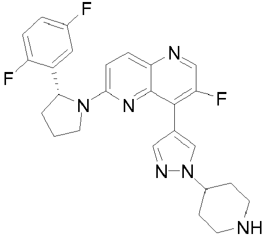 | Example 67 | 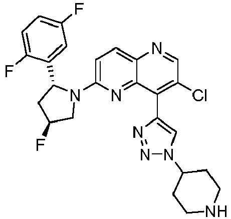 |
| Example 22 | 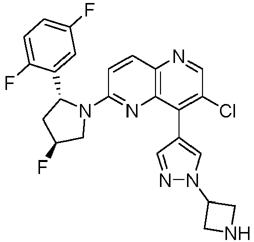 | Example 69 | 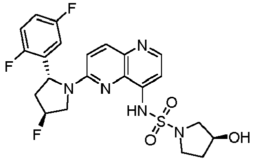 |
| Example 23 | 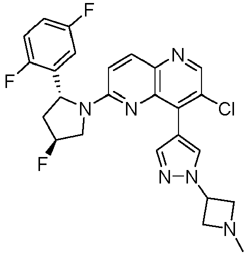 | Example 73 | 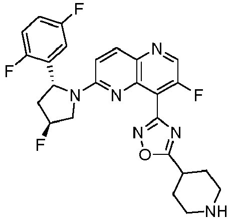 |
| Example 24 | 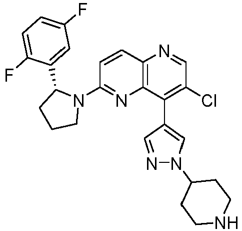 | Example 74 | 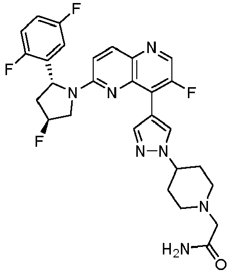 |
| Example 25 | 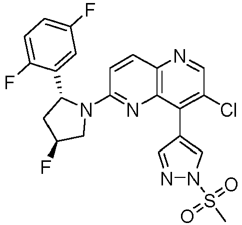 | Example 77 | 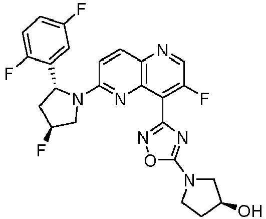 |
| Example 26 | 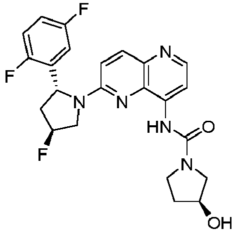 | Example 79 | 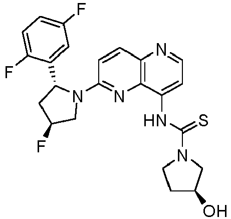 |
| Example 27 | 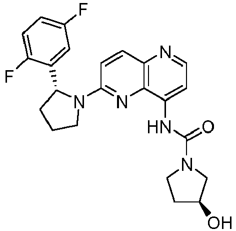 | Example 83 | 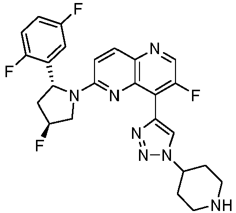 |
| Example 28 | 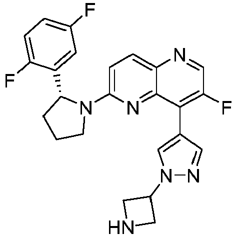 | Example 84 | 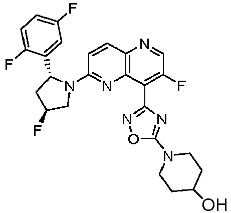 |
| Example 29 | 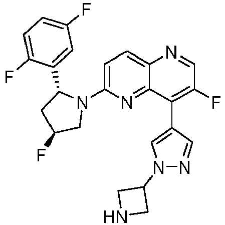 | Example 89 | 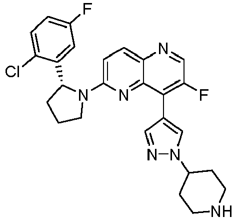 |
| Example 30 | 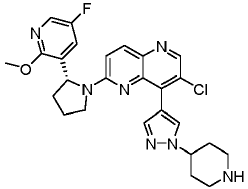 | Example 90 | 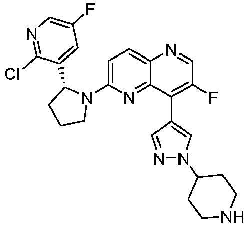 |
| Example 31 | 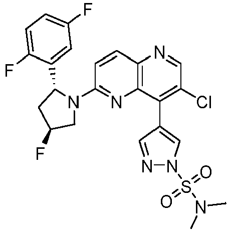 | Example 92 | 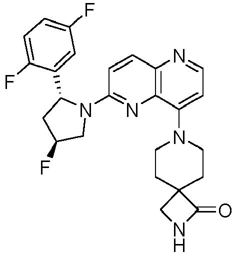 |
| Example 32 | 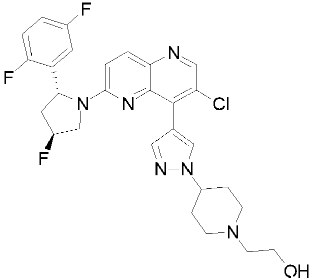 | Example 93 | 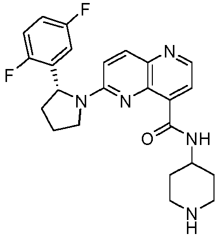 |
| Example 33 | 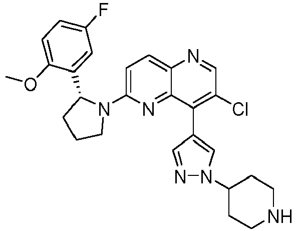 | Example 94 | 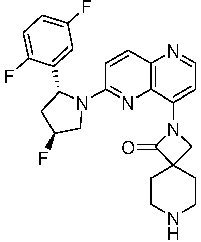 |
| Example 35 | 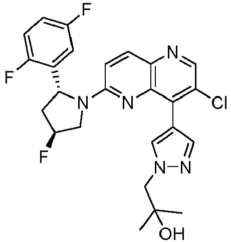 | Example 95 | 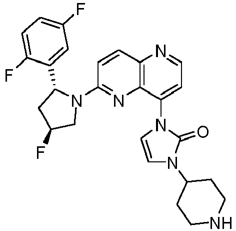 |
| Example 36 | 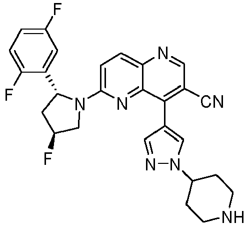 | Example 97 | 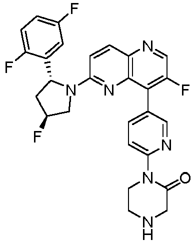 |
| Example 37 | 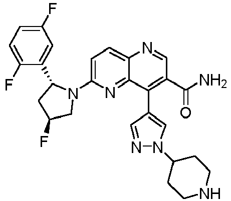 | Example 98 | 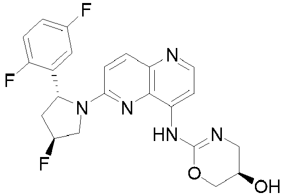 |
| Example 38 | 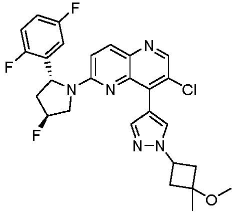 | Example 99 | 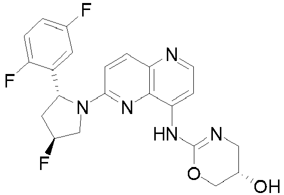 |
| Example 39 | 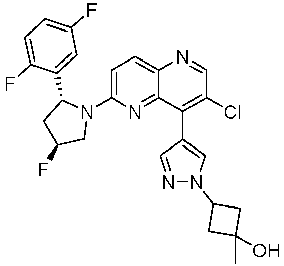 | Example 100 | 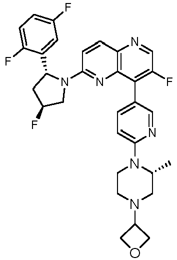 |
| | | Example 101 | 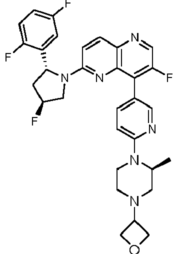 |

6. A compound of formula IV: 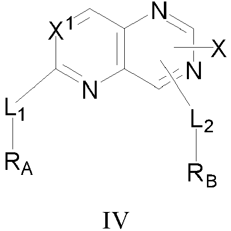 wherein,
X is H, D or halogen;
X¹ is CR or N;
R is selected from the group consisting of H, D, fluorine, chlorine, -OH, -NH₂;
L₁ is selected from the group consisting of a substituted or unsubstituted 5-10 membered heterocycloalkylene group comprising 1-3 heteroatoms selected from N, S or O, or a substituted or unsubstituted -(X³)_{y}-, wherein each X³ is independently selected from the group consisting of: a substituted or unsubstituted C₁-C₈ alkylene group, -O-, -C(=O)-, -CONH-, -NHCO-, -S-, -S(=O)-, -S(=O)₂- and -NH-;
L₂ is a substituted or unsubstituted 5-10 membered heterocycloalkylene group comprising 1-3 heteroatoms selected from N, S or O, 5-10 membered heteroaryl comprising 1-3 heteroatoms selected from N, S or O;
y is selected from 1 or 2; Z is selected from 0, 1 or 2;
R_{A} is selected from the group consisting of H, substituted or unsubstituted C₆-C₁₀ aryl, substituted or unsubstituted 5-10 membered heteroaryl which comprising 1-3 heteroatoms selected from N, S or O;
R_{B} is selected from the group consisting of H, NH₂, OH, -COOH, substituted or unsubstituted C₁-C₈ alkyl, substituted or unsubstituted C₃-C₁₀ cycloalkyl, substituted or unsubstituted C₁-C₈ alkoxy, substituted or unsubstituted C₆-C₁₀ aryl, substituted or unsubstituted 5-10 membered heteroaryl comprising 1-3 hetero atoms selected from N, S or O, substituted or unsubstituted 5-10 membered heterocyclic group comprising 1-3 heteroatoms selected from N, S or O (including a monocyclic, bicyclic, spiro or bridged ring);
unless otherwise specified, the "substituted" means that a group is substituted by one or more (e.g., 2, 3, 4, etc.) substituents selected from the group consisting of a halogen, C1-C6 alkoxy, halogenated C1-C6 alkyl, halogenated C1-C6 alkoxy, halogenated C3-C8 cycloalkyl, methyl sulfuryl, -S(=O)₂NH₂, oxo (=O), -CN, hydroxy, -NH₂, carboxyl, C1-C6 amido (-C(=O)-N(Rc)₂ or -NH-C(=O)(Rc), Rc is H or C1-C5 alkyl), C1-C6 alkyl-(C1-C6 amido), 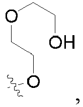
or a substituted or unsubstituted group selected from the group consisting of a C1-C6 alkyl unsubstituted or unsubstituted by one or more hydroxyls, C3-C8 cycloalkyl, C1-C6 amido, C6-C10 aryl, 5-10 membered heteroaryl comprising 1-3 heteroatoms selected from N, S, or O, 5-12 membered heterocyclic ring group comprising 1-3 heteroatoms selected from N, S or O (including a monocyclic, bicyclic, spiro or bridged ring), -(CH₂)-C6-C10 aryl, -(CH₂)-(5-10 membered heteroaryl comprising 1-3 heteroatoms selected from N, S, or O), wherein the substituent is selected from the group consisting of a halogen, C1-C6 alkyl unsubstituted or unsubstituted by one or more hydroxyls, C1-C6 alkoxy, oxo, -CN, -NH₂, -OH, C6-C10 aryl, C1-C6 amino, C1-C6 amido, 5-10 membered heteroaryl comprising 1-3 heteroatoms selected from N, S, or O; 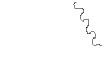
is the connection site of the group; with the proviso that compounds of formula I are chemical stable structures.

7. The compound of claim 1, wherein the compound is of the structure of formula V: 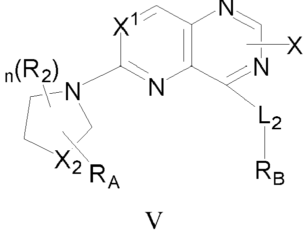 wherein the X₂ is selected from the group consisting of C=O, -CH₂-, O and NH.

8. The compound of claim 1, wherein the compound is of the structure of formula VI:

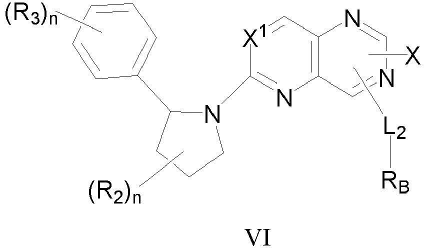

9. The compound of claim 1, wherein the compound is selected from the following table:
| Compound | Structure | Compound | Structure |
|---|---|---|---|
| Example 46 | 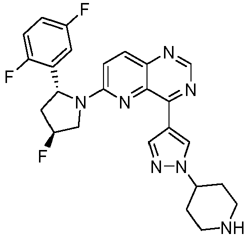 | Example 58 | 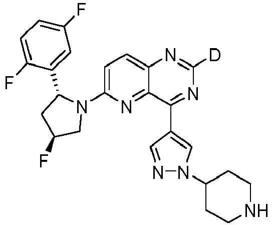 |
| Example 52 | 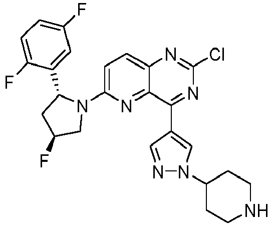 | Example 59 | 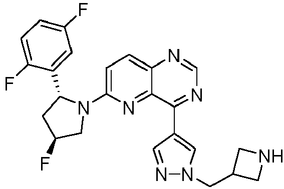 |
| Example 53 | 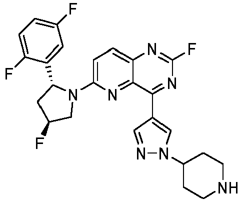 | Example 80 | 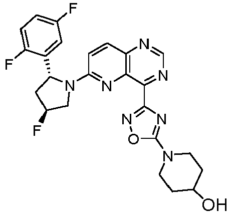 |
| Example 64 | 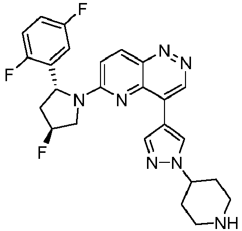 | Example 81 | 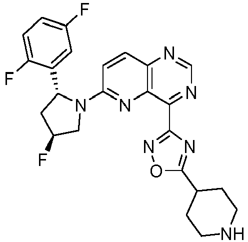 |
| Example 68 | 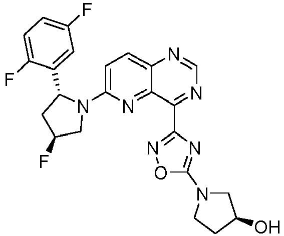 | Example 82 | 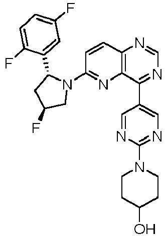 |
| Example 70 | 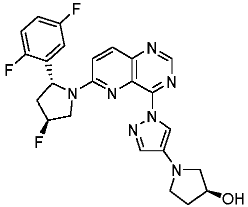 | Example 85 | 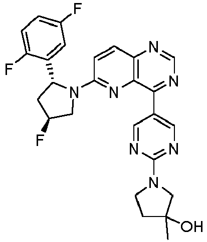 |
| Example 71 | 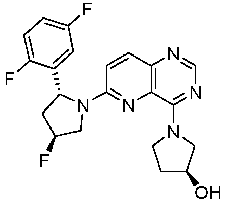 | Example 86 | 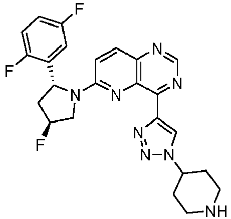 |
| Example 72 | 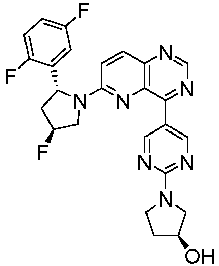 | Example 87 | 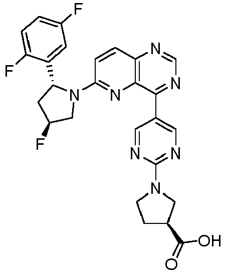 |
| Example 75 | 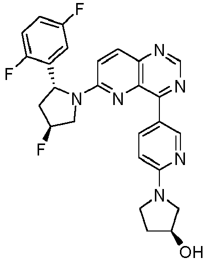 | Example 88 | 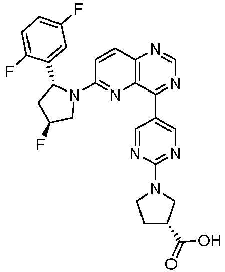 |
| Example 76 | 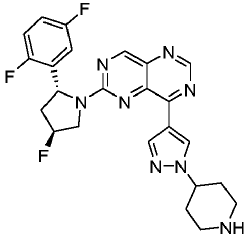 | Example 91 | 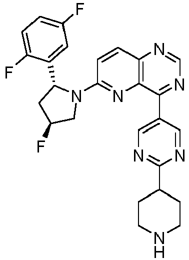 |
| Example 78 | 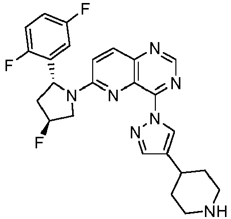 | Example 96 | 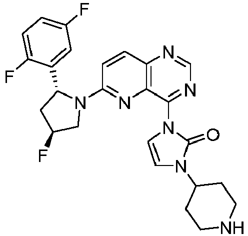 |
| Example 80 | 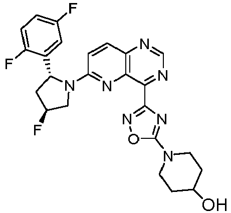 | | |

10. A pharmaceutical composition, wherein the pharmaceutical composition comprises (1) the compound of claim 1, or a stereoisomer thereof, tautomer thereof, or a pharmaceutically acceptable salt, hydrate or solvate thereof, and (2) pharmaceutically acceptable carriers.

11. The use of claim 10, wherein the disease is selected from the group consisting of cancer, proliferative disease, pain, skin disease or condition, metabolic disease, muscle disease, neurological disease, autoimmune disease, itching caused by dermatitis, inflammation related diseases, bone related diseases.

12. Use of the compound of claim 1, or a stereoisomer or a tautomer thereof, or a pharmaceutically acceptable salt, hydrate or solvate thereof, or the pharmaceutical composition of claim 9, in the preparation of a pharmaceutical composition for preventing and/or treating diseases related to TRK function abnormalities (abnormal activation functions induced by TRK gene amplification, overexpression, mutation or gene fusion).

13. The use of claim 12, wherein the disease is selected from the group consisting of cancer, proliferative disease, pain, skin disease or condition, metabolic disease, muscle disease, neurological disease, autoimmune disease, itching caused by dermatitis.

14. A TRK kinase inhibitor, wherein the inhibitor comprises the compound of claim 1, or a stereoisomer or tautomer thereof, or a pharmaceutically acceptable salt, hydrate or solvate thereof.
